# EUROPEAN PATENT APPLICATION

(11) **EP 2 902 013 A1**
(43) Date of publication of application: **05.08.2015**
(21) Application number: 15153333.8
(22) Date of filing: 16.10.2009
(51) Int. Cl.: A61K 9/127, A61K 48/00, C12N 15/11, C12N 15/88

(54) **Processes and Compositions for Liposomal and Efficient Delivery of Gene Silencing Therapeutics**

(30) Priority: 16.10.2008 US 106062 P; 07.04.2009 US 167379 P
(62) Divisional of application: 09744527.4
(71) Applicant: Marina Biotech, Inc., Bothell WA 98021-7266 (US)
(72) Inventor: POLISKY, Barry A., Boulder, CO 80302 (US); ADAMI, Roger C., Bothell, WA 98012 (US); TEMPLIN, Micheal V., Bothell, WA 98012 (US); HARVIE, Pierrot, Bothell, WA 98011 (US); JOHNS, Rachel E., Shoreline, WA 98155 (US); GIYANANI, Jaya S., Redmond, WA 98052 (US); HOUSTON, Micheal E., Acton, MA 01720 (US)
(74) Representative: Srinivasan, Ravi Chandran

(57) **Abstract**

Processes and compositions for liposomal delivery of therapeuticals prepared by contacting an aqueous solution of an active agent with a solution of liposome-forming components containing one or more DILA2 amino acid compounds or lipids in organic solvent to form an impinging stream. A protocol including flow rates, pH, and an incubation period are used to control formation of liposomal components for therapeutic applications. The impinging stream may be collected and incubated to prepare a liposomal formulation which encapsulates the active agent. The composition can be quenched with buffer and filtered by tangential flow and diafiltration and other means for finishing as a pharmaceutical composition. An efficiency for delivering a drug cargo is provided. Compositions can include a liposome containing one or more carrier particles, each carrier particle having an active agent and a peptide, wherein the ratio of the mass of the peptide plus the mass of the liposome to the mass of the active agent is less than about 15.

## Description

### FIELD OF THE INVENTION

This disclosure relates generally to processes, compositions and uses for delivery of biologically active agents and drug agents. The processes and compositions of this disclosure are useful for delivery of therapeutic agents to selected cells, tissues, organs or subjects. Embodiments of this invention may provide for delivery of pharmaceuticals and therapeutic agents, including nucleic acid agents, and methods for making and using materials to effect drug delivery. In particular, this invention relates to processes and compositions containing liposomes or lamellar vesicles, and other forms of delivery-enhancing compositions and formulations, as well as therapeutic methods and uses for these delivery materials.

### SEQUENCE LISTING

This application includes a Sequence Listing submitted herewith via EFS-Web as an ASCII file created on October 14,2009, named MD-08-16PCT.txt, which is 99,622 bytes in size, and is hereby incorporated by reference in its entirety.

### BACKGROUND

The delivery of a therapeutic compound to a subject can be impeded by limited ability of the compound to reach a target cell or tissue, or by restricted entry or trafficking of the compound within cells. Delivery of a therapeutic material is in general restricted by membranes of cells. These barriers and restrictions to delivery can result in the need to use much higher concentrations of a compound than is desirable to achieve a result, which brings the risk of toxic effects and side effects.

A further limitation in delivering certain therapeutic compounds is the need to protect the compound from degradation in the transport process. In particular, systemic delivery via blood circulation can subject the compound to a variety of proteins, enzymes and immunological components and factors.

One strategy for delivery is to improve transport of a compound into cells using natural or synthetic lipophilic or polymeric carrier molecules. These materials can take advantage of mechanisms that exist for selective entry into a cell, while still excluding exogenous molecules such as nucleic acids and proteins. For example, a cationic lipid may interact with a drug agent and provide contact with a cell membrane. Certain natural and synthetic lipophilic molecules can also be organized into liposomes or particles as carriers for drug agents. Liposomes of nanometer or submicron dimension can take advantage of mechanisms that exist for selective entry into a cell, such as endocytosis. Liposomal drug carriers can protect a drug molecule from degradation while improving its uptake by cells. Also, liposomal drug carriers can encapsulate or bind certain compounds by electrostatic and other interactions, and may interact with negatively charged cell membranes to initiate transport across a membrane.

A drawback of liposomes is that biological activity of a therapeutic liposomal formulation will generally depend on the degree of loading of the active agent into the liposomes. In general, a high degree of loading is desirable to provide high therapeutic activity. Further, the loading of liposomes may require additional carrier molecules that will remain within the formulation.

Another limitation of liposomes for drug agent delivery is that using them adds mass to the delivery formulation. The biological activity of a therapeutic formulation and its relative toxicity will be affected by the nature and mass of additional components such as lipophilic molecules and carriers used to prepare the formulation. Conventional lipid-based liposomal formulations for delivery of active agents can have a ratio of more than ten to fifteen times the mass of lipid molecules to the mass of active agent. In general, a lower ratio of lipid or carrier to active agent is more efficient and desirable. Such liposomal formulations for nucleic acid agents may contain no more than a few hundred copies of the nucleic acid agent molecule per liposome.

The understanding of regulatory RNA and the development of RNA interference (RNAi), RNAi therapy, RNA drugs, antisense therapy, and gene therapy, among others, has increased the need for effective means of introducing active nucleic acid agents into cells. In general, nucleic acids are stable for only limited times in cells or plasma. However, nucleic acid-based agents can be stabilized in compositions and formulations which may then be dispersed for cellular delivery.

What is needed are processes, compositions, and uses for systemic and local delivery of drugs and biologically active molecules. Among other things, there is a need for processes for making and using delivery structures and carriers, including liposomal forms, that increase the efficiency of delivery of biologically active and therapeutic molecules. It is desirable to have efficient delivery along with high biological activity using reduced amounts of carrier materials, especially for liposomal formulations, gene silencing therapeutics, and other agents.

### BRIEF SUMMARY

This disclosure provides novel processes, compositions and formulations for intracellular and *in vivo* delivery of drug agents for use, ultimately, as a therapeutic, which in general maintain cytoprotection and relatively low toxicity. The methods and compositions of this disclosure are useful for delivery of drug agents to selected cells, tissues, and organs.

In some aspects, this disclosure provides processes, compositions and methods to deliver active nucleic acid agents or molecules to cells. The active agents may provide therapeutic or pharmacological effects, either through pharmaceutical action, or by producing the response of RNA interference, or antisense or ribozyme effects. Active agents of this disclosure may be useful in the regulation of genomic expression, or for gene therapy.

Embodiments of this invention provide a range of processes for making a composition, including liposomal compositions, containing one or more active agents by providing a first stream comprising an aqueous buffer solution of an active agent, providing a second stream comprising a non-aqueous solution of one or more liposome-forming compounds in organic solvent, impinging the first stream on the second stream, thereby forming an impinging stream having a concentration of the organic solvent of from about 20% to about 50% v/v. The impinging stream may have a pH of from about 6 to about 7.4. The impinging stream can be incubated in a collection reservoir for a period of from about 0.5 hours to about 8 hours at a temperature of from about 20 °C to about 35 °C, thereby forming an incubate comprising liposomes.

In certain embodiments, processes for making a composition containing one or more active agents may include quenching the incubate by adding buffer to the incubate sufficient to make the concentration of the organic solvent less than about 20% v/v.

In some aspects, a liposome-forming compound of this invention may be one or more DILA2 amino acid compounds. A DILA2 amino acid compound is a synthetic organic compound containing an amino acid group that may form a liposome. DILA2 amino acid compounds can contain a delivery-enhancing or lipophilic tail at either the N-terminus or the C-terminus of the amino acid group, or at both termini.

In some variations, processes for making a composition containing one or more active agents may further include that the volume flow rate of the first stream, which contains the active agent, is two times or more the volume flow rate of the second stream, which contains the liposome-forming molecules. In certain variations, the volume flow rate of the first stream is three times or more the volume flow rate of the second stream, or five times or more the volume flow rate of the second stream.

An active agent of this disclosure may be a UsiRNA, a nucleic acid-containing agent, a gene-silencing agent, a gene-regulating agent, an antisense agent, a peptide nucleic acid agent, a ribozyme agent, an RNA agent, or a DNA agent. In some embodiments, the active agent may be a pharmaceutical compound, or a small molecule pharmaceutical.

Processes for making a liposomal composition containing one or more active agents may further include adding buffer to the collection reservoir to adjust the concentration of the organic solvent. The pH of the impinging stream may be adjusted to be from about 3 to about 6. The incubating step may be performed at a pH from about 3 to about 6.

In certain aspects, the active agent may be encapsulated in liposomes at a level greater than about 50%, or greater than about 70%.

In some aspects, this invention may provide a liposomal composition that retains gene-silencing activity for 7 days at a temperature of 45 °C. In certain aspects, the liposomal composition may retain encapsulation of the active agent for 7 days at a temperature of 45 °C.

A process of this invention may include filtering the incubate by tangential flow filtration and diafiltration. After tangential flow filtration the liposomes may be of uniform size less than about 160 nm in diameter, and may have an average diameter from about 40 nm to about 160 nm, or from about 80 nm to about 150 nm. In further embodiments, the incubate may be sterilized, and the organic solvent may be exchanged with a different pharmaceutically-acceptable buffer.

In some processes of this disclosure, the incubate may be filtered by tangential flow filtration and diafiltration. In certain embodiments, the incubate may be sterilized.

A process of this invention may include adding organic solvent to the first stream at a concentration of from about 1 % to about 40% v/v.

The organic solvent may be a (C1-6)alkanol at a concentration of about 40 to about 99% v/v in sterile water for injection, or about 70 to about 95%.

The incubating period of a process of this disclosure may be from about 1 hours to about 4 hours in length.

This invention further contemplates a pharmaceutical composition made by any variation of the disclosed processes.

In general, this disclosure includes methods for delivering a therapeutic nucleic acid to a biological cell.

In some embodiments, this disclosure provides methods for inhibiting expression of a gene in a biological cell by preparing a composition according to a process of this invention and treating the cell with the composition.

Methods for inhibiting expression of a gene in a mammal disclosed herein include preparing a composition according to a process of this disclosure and administering the composition to the mammal.

Embodiments of this invention may further provide methods for treating a disease in a human by preparing a composition according to a process of this disclosure and administering the composition to the human, wherein the disease is cancer, bladder cancer, liver cancer, liver disease, hypercholesterolemia, an inflammatory disease, a metabolic disease, inflammation, arthritis, rheumatoid arthritis, encephalitis, bone fracture, heart disease, and viral disease.

This disclosure further contemplates uses of a composition for treating a disease, and in the preparation of a medicament for treating a disease.

This invention provides a range of compositions and formulations for delivering a biological agent to a cell. More particularly, in certain aspects, this disclosure provides liposomal formulations and carrier particles that are nanometer scale in size. The carrier particles can be loaded into liposomes, may exhibit increased stability in delivery, and can efficiently deliver a drug agent to modulate gene expression or activity.

This disclosure provides compositions, methods and uses for improving systemic and local delivery of drugs and biologically active molecules. Among other things, this application provides novel compositions and methods for making and using delivery structures and carriers which can deliver an active agent within a cell with increased efficiency of delivery.

In some embodiments, this disclosure provides a composition comprising a liposome containing one or more carrier particles, each carrier particle comprising an active nucleic acid agent and a peptide, wherein the ratio of the mass of the peptide plus the mass of the liposome to the mass of the nucleic acid agent is less than about 15, or less than about 12, or less than about 10, or less than about 9, or less than about 8, or less than about 5.

In certain embodiments, this invention provides a composition having a knockdown activity of 50% or greater, or 70% or greater, or 90% or greater for gene silencing of ApoB *in vivo.*

In some variations, the compositions of this disclosure may contain a liposome comprising an amino acid lipid.

In certain aspects, a composition of this disclosure may contain a charged carrier particle, a negatively charged carrier particle, or a positively charged carrier particle.

In certain variations, the active nucleic acid agent is an RNAi-inducing agent or an antisense agent. Each liposome may contain 500 or more copies, or 1000 or more copies, or 5000 or more copies of the active agent molecule.

In some variations, the peptide used for a carrier particle is a cleavable peptide or a crosslinkable peptide. In some embodiments, the peptide is PN4110 or PN183.

This disclosure provides a method for delivering an active nucleic acid agent to a cell comprising preparing a liposomal composition and treating the cell with the composition.

In certain aspects, this disclosure provides a method for inhibiting expression of a gene in a cell comprising preparing a liposomal composition and treating the cell with the composition.

In some aspects, this disclosure provides a method for inhibiting expression of a gene in a mammal comprising preparing a liposomal composition and administering the composition to the mammal.

In some embodiments, this disclosure provides a method for treating a disease in a human, the disease being selected from inflammatory diseases including rheumatoid arthritis, metabolic diseases including hypercholesterolemia, liver disease, encephalitis, bone fracture, heart disease, viral disease including hepatitis and influenza, and cancer, comprising preparing a liposomal composition and administering the composition to the human.

In certain embodiments, this disclosure provides a use of a liposomal composition in the preparation of a medicament for treating a disease including inflammatory diseases including rheumatoid arthritis, metabolic diseases including hypercholesterolemia, liver disease, encephalitis, bone fracture, heart disease, viral disease including hepatitis and influenza, and cancer.

In some variations, this disclosure provides a use of a liposomal composition for treating a disease selected from inflammatory diseases including rheumatoid arthritis, metabolic diseases including hypercholesterolemia, liver disease, encephalitis, bone fracture, heart disease, viral disease including hepatitis and influenza, and cancer.

This summary, taken along with the detailed description of the invention, as well as the figures, the appended examples and claims, as a whole, encompass the disclosure of the invention.

### BRIEF DESCRIPTION OF THE DRAWINGS

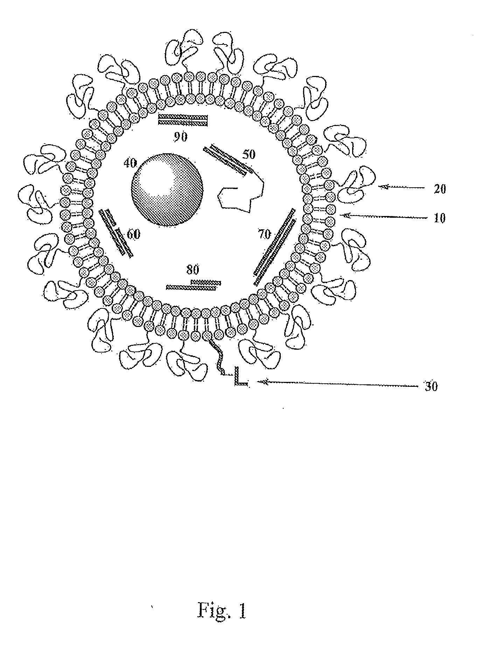

FIG. 1: Schematic representation of a liposomal embodiment of this invention in which certain liposome-forming compounds form a bilayer vesicle 10. In this embodiment, the outer layer of the liposome is protected by polyethyleneglycol chains 20 attached to a head group of one of the liposome-forming molecules. The outer layer of the liposome also presents a ligand 30 for specific targeting of a cell or tissue. The liposomal vesicle contains, in this embodiment, a cargo of active interfering RNA components including a condensed RNA nanoparticle 40, a two-stranded RNA duplex peptide conjugate 50, a three-stranded mdRNA 60, a dicer enzyme substrate RNA 70, a dsRNA with a long overhang 80, and an siRNA with blunt ends 90, which are pooled in this embodiment.

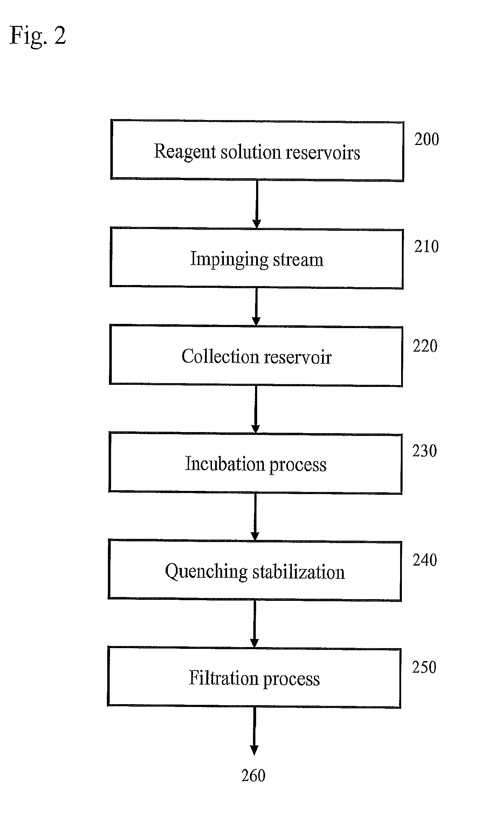

FIG. 2: Flow chart for certain embodiments for preparing liposomal compositions of this disclosure. Reagent solutions including an active agent solution, a buffer solution, and a solution of one or more liposome-forming compounds are prepared separately and provided in reservoirs 200. The active agent solution and the solution of liposome-forming compounds are contacted in an impinging stream 210. The impinging stream is collected in a collection reservoir 220. The collected material is held in the reservoir for an incubation process 230, after which step the material is stabilized by quenching 240. The quenched material is subjected to a filtration process 250. The filtrate output 260 continues to a finishing process.

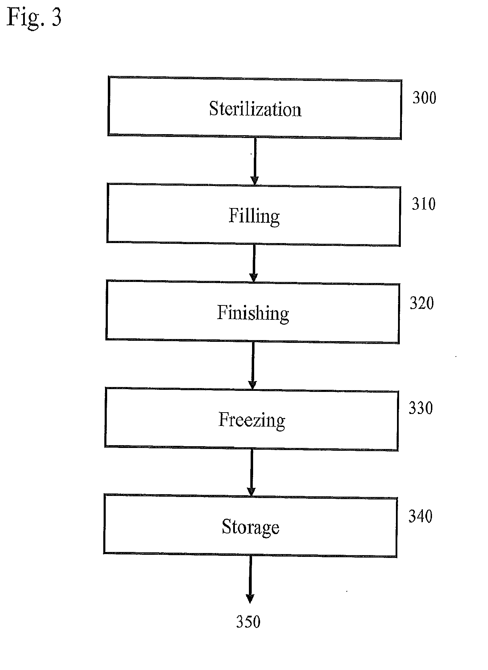

FIG. 3: Flow chart for certain embodiments for finishing liposomal compositions of this disclosure. The liposomal composition, which may be a filtrate output material from preparation of the liposomal composition, is sterilized **300.** Vessels for carrying the sterilized composition are filled **310** and finished **320,** after which the sterile composition is frozen **330** for storage **340.** The final composition is shipped **350** for use.

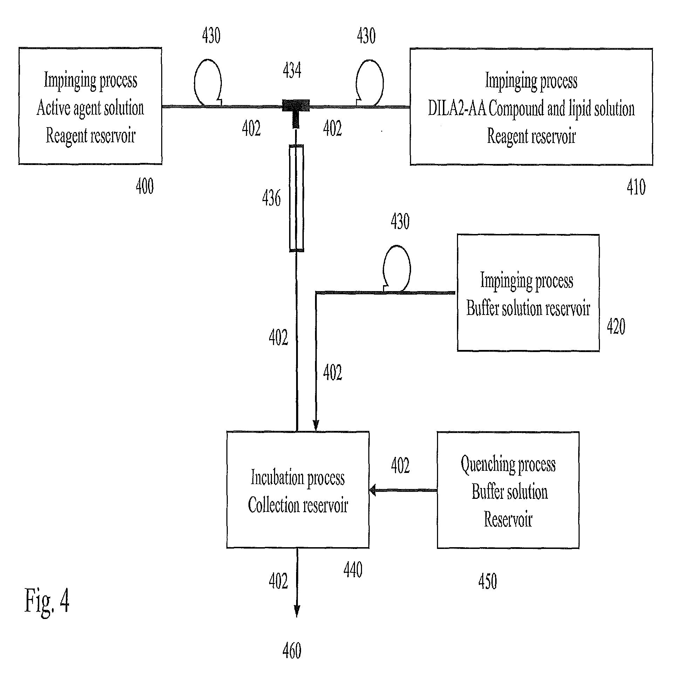

FIG. 4: Process diagram for certain embodiments for preparing liposomal compositions of this disclosure. An active agent solution is maintained in a reservoir **400.** A solution containing liposome-forming components such as a DILA2 compound or a lipid is maintained in a separate reservoir **410.** A buffer solution is maintained in another reservoir **420.** The solutions are pumped using separate peristaltic pumps **430** at independently selected flow rates. The solutions may optionally pass through an in-line filter, or be filtered before being charged to the reservoir. In an impinging process, the active agent solution is contacted with the solution containing the liposome-forming components at a contact point **434.** The impinging stream may optionally pass through one or more mixing tubes **436.** The impinging stream enters a collection reservoir **440** for an incubation process. A quenching process buffer solution may optionally be maintained in a separate reservoir **450.** The liposomal composition **460** exits the collection reservoir **440** to enter a filtration process.

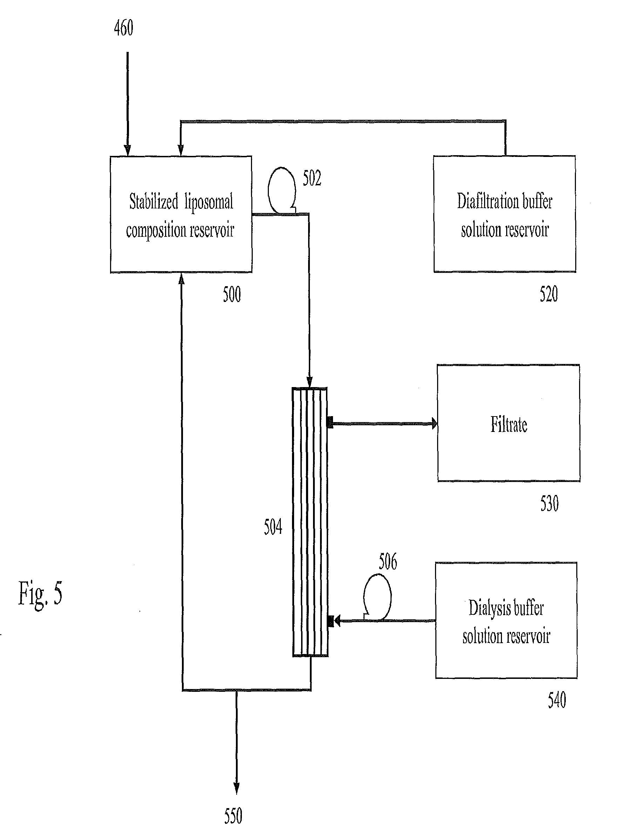

FIG. 5: Process diagram for certain embodiments for preparing liposomal compositions of this disclosure. A stabilized liposomal composition is provided in a reservoir **500** from the output **460** of the collection reservoir. A diafiltration buffer solution is maintained in a separate reservoir **520.** A peristaltic pump **502** provides circulation of the stabilized liposomal composition from reservoir **500** through a tube containing a hollow fiber membrane **504.** Tangential flow filtration occurs via this circulation to concentrate the stabilized liposomal composition by the removal of filtrate **530.** The addition of replacement buffer from reservoir **520** allows for diafiltration at a fixed or variable volume. Optionally, dialysis of the stabilized liposomal composition may be performed using peristaltic pump **506** to drive the dialysis buffer solution from reservoir **540.** The stabilized liposomal composition at a particular concentration is output **550** to a finishing process.

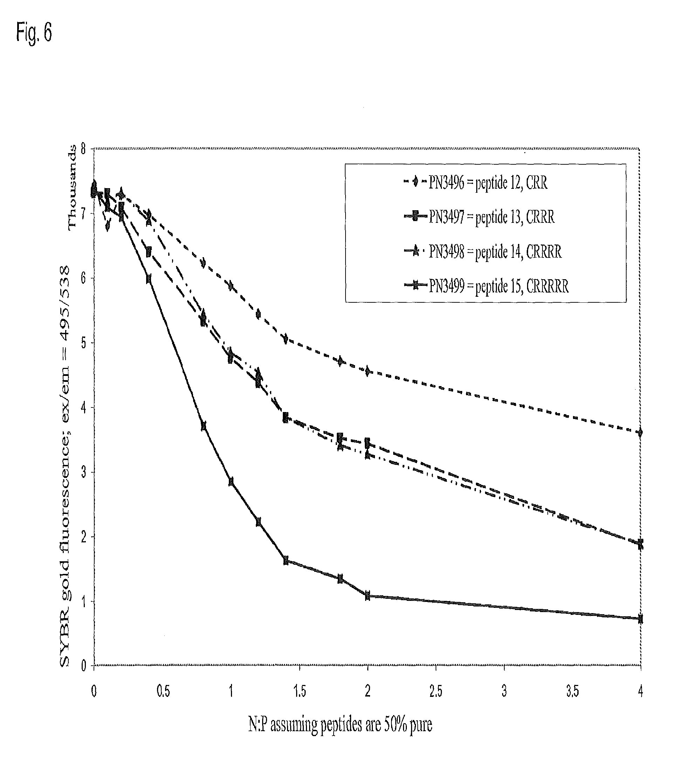

FIG. 6: Figure 6 shows that the binding of a polyarginine binding region to dsRNA increased with the length of the polyarginine binding region. In Figure 6, the strongest binding (best ability to displace SYBR-Gold dye) was observed with PN3499, which was a dimer peptide containing at total of 10 arginines.

### DETAILED DESCRIPTION OF MODES

Embodiments of this invention can provide for delivery of pharmaceuticals and therapeutic agents, including nucleic acid agents, and methods for making and using materials to effect drug delivery.

This invention further relates to novel drug delivery enhancing processes and compositions that are useful for delivering various molecules and structures to cells. This invention provides a range of processes, compounds, compositions, formulations, and uses directed ultimately toward drug delivery, therapeutics, and the diagnosis and treatment of diseases and conditions, including those that respond to modulation of gene expression or activity in a subject. More specifically, this invention relates to processes and compositions containing liposomes or lamellar vesicles, and other forms of delivery-enhancing compositions and formulations, as well as therapeutic methods and uses for these delivery materials.

The processes and compositions of this disclosure may further be used for delivery of therapeutic, prophylactic, and diagnostic agents such as nucleic acid agents, polynucleotides, peptides, proteins, and small molecule compounds and drugs.

The compositions and methods of this disclosure are useful for delivery of therapeutic agents in forms such as encapsulated within liposomes or lamellar vesicles. These forms may include nanoparticles of various diameters.

The understanding of regulatory RNA and the development of RNA interference (RNAi or iRNA), RNAi therapy, RNA drugs, antisense or ribozyme therapy, and DNA gene therapy, among others, has increased the need for effective means of introducing active nucleic acid agents into cells. In general, nucleic acids are stable for only limited times when introduced into cells or blood. However, nucleic acid-based agents can be stabilized in compositions and formulations which may then be administered and dispersed for cellular delivery.

Nucleic acid agents include any nucleic acid-containing moieties such as gene-silencing agents, gene-regulating agents, antisense agents, peptide nucleic acid agents, ribozyme agents, RNA agents, and DNA agents.

In some embodiments, the compositions and methods of this disclosure are useful for delivery of a therapeutic agent encapsulated in a liposome. In these embodiments, the therapeutic agent may be referred to as the cargo.

For example, Fig. 1 shows a schematic representation of a liposomal embodiment of this invention in which various lipophilic molecules form a bilayer vesicle **10.** In this embodiment, the outer layer of the liposome is protected by polyethyleneglycol chains **20** attached to a head group of one of the lipophilic molecules. The outer layer of the liposome also presents a ligand **30** for specific targeting of a cell or tissue. The liposomal vesicle contains, in this embodiment, a cargo of active RNA components including a condensed RNA nanoparticle **40,** a two-stranded RNA duplex peptide conjugate **50,** a three-stranded mdRNA **60,** a dicer substrate RNA **70,** a dsRNA with a long overhang **80,** and an siRNA with blunt ends **90,** which are pooled in this embodiment. Other forms of therapeutic cargo may include microRNA, hairpin RNA, DNA or ribozyme forms.

In general, any active agent can be utilized as cargo in the processes and compositions of this disclosure. In some embodiments, the cargo may be a small organic molecule pharmaceutical agent. In certain embodiments, the cargo may be a negatively charged or neutral therapeutic agent.

The processes and compositions provided in some aspects of this disclosure may deliver a therapeutic agent in a releasable form or composition. Releasable forms and compositions include molecules that bind and release an active agent, molecules that bind an active agent and discharge a moiety that assists in release of the agent, molecules that bind an active agent and are subsequently modulated in form within a biological compartment to assist in release of the agent, and compositions containing molecules that bind an active agent admixed with a release mediator compound.

In certain aspects, this disclosure provides methods and apparatuses for making liposomal compositions suitable for delivery of therapeutic agents. In certain embodiments, an active agent of this disclosure is a UsiRNA. The methods of this disclosure may provide liposomal compositions of nucleic acid agents such as two- or three-stranded RNA structures, RNA peptide conjugates, condensed RNA nanoparticles, dicer substrate RNAs, dsRNAs, siRNAs, nucroRNAs, hairpin RNAs, and other active RNA forms.

The active agent of this disclosure may be a peptide condensate of an active RNA agent. For example, nanoparticles formed by condensing an active RNA agent with a peptide or other biomolecule, condensates of an RNA with a polymeric species, can be loaded as cargo into a liposomal composition of this invention. The nanoparticles may be crosslinked.

In further embodiments, an active agent of this disclosure may be a peptide, a protein, a protease, an antibody, a monoclonal antibody, an antibody-based drug, a vaccine agent, or a small molecule drug.

A composition containing an active agent may be an aqueous solution.

As used herein, the term aqueous solution refers to a water solution, a sterile water solution, or any solution for which the majority of the solvent is water. An aqueous solution may contain some organic solvent, for example.

Examples of aqueous solvents include water, sterile water for injection, Ringer's solution and isotonic sodium chloride solution.

A composition containing a liposome-forming molecule or lipophilic molecule may be a non-aqueous solution.

As used herein, the term non-aqueous solution refers to any solution for which the majority of the solvent is not water. A non-aqueous solution may contain some water.

Examples of non-aqueous solvents include organic solvents that are miscible with water, alkanols, (C1-6)alkanols, ethanol, isopropanol, isobutanol, secbutanol, t-butanol, alkanol-water, ethanol-water, acetonitrile, acetone, ketones, dimethylsulfoxide, dimethylformamide, surfactant solutions, detergent solutions, and mixtures thereof.

### Processes for liposomal compositions

Embodiments of this invention may provide a range of processes for making a liposome-containing composition containing an active agent, as well as methods for drug delivery.

In some aspects, a liposomal composition can be made by impinging two compositions, for example, a composition containing one or more active agents, and a separate composition containing one or more liposome-forming molecules.

In general, the liposome structures of this disclosure are not composed in fully active forms until all steps of the preparation process have been completed. Certain time periods are required for each step in the processes of this invention. The rates of formation of liposomal compositions will in general depend on the unpredictable effects of the combination of many variables, for example, flow rates, temperature, pH, and the concentration of each component. In some embodiments, an incubation period is used to control the formation process.

Fig. 2 shows a flow chart for certain embodiments for preparing liposomal compositions of this disclosure. Referring to Fig. 2, reagent solutions, including an active agent solution, a buffer solution, and a solution of one or more liposome-forming compounds are prepared separately and provided in solution reservoirs **200.** The reagent solutions can optionally be filtered separately or prepared aseptically. The active agent solution and the solution of liposome-forming compounds are contacted in an impinging process **210.** The impinging stream is collected in a collection reservoir **220.** The collected material is held in the reservoir for an incubation process **230,** after which steps the material is stabilized by quenching **240.** The quenched material is subjected to a filtration process **250.** The filtrate output **260** is removed to a finishing process.

This invention includes embodiments of a process for making a liposomal composition which includes an impinging process. An impinging process may have one or more steps for creating an impinging stream by impinging a composition containing an active agent on a composition containing liposome-forming molecules.

In some aspects, a process of this disclosure for making a liposomal composition of an active agent may have one or more steps of an incubation process, An incubation process can include collecting and holding an impinging stream in a reservoir for an incubating period.

Embodiments of this invention may further include a process for making a liposomal composition in which the incubated composition is quenched. Quenching may be done by adding a solvent, buffer or diluent to a stream or mixture of the incubated composition. Quenching can dilute the concentration of a particular component such as an organic solvent or dispersant below a prescribed level. In general, quenching of an incubated composition may form a composition which is stable in relation to further process or finishing steps. Steps of quenching are operable to stabilize the incubated composition which may contain liposomal structures.

Incubation of the impinging stream in a collection reservoir, along with other steps of a process, can provide a liposomal formulation in which an active agent is highly encapsulated by liposomes. For example, in certain embodiments, formation of the liposomal compositions and structures of this disclosure may require any of the steps of impinging, mixing, diluting, collecting, incubating, adjusting pH, quenching, and filtering.

Processes for making a liposomal composition of this disclosure may further include one or more steps of filtration. Steps of filtration may be used to change various process parameters, for example, to control or alter the concentration of a component, or to alter a particle size or dispersity, as well as other physical solution parameters.

Fig. 3 shows a flow chart for certain embodiments for finishing liposomal compositions of this disclosure. Referring to Fig. 3, the liposomal composition, which may be a filtrate output material from preparation of the liposomal composition, is sterilized **300.** Reservoirs for carrying the sterilized composition are filled **310** and finished **320,** after which the sterile composition is frozen **330** for storage **340.** The final composition is shipped **350** for use.

Processes of this disclosure involving sterilization, fill and finish of materials to containers, and storage of finished formulations may use steps and methods known in the art, such as those described in Remington's Pharmaceutical Sciences (18th ed. 1990).

Some methods for evaluating encapsulation, sizing, and general preparation of liposomes are given, for example, in WO200100537, U.S. Pat. Publ. Nos. 20040142025 and 20070252295, and U.S. Pat. No. 6,843,942.

### Impinging and mixing

In certain aspects, this invention provides a range of methods and process conditions for making a liposomal composition of an active agent.

Fig. 4 shows a process diagram for certain embodiments for preparing liposomal compositions of this disclosure. Referring to Fig. 4, a solution of an active agent is maintained in a reservoir **400.** A solution containing liposome-forming components such as a DILA2 amino acid compound or a lipid is maintained in a separate reservoir **410.** A buffer solution is maintained in another reservoir **420.** The solutions are pumped through transfer tubes **402** using separate peristaltic pumps **430** at independently selected flow rates. The solutions may optionally pass through an in-line filter, or be filtered before being charged to the corresponding reservoir. In an impinging process, the active agent solution can be contacted with the solution containing the liposome-forming components at a contact point **434.** The contact point may be of any shape, angle, orientation or size. The impinging stream may optionally pass through one or more turbulent mixing tubes **436.** The impinging stream enters a collection reservoir **440.** A buffer solution can be pumped from reservoir **420** into the collection reservoir **440.** The mixture collected in the collection reservoir **440** can be held for a period of time in an incubation process. A quenching process buffer solution may optionally be maintained in a separate reservoir **450.** The quenched liposomal composition **460** exits the collection reservoir **440** to enter a filtration process.

In certain embodiments, the buffer solution in reservoir **450** may optionally be used to dilute the impinging stream, and may be contacted with the impinging stream before the collection vessel, or before any mixing tube.

As discussed above, to form a liposomal composition, certain processes of this disclosure provide an impinging stream which undergoes mixing in the transfer tubes and optionally in a turbulent mixing tube, collection in a vessel or reservoir, an incubation process, and quenching. The quenched material is output for filtration and finishing.

An impinging stream will in general result from contacting a composition of an active agent with a composition containing liposome-forming molecules. The impinging stream may serve only to contact the compositions to form a single stream. The impinging stream may not in general provide complete interdispersion or intermixing of the compositions. In optional steps, an impinging stream may be subjected to turbulent mixing conditions.

The pH of the impinging stream may be controlled in the range of from about 3 to about 9. In some embodiments, the pH of the impinging stream is from about 5 to about 8, or from about 6 to about 7, or about 7.4. In certain variations, the pH of the impinging stream is from about 3 to about 6. The pH of the impinging stream can be adjusted during transfer of the impinging stream through the transfer tubes, or in a mixing tube, or in the collection reservoir. In certain embodiments, the initial pH of the impinging stream is from about 5 to about 8, or from about 6 to about 7.4, and the pH is adjusted after the initial impingement to a range of from about 3 to about 6. In certain embodiments, the pH of the impinging stream is always about 7.4.

The compositions used to form the impinging stream may optionally be filtered before impinging. A composition containing one or more active agents of this disclosure may be filtered by, for example, flow filtration techniques to remove undesirable particles or phases larger in dimension than about 200 nanometers (nm), or larger than about 300 nm, or larger than about 500 nm. A composition containing various liposome-forming molecules may be filtered by, for example, flow filtration techniques to remove undesirable particles or phases larger in dimension than about 200 nm, or larger than about 300 nm, or larger than about 500 nm.

The temperature of the impinging stream can be controlled in the range of from about 15° C to about 37° C.

Aspects of this invention further provide that the composition of the impinging stream may be controlled using the flow rates for the compositions that are impinged. In general, each composition will stream through a tube of a selected diameter, therefore, the relative volume flow rates of the streams that are combined in an impinging stream provides a description of the concentration of various components in the impinging stream.

For example, when an impinging stream is formed by impinging two separate streams flowing through tubes of the same diameter, then the flow rates in the separate streams will determine the concentrations of the components in the impinging stream, as compared to the concentrations of the components in the original streams. Thus, the flow rates can be used to provide a desired composition in the impinging stream for making a liposomal composition having particular characteristics.

In certain embodiments, the flow rates of the streams can be used to control the concentrations of the active agents relative to the liposome-forming molecules, as well as other parameters including the concentration of a solvent or salt, as well as mixing and shear forces. Embodiments of this invention include processes for making a liposomal formulation in which encapsulation of active agents and liposome particle size are advantageously enhanced by adapting the flow rates of the process apparatus.

In certain aspects, a process of this invention may employ tubes of the same diameters for impinging a stream of a composition of the active agents on a stream of a composition containing lipophilic molecules. In certain variations, the flow rates of the compositions may be equal, or unequal. In particular embodiments, the flow rate of the composition containing the active agents may be unequal to the flow rate of the composition containing the lipophilic molecules. For example, in certain embodiments, the flow rate of the composition containing the active agents may be as much as twice the flow rate of the composition containing the lipophilic molecules. In other variations, the flow rate of the composition containing the active agents may two times or more than the flow rate of the composition containing the lipophilic molecules, or in some embodiments three times or more than the flow rate of the composition containing the lipophilic molecules, or five times or more than the flow rate of the composition containing the lipophilic molecules.

In general, the tubes of the apparatus may be of any diameter. In certain embodiments, a process of this invention may employ tubes of different diameters for impinging a stream of a composition of the active agents on a stream of a composition containing liposome-forming molecules. In certain variations, the diameters of the tubes may be equal, or unequal. For example, in certain embodiments, the diameter of the tube containing the solution of active agents may be three-quarters the diameter of the tube containing the solution of lipophilic molecules. In other variations, the diameter of the tube containing the solution of active agents may be half the diameter of the tube containing the solution of lipophilic molecules. In other variations, the diameter of the tube containing the solution of active agents may be greater than the diameter of the tube containing the solution of lipophilic molecules.

In some embodiments, the impinging stream may be further mixed using certain means for flow-through mixing. Means for flow-through mixing include a mixer having one or more channels, capillaries, or pathways arranged to change the direction of flow, where the channels, capillaries, or pathways may diverge and re-connect one or more times to provide turbulent mixing. Means for flow-through mixing may optionally include a mechanical agitator, a shaker, or a stirring rod, blade, paddle, plate, or vane.

The Reynolds number for turbulent mixing may be greater than 2000, or greater than 2400.

The residence time of the mixture stream in a turbulent mixer can be controlled by adjusting the flow rate of the impinging stream. In some variations, the flow rates and residence time of the impinging stream in the turbulent mixer can be used to control the sizing of the liposome particles.

An example of a turbulent mixing tube means for flow-through mixing is Cole-Parmer in-line static mixer K-04669-52, 316 stainless steel tube mixer; 3/16" tube OD, 21 elements.

The vessels, tubes, and other flow components of the apparatus used in each process of this disclosure may be made of any material that is inert to the reactants and solvents used, and suitable for the reaction conditions such as temperature and pH. Examples of materials include polymers, metals, stainless steel, glass, and ceramics. The vessels, tubes, and other flow components may also be coated with an inert substance.

The vessels, tubes, and other flow components are in general in fluid communication with one or more controllable pumps that allow for control of the flow rate of the solutions and mixtures in each step. The apparatus may include various valves, for example check valves, to control the flow. The vessels, tubes, and other flow components may be attached with various fasteners, which may include ferrules or o-rings. The apparatus may include temperature sensors at various points in the flow pathway.

The methods and apparatuses of this disclosure may be used in a batch or a continuous process.

### Collection reservoir, incubation process, and quenching process

Referring to Fig. 4, in some embodiments, the impinging stream enters a collection reservoir **440.** In the collection reservoir **440,** the collected impinging stream is subjected to an incubation process.

An incubation process may include one or more steps of mixing the collected material, one or more steps of dilution with a dilution buffer, one or more steps of adjusting the pH in the collection reservoir, and one or more steps of holding the collected material for an incubation period at a particular temperature.

The collected material can be mixed in the collection reservoir using, for example, a mechanical agitator, a rocker, or a stirring rod, blade, paddle, plate, or vane.

In some variations, an impinging stream may be formed by contacting a composition of an active agent with a composition containing liposome-forming compounds, and adding a buffer, solvent or diluent to the impinging stream. The addition of buffer, solvent or diluent may occur before mixing or collection of the impinging stream, or may be done in the collection reservoir as part of an incubating process. The addition of buffer, solvent or diluent reduces the concentrations of the active agent, liposome-forming molecules, and other components such as another solvent in the collection reservoir.

In some embodiments, the addition of buffer, solvent or diluent to the impinging stream, whether in the transfer tubes, or in the mixing tube, or in the collection reservoir, may dilute the concentration of the organic solvent to about 50% (v/v) or lower, or about 40% (v/v) or lower, or about 35% (v/v) or lower, or about 33% (v/v) or lower, or about 30% (v/v) or lower, or about 25% (v/v) or lower, or about 22% (v/v) or lower, or about 20% (v/v), or lower.

The pH of the collected mixture or composition in the collection reservoir can be controlled in the range of from about 3 to about 9. In some embodiments, the pH of the collected impinging stream is adjusted to be from about 5 to about 8, or from about 6 to about 7.4. In certain embodiments, the pH of the collected mixture is from about 5 to about 8, and the pH is adjusted after the initial impingement to a range from about 3 to about 6. In some variations, the pH of the collected impinging stream is maintained at about 7.4. A liposomal composition of this disclosure may also be formed in a process for which the pH is about 7.4 in each step.

In some aspects, the collected mixture or composition in the collection reservoir is subjected to an incubation hold period. The length of the hold period of the incubate in the collection reservoir may range from a few minutes to several hours, or from about 15 minutes to about eight hours, or from about 0.5 hours to about 8 hours, or from about 0.5 hours to about 4 hours, or from about 1 hour to about 4 hours, or from about 1 hour to about 2 hours.

In some variations of the process, turbulent mixing occurs after dilution of the impinging stream with buffer, solvent or diluent, and the hold period of the incubate may range from about 0.5 hours to about 8 hours, or from about 1 hour to about 4 hours, or from about 1 hour to about 2 hours.

In certain variations, turbulent mixing occurs before dilution of the impinging stream with buffer, solvent or diluent, and the hold period of the incubate may range from a few minutes to several hours, or from about 15 minutes to about eight hours, or from about 0.5 hours to about 8 hours, or from about 0.5 hours to about 4 hours, or from about 1 hour to about 4 hours, or from about 1 hour to about 2 hours.

The length of the incubating period for the incubation process may depend in general on other process parameters such as the flow rates of the impinging stream, as well as the temperature and pH.

During the hold period, the temperature of the collected composition in the collection reservoir can be controlled in the range of from about 15° C to about 37° C, or from about 22° C to about 35° C.

In some aspects, the incubation process may be terminated by quenching the incubate with rapid addition of buffer, solvent or diluent. The quenching step may reduce the concentration of the organic solvent to about 20% (v/v) or lower, or about 15% (v/v) or lower, or about 10% (v/v) or lower, or about 5% (v/v) or lower.

In some embodiments, the quenching process may further provide a stabilized liposomal composition containing liposomes that encapsulate the active agent.

### Filtration and finishing

As discussed above, in some embodiments, an impinging stream undergoes mixing, collection, an incubation process, and a quenching process. The quenched material may be a stabilized liposomal composition which is output for filtration and finishing.

Fig. 5 shows a process diagram for certain embodiments for preparing liposomal compositions of this disclosure by filtration and finishing. Referring to Fig. 5, a stabilized liposomal composition, such as the quenched liposomal composition **460,** is charged to a reservoir **500.** A diafiltration buffer solution is maintained in a separate reservoir **520.** A peristaltic pump **502** provides circulation of the stabilized liposomal composition from reservoir **500** through a tube containing a hollow fiber membrane **504.** Tangential flow filtration occurs via this circulation to concentrate the stabilized liposomal composition, and filtrate **530** is removed from the tube containing the hollow fiber membrane **504.** The addition of replacement buffer from reservoir **520** to the circulation allows for diafiltration at a fixed or variable volume. The stabilized liposomal composition may also be diluted by the addition of buffer to attain the final concentration of the active agent in the formulation. Optionally, dialysis of the stabilized liposomal composition may be performed using peristaltic pump **506** to drive the dialysis buffer solution from reservoir **540.** The stabilized liposomal composition at a particular concentration is output **550** to a finishing process.

In general, the filtrate **530** may contain organic solvent and unencapsulated active agent. Thus, the removal of filtrate may remove and reduce the concentration of the organic solvent in the stabilized liposomal composition, as well as remove the unencapsulated active agent.

In general, the quenched incubate may have a concentration of the active agent that is below the range desirable for preparing a pharmaceutical composition. In some embodiments, the quenched incubate may have a concentration of non-aqueous solvent that is too high for preparing a pharmaceutical composition. These concentrations can be adjusted by tangential flow filtration and diafiltration, as discussed above.

In some embodiments, the quenched incubate is circulated to a hollow fiber tangential flow filtration apparatus, or cartridge or cassette tangential flow filtration apparatuses. When cycled without the addition of buffer or solvent, tangential flow filtration retains the liposomal compositions in a decreasing volume of buffer and solvent, thereby increasing its concentration.

A similar apparatus may be used in diafiltration mode to remove non-aqueous solvent and replace it with diafiltration buffer. In diafiltration mode, the volume of the circulating retentate is held essentially constant by adding diafiltration buffer. Thus, the concentration of organic solvent decreases as it enters the permeate and is removed.

The concentration of the active agent may be adjusted by the addition of buffer to the retentate of the diafiltration step to achieve a desired final concentration. The concentration-adjusted retentate may thereafter be provided to a sterilization unit in which direct flow filtration is used to sterilize the retentate product solution. The sterilized product may be used in a sterile vial-filling process, and the product vials stored at low temperature. Flash freezing, lyophilizing, and low temperature lyophilizing, and other means can be used to prepare and store the product.

In certain embodiments, to reach a desired active agent concentration the quenched incubate may be concentrated first by tangential flow filtration, followed by diafiltration to remove organic solvent, then followed by additional tangential flow filtration, and lastly dilution with buffer, solvent or diluent.

Examples of methods and materials for filtration are given Mark C. Porter, Handbook of Industrial Membrane Technology (Noyes 1990), pp. 186-87. Some aspects of filtration are given in Munir Cheryan, Ultrafiltration and Microfiltration Handbook (1998).

### Encapsulation of Active Agents

The degree of encapsulation of the active agent by the liposome particles is in general affecting by many process parameters.

In some embodiments, the degree of encapsulation of the active agent by the liposome particles after the incubation process is 50% or greater, or 60% or greater, or 70% or greater, or 80% or greater, or 90% or greater, or 95% or greater, or 96% or greater, or 97% or greater, or 98% or greater, or 99% or greater, or essentially 100%.

The active agent liposomal compositions of this disclosure in general contain liposome particles of uniform size. The liposomal particle size may be about 300 nm in diameter or less, or about 250 nm or less, or about 200 nm or less, or about 180 nm or less, or about 160 nm or less, or about 150 nm or less, or about 140 nm or less, or about 130 nm or less, or about 120 nm or less, or about 110 nm or less, or about 100 nm or less, or about 90 nm or less, or about 80 nm or less, or about 70 nm or less.

The liposomal particle size may range from about 50 nm to about 500 nm, or from about 60 nm to about 400 nm, or from about 70 nm to about 300 nm, or from about 70 nm to about 200 nm, or from about 70 nm to about 160 nm, or from about 80 nm to about 160 nm.

In some variations, the stabilized liposomal composition may contain less than about 10% of the active agent that is outside of the liposome particles and not encapsulated, or less than about 8% of the active agent that is not encapsulated, or less than about 5% of the active agent that is not encapsulated, or less than about 4% of the active agent that is not encapsulated, or less than about 3% of the active agent that is not encapsulated, or less than about 2% of the active agent that is not encapsulated, or less than about 1% of the active agent that is not encapsulated.

The level of encapsulation of the active agent in the stabilized liposomal composition may range from about 70% to about 99%, or from about 80% to about 99%, or from about 90% to about 99%, or from about 95% to about 99%. The level of encapsulation of the active agent in the stabilized liposomal composition may be essentially 100%.

### Efficient Delivery of Gene Silencing Therapeutics

This disclosure relates generally to novel compounds and compositions, as well as methods and uses thereof, for delivery of biologically active agents and drug agents. The compounds and compositions of this disclosure are useful for delivery of therapeutic agents to selected cells, tissues, organs or subjects. More particularly, this disclosure relates to the delivery of therapeutic agents, including nucleic acid agents, and methods for making and using materials containing peptides to effect delivery of biologically active agents and drug agents.

This disclosure provides a range of compounds, compositions, methods and uses for efficient systemic and local delivery of drugs and biologically active molecules. Efficient delivery can be provided by a high degree of loading of an active agent into liposomes using various carrier molecules including peptides. The compounds and compositions of this disclosure can achieve a high efficiency of delivery of an active agent.

One measure of the efficiency of delivery is the delivery efficiency ratio. As used herein, the delivery efficiency ratio is the ratio of the total mass of carrier molecules to the mass of the active agent. The lower the delivery efficiency ratio, the less is the mass of carrier material compared to active agent, and the less is the potential for unwanted toxicity and side effects. As used herein, a lower delivery efficiency ratio is more advantageous and desirable.

This invention relates generally to the fields of carriers and formulations for delivery of nucleic acids. Carriers for nucleic acids include compounds and compositions formed with peptide components including crosslinkable and cleavable peptide structures. More particularly, this invention provides crosslinkable peptide structures and cleavable peptide structures which bind with a nucleic acid to form complexes or condensate compositions.

In some embodiments, this disclosure provides complexes formed from a peptide and a nucleic acid. These complexes include core structures having a complex of a peptide and a nucleic acid, and core structures having various layers of peptides and nucleic acids. Peptides suitable for forming a complex of this invention with a nucleic acid include any cationic peptide.

In some aspects, a complex, condensate, or nanoparticle of a peptide and a nucleic acid may be loaded into a liposomal formulation. Liposomal formulations of this disclosure can provide stable delivery systems for biologically active agents and drug agents, in particular, nucleic acid agents.

In some respects, the compositions and formulations of this disclosure can provide biological activity with reduced toxicity.

Methods of using the carriers, peptides, nucleic acid constructs or complexes with peptides, and formulations of this disclosure in altering gene expression or activity are also provided, optionally in combination with cell-targeting components and other pharmaceutical formulation components.

This invention provides a range of carrier compositions for delivering a biologically active agent to a cell. More particularly, this disclosure provides a range of carrier structures that are nanometer scale in size having a nucleic acid agent condensed into small particles. The carrier particles can have increased stability in delivery, and can efficiently deliver an active agent. Formulations of carrier particles loaded into liposomes can provide increased stability and delivery efficiency.

The novel compounds and compositions of this disclosure can achieve a range of advantageous delivery efficiency ratios. In some embodiments, the compositions of this invention provide a delivery efficiency ratio for an RNAi-inducing agent of less than fifteen, or less than ten.

The compositions and methods of this disclosure may be useful for delivery of therapeutic, prophylactic, and diagnostic agents such as nucleic acids, polynucleotides, peptides, proteins, and small molecule compounds and drugs. These compositions may include nanoparticles of various diameters.

This disclosure provides novel compounds, compositions and formulations for intracellular and *in vivo* delivery of an active agent for use, ultimately, as a therapeutic, which in general maintain cytoprotection and relatively low toxicity. The compounds and compositions of this disclosure are useful for delivery of active agents to selected cells, tissues, organs or compartments in order to alter a disease state or a phenotype.

In some aspects, this disclosure provides compounds, compositions and methods to deliver RNA structures to cells to produce the response of RNA interference, antisense effects, or the regulation or modulation of genomic expression.

In some variations, this disclosure provides compounds, compositions and methods to deliver DNA structures or DNA-containing materials to cells.

As used herein, the term "peptide nucleic acid complex" refers to a peptide bound or complexed to a nucleic acid.

### Efficient delivery of RNAi-inducing and antisense agents

In some aspects, the compositions and methods of this invention provide efficient delivery of an active agent by providing carrier particles having a high concentration or density of the active agent molecules. The carrier particles can be loaded into liposomes to provide a high concentration or density of the active agent molecules in a pharmaceutical formulation.

In certain aspects, the compositions and methods of this invention provide formulations of an RNAi-inducing agent or an antisense agent having a range of delivery efficiency ratios. A formulation for an RNAi-inducing agent or an antisense agent of this disclosure may advantageously have a delivery efficiency ratio of less than fifteen, or less than twelve, or less than ten, or less than nine, or less than eight, or less than five.

In certain embodiments, efficient delivery can be achieved using a carrier particle composed of a nucleic acid agent condensed with a cationic peptide. For example, based on the charges of the cationic peptides combined with a nucleic acid agent such as an RNAi-inducing agent or an antisense agent, the carrier particle can include structures in which up to six or more peptide binding regions may bind to an active RNA agent.

In some variations, in a formulation containing carrier particles composed of a nucleic acid agent, such as an RNAi-inducing agent or an antisense agent, loaded into liposomes, the liposomes may have over 500, or over 1,000, or over 5,000, or over 6,000, or over 7,000, or over 8,000, or over 9,000, or over 10,000 or more copies of the RNAi-inducing agent or antisense agent molecules per particle.

For example, in some aspects, for spherical particles having an N:P of 2, a density of 1 g/cc, a particle volume of 1.26x10⁶ nm³, composed of peptide having MW 3781.2 (net 7 cationic charges) and duplex RNA having MW 13,500 (net 40 anionic charges), the mass of a particle is 1.26x10⁻⁹ micrograms, and the particle has 11.4 peptides per duplex RNA. In this example, the delivery efficiency ratio is the ratio of the mass of peptide to the mass of the RNA which is 3.2. The fraction of the mass of the particle represented by RNA is 0.24, the number of duplex RNA molecules in the particle is 13,369, and the number of peptide molecules per particle is 1.53 x10⁵. In other words, a formulation containing these carrier particles and no additional carrier molecules would have a delivery efficiency ratio of 3.2 and an RNAi-agent mass fraction loading of 24% based on the particles.

### Liposomal formulations

In some aspects, the carrier particles of this invention may be loaded or encapsulated in a liposomal formulation. For example, in some embodiments, carrier particles may be delivered as encapsulated in a liposomal formulation such as disclosed in U.S. Pat. Application No. 12/114,284.

In certain embodiments, a pharmaceutical formulation of carrier particles of this invention delivered as encapsulated in a liposomal formulation may increase the payload of a duplex RNA by 20-fold compared to a liposomal formulation of the RNA without the peptide carrier particle composition of this invention.

For example, in some embodiments, a pharmaceutical formulation of carrier particles of this invention delivered as encapsulated in a liposomal formulation may decrease the amount of carrier mass by 45% compared to a liposomal formulation of the RNA without the peptide carrier particle composition of this invention.

In some embodiments, a pharmaceutical formulation of carrier particles for an RNA agent includes a peptide-containing delivery system which uses peptides in delivering nucleic acids. This system can increase the payload of RNA agent which can be incorporated into a liposomal formulation. Using a peptide-containing nanoparticle, the efficiency of delivery may be enhanced, as well as the tissue distribution pattern of the delivery system. In some embodiments, the delivery system may demonstrate an increase in RNA payload up to 20-fold per liposomal particle, while reducing the total amount of carrier excipients by approximately 45 percent. In some variations, the system may achieve a 30% reduction in RNA agent dose as compared to a liposomal formulation without peptides, for example, as measured by *in vivo* knockdown of ApoB, while maintaining 85% knockdown in mouse liver and knockdown in mouse jejunum. Thus, a pharmaceutical formulation of carrier particles of this invention may significantly improve the delivery efficiency of an RNA agent, such as an siRNA, mdRNA, or an antisense agent.

### Carrier nanoparticles

In some embodiments, carrier particles of this invention can be prepared as described in U.S. Pat. Application No. 61/106,062, filed October 16, 2008.

The carrier particles of this disclosure are generally of uniform particle size. The carrier particle size may be about 300 nm in diameter or less, or about 250 nm or less, or about 200 nm or less, or about 180 nm or less, or about 160 nm or less, or about 150 nm or less, or about 140 nm or less, or about 130 nm or less, or about 120 nm or less, or about 110 nm or less, or about 100 nm or less, or about 90 nm or less, or about 80 nm or less, or about 70 nm or less.

The active agent carrier particles of this disclosure may have a range of particle sizes, for example, from about 50 nm to about 500 nm, or from about 60 nm to about 400 nm, or from about 70 nm to about 300 nm, or from about 70 nm to about 200 nm, or from about 70 nm to about 160 nm, or from about 80 nm to about 160 nm.

In some embodiments, the active agent carrier particles of this disclosure may be negatively charged. For example, carrier particles composed of an RNAi-agent and a cationic peptide may be condensed so that the particles retain a negative charge.

In some embodiments, the active agent carrier particles of this disclosure may be positively charged. For example, carrier particles composed of an RNAi-agent and a cationic peptide may be condensed so that the particles acquire a positive charge.

### Carriers and peptide binding regions

In some aspects, carrier compounds and compositions of this disclosure may be formed with peptide components that condense with a biologically active nucleic acid component by binding to the nucleic acid to form particles of nanometer dimensions.

In some embodiments, peptides suitable for carrier compositions of this disclosure are described in U.S. Pat. Application No. 61/116,258, filed November 19, 2008.

A carrier may be formed when one peptide having one or more binding regions binds to a nucleic acid.

In certain variations, more than one cationic binding region of a peptide may bind to the same or different nucleic acid molecules.

The crosslinkable and cleavable peptide structures of this disclosure may advantageously have a plurality of cationic residues which are distributed along the peptide chain in one or more binding regions. Variation of the number and distribution of cationic residues can be used vary the strength of binding of the peptide to an active agent.

Peptides of this invention include a cationic peptide having a binding region with sufficient positive charge to bind to a nucleic acid and one or more linker groups. A binding region of a peptide of this invention may have sufficient positive charge to bind to a nucleic acid. Linker groups can link to each other to crosslink two or more peptides into a single molecule.

Peptides capable of condensing with an active nucleic acid agent to form a carrier particle of this disclosure may have sufficient positive charge to bind to a nucleic acid and sufficient linker groups to form a self-crosslinked construct that includes a bound nucleic acid.

This disclosure provides peptides having sufficient positively-charged residues to bind to a nucleic acid, and being capable of forming a crosslinked peptide.

In some embodiments, the biologically active agent is a nucleic acid agent which can bind with a cationic peptide. A nucleic acid agent may bind one, two, three, four, five, or six peptides, or more, to form a complex. A condensate particle may be formed by aggregation and binding of nucleic acid-peptide complexes.

In some embodiments, a nucleic acid agent may bind portions of more than one peptide such that the peptide attaches to more than one nucleic acid agent.

Carrier structures or constructs can be formed by admixing a crosslinkable or cleavable peptide of this invention with a biologically active agent to which the peptide binds. Binding of the peptide to the agent can be performed at the same time as crosslinking of the peptide occurs, or before or after the peptides are crosslinked.

In some aspects, the carrier is a crosslinked peptide construct which may be a condensate of a peptide and a nucleic acid. The condensate may form a carrier particle of nanometer dimension which can incorporate a biologically active agent such as a nucleic acid.

### Crosslinkable peptides

In some embodiments, the crosslinkable peptides of this invention may contain a crosslinkable terminal residue or group.

For example, a crosslinkable peptide may have a single terminal cysteine residue which may crosslink by forming an interpeptide disulfide bond resulting in dimers of the peptide.

In some variations, the peptides may contain one or more sulfhydryl groups which can crosslink to form a multimeric peptide construct which binds to, and may be a carrier for a biologically active agent.

In some embodiments, a crosslinkable group may form a cleavable crosslink that may be cleaved at low pH or may be cleaved by the action of a protein or enzyme. Examples of cleavable crosslinks include chemically-cleavable acid labile crosslinks and enzyme-cleavable crosslinks.

Examples of crosslinkable groups include organic groups having up to 1000 atoms, a bifunctional linker, a bifunctional crosslinker, and a heterobifunctional linker. The crosslinkable groups may be substituents of a peptide residue, or may be attached at the terminus of the peptide.

In certain embodiments, crosslinkable peptide structures include peptides having crosslinkable groups at each terminus. In some variations, crosslinkable peptide structures include dimers, trimers, and multimers of peptides having crosslinkable groups at each terminus.

### Cleavable peptides

In some aspects, this disclosure provides cleavable peptides containing an internal cleavable linker group located between portions of a peptide sequence.

In some embodiments, a cleavable peptide may have two cationic binding regions linked together by a cleavable group. The cleavable group may be cleaved to detach various binding regions of the peptide from each other.

The cationic binding regions may bind to a biologically active agent such as a nucleic acid.

In some variations, cleavage of the linker group of the peptide to detach the binding regions can allow more rapid dissociation of a peptide from a biologically active agent compared to a peptide that would not be cleaved.

An intracellularly-cleavable linker may be cleaved by chemical reduction, or by the action of various proteins or enzymes in the intracellular environment.

### Condensate particles and releasable forms

Compounds and compositions of this disclosure include condensate particles or carriers composed of one or more peptide components and one or more active agents.

In general, condensate particles formed with a peptide and an active agent may be anionic, neutral, or cationic. For delivery of the carrier particles *in vivo,* a neutral or cationic form may be preferred. A condensate particle may be referred to as a core particle.

In some embodiments, a condensate particle may be formed with a first portion of a crosslinkable peptide and an active agent. One or more additional layers of the same or different crosslinkable peptide may be added to the particle.

In some variations, a condensate particle may be formed with a first portion of a cleavable peptide and an active agent. One or more additional layers of the same or different cleavable peptide may be added to the particle.

In certain embodiments, a condensate particle may be formed with a first portion of a cleavable peptide and an active agent. One or more additional layers of a crosslinkable peptide may be added to the particle.

In some variations, a condensate particle may be formed with a first portion of a crosslinkable peptide and an active agent. One or more additional layers of a cleavable peptide may be added to the particle.

In some embodiments, a condensate particle that is anionic may be formed with a first portion of a crosslinkable or cleavable peptide and an active nucleic acid agent. An additional layer or layers of a cationic crosslinkable or cleavable peptide may be added to the anionic particle to form a neutral or cationic carrier particle.

In certain variations, a condensate particle that is anionic may be formed with a first portion of a crosslinkable or cleavable peptide and an active nucleic acid agent. An additional layer or layers of a cationic crosslinkable or cleavable peptide may be added to the anionic particle to form a neutral or cationic carrier particle. An additional layer or layers of an anionic endosomolytic compound may be added to the neutral or cationic carrier particle to form a layered neutral or cationic carrier particle.

In some aspects, the active agents may be one or more drug compounds, one or more antisense agents, one or more RNAi-inducing agents, or one or more DNA-containing agents.

In some embodiments, a composition or formulation of this disclosure may be prepared by loading condensate particles or layered carrier particles into cationic liposomes.

In some embodiments, the compositions and methods of this disclosure may provide delivery of therapeutic agents in releasable forms or compositions. Releasable forms and compositions include molecules that bind and release an active agent, molecules that bind an active agent and discharge a moiety that assists in release of the agent, molecules that bind an active agent and are subsequently modulated in form within a biological compartment to assist in release of the agent, and compositions containing molecules that bind an active agent admixed with a release mediator compound.

As used herein, releasable forms include those containing a crosslinkable or cleavable peptide of this disclosure, or a form containing an endosomolytic compound or material.

A condensate or carrier particle may contain a cleavable peptide structure or matrix. Cleavage of a peptide structure can be triggered by certain events such as entry of the carrier into a biological environment or compartment containing a compound which can cleave the peptide crosslinks. Cleavage of peptide linker groups can occur intracellularly in the cytosol or in various cellular or extracellular compartments.

Cleavage of disulfide peptide linker groups can be done chemically, for example, by reduction of the disulfide with tris(2-carboxyethyl) phosphine hydrochloride (TCEP), dithiothreitol (DTT), or mercaptoethanol.

In certain embodiments, a disulfide reductase may be used to cleave peptide disulfide bonds.

Once within a cell, disulfide crosslinks may be reduced, thereby releasing an active agent for efficient delivery. The environment of the endosome is believed to be reducing and to mediate disulfide reduction and release of the active agent.

Release within a cell can occur by disruption or cleavage of the peptide crosslinks, as well as by dissociation of the biologically active agent from the peptide.

The peptides and peptide constructs of this invention may advantageously contain one, two, or more binding regions having one or more positively-charged amino acid residues. The binding regions can be attached in a chain where one positively-charged binding region is cleavably-linked to the next binding region by a cleavable crosslink.

The cationic regions may serve as binding regions for an active agent, such as a nucleic acid agent, and several cationic regions may bind to the same active agent to cooperatively attach the peptide to the active agent.

In certain embodiments, a releasable form of this disclosure includes a condensate particle of a peptide and a nucleic acid, where the peptide component includes crosslinks that can be cleaved to effect release of the nucleic acid. Cleavage of linker groups of the peptides may be triggered by a change in the environment of the peptide such as would occur in transport from extracellular to intracellular domains, or during endocytosis or uptake and delivery of endosomes by cells.

Examples of cleavable linkers for peptides include acid-cleavable groups such as hydrazone which may be cleaved during endocytosis or through intracellular interaction with lysosomes.

In some embodiments, release of the active agent may be provided by an acid-labile linker.

Examples of acid-labile linkers include linkers containing an orthoester group, a hydrazone, a cis-acetonyl, an acetal, a ketal, a silyl ether, a silazane, an imine, a citriconic anhydride, a maleic anhydride, a crown ether, an azacrown ether, a thiacrown ether, a dithiobenzyl group, a cis-aconitic acid, a cis-carboxylic alkatriene, methacylic acid, and mixtures thereof.

Examples of acid-labile groups and linkers are given in U.S. Patent Nos. 7,098,032; 6,897,196; 6,426,086; 7,138,382; 5,563,250; and 5,505,931.

Examples of cleavable linkers for peptides include Cathepsin-cleavable linkers such as Val-Cit which may be cleaved by intracellular Cathepsins. Examples of substrate sequences for Cathepsin B, D, and L are shown in Tables 1, 2, and 3, respectively. Cleavable linkers include di-, tri-, and tetrapeptide subunits of Cathepsin B, D, and L substrates (P2-P2').

**Table 1: Cathepsin B substrates**

| SEQ ID NO: | P4 | P3 | P2 | P1 | P1' | P2' | P3' | P4' |
|---|---|---|---|---|---|---|---|---|
| 1 | - | Abz | Phe | Arg | Ala | Lyd | - | - |
| 2 | - | Abz | Phe | Arg | Lyd | Trp | - | - |
| 3 | - | Abz | Phe | Arg | Nph | Phe | - | - |
| 4 | - | Abz | Phe | Arg | Phe | Lyd | - | - |
| 5 | Asn | Phe | Phe | Gly | Val | Gly | Gly | Glu |
| 6 | Cys | Pro | Val | Thr | Tyr | Gly | Gln | Cys |
| 7 | Gin | Ala | Ser | Arg | Ser | Phe | Asn | Gin |
| 8 | Ser | Arg | Ser | Phe | Asn | Gln | Gly | Arg |
| 9 | Ala | Ser | Arg | Ser | Phe | Asn | Gln | Gly |
| 10 | Boc | Gly | Arg | Arg | AMC | - | - | - |
| 11 | - | - | Bz | Arg | NH2 | - | - | - |
| 12 | - | - | Bz | Gly | Arg | - | - | - |
| 13 | Tyr | Leu | Lys | Arg | Leu | Cys | Gly | Thr |
| 14 | Lys | Arg | Leu | Cys | Gly | Thr | Phe | Leu |
| 15 | Phe | Val | Asn | Gln | His | Leu | Cya | Gly |
| 16 | Leu | Cya | Gly | Ser | His | Leu | Val | Glu |
| 17 | His | Leu | Val | Glu | Ala | Leu | Tyr | Leu |
| 18 | Val | Glu | Ala | Leu | Tyr | Leu | Val | Cya |
| 19 | Glu | Ala | Leu | Tyr | Leu | Val | Cya | Gly |
| 20 | Leu | Tyr | Leu | Val | Cya | Gly | Glu | Arg |
| 21 | Val | Cya | Gly | Glu | Arg | Gly | Phe | Phe |
| 22 | Gly | Glu | Arg | Gly | Phe | Phe | Tyr | Thr |
| 23 | Gly | Phe | Phe | Tyr | Thr | Pro | Lys | Ala |
| 24 | Leu | Lys | Pro | Ala | Lys | Ser | Ala | Arg |
| 25 | Ala | Pro | Leu | Lys | Pro | Ala | Lys | Ser |
| 26 | Lys | Pro | Ala | Lys | Ser | Ala | Arg | Ser |
| 27 | Lys | Leu | Ser | Gly | Phe | Ser | Phe | Lys |
| 28 | Lys | Ser | Phe | Lys | Leu | Ser | Gly | Phe |
| 29 | Ala | Tyr | Arg | Arg | Phe | Tyr | Gly | Pro |
| 30 | Gin | Trp | Leu | Gly | Ala | Pro | Val | Pro |
| 31 | Met | Lys | Leu | Thr | Leu | Lys | Gly | Gly |
| 32 | Lys | Lys | Leu | Thr | Val | Asn | Pro | Gly |
| 33 | Leu | Ser | Lys | Lys | Val | Lys | Asn | Met |
| 34 | Thr | Phe | Leu | Arg | Leu | Ala | Ala | Leu |
| 35 | Ser | Leu | Asn | His | Tyr | Ala | Gly | Tyr |
| 36 | Leu | Leu | Val | Tyr | AMC | - | - | - |
| 37 | Arg | Glu | Ala | Ala | Ser | Gly | Asn | Phe |
| 38 | Pro | Thr | Val | Gly | Ser | Phe | Gly | Phe |
| 39 | Glu | Val | Asp | Leu | Leu | Ile | Gly | Ser |
| 40 | Pro | Arg | Phe | Lys | Ile | Ile | Gly | Gly |
| 41 | - | Z | Arg | Arg | AMC | - | - | - |
| 42 | - | Z | Arg | Arg | NAN | - | - | - |
| 43 | - | Z | Leu | Arg | AMC | - | - | - |
| 44 | - | Z | Phe | Arg | AMC | - | - | - |
| 45 | - | Z | Phe | Arg | AMC | - | - | - |
| 46 | - | Z | Phe | Arg | NAN | - | - | - |

**Table 2: Cathepsin D substrates**

| SEQ ID NO: | P4 | P3 | P2 | P1 | P1' | P2' | P3' | P4' |
|---|---|---|---|---|---|---|---|---|
| 47 | Abz | Ile | Glu | Phe | Nph | Arg | Leu | NH2 |
| 48 | Leu | Leu | Ser | Ala | Leu | Val | Glu | Thr |
| 49 | Ile | Thr | Leu | Leu | Ser | Ala | Leu | Val |
| 50 | Leu | Ser | Ala | Leu | Val | Glu | Thr | Arg |
| 51 | Val | Val | Ile | Ala | Thr | Val | Ile | Val |
| 52 | Ile | Ile | Gly | Leu | Met | Val | Gly | Gly |
| 53 | Val | Ile | Thr | Leu | Val | Met | Leu | Lys |
| 54 | Lys | Leu | Val | Phe | Phe | Ala | Glu | Asp |
| 55 | Leu | Val | Phe | Phe | Ala | Glu | Asp | Val |
| 56 | Thr | Tyr | Lys | Phe | Phe | Glu | Gln | Met |
| 57 | Val | Ile | Ala | Thr | Val | Ile | Val | Ile |
| 58 | Ile | Val | Ile | Thr | Leu | Val | Met | Leu |
| 59 | Leu | Gly | Asp | Phe | Phe | Arg | Lys | Ser |
| 60 | Ile | Lys | Asp | Phe | Leu | Arg | Asn | Leu |
| 61 | Gly | Tyr | Asp | Leu | Ser | Phe | Leu | Pro |
| 62 | Ala | Pro | Gly | Phe | Leu | Gly | Leu | Pro |
| 63 | Thr | Met | Thr | Leu | Ser | Lys | Ser | Thr |
| 64 | Asn | Tyr | Phe | Leu | Asp | Val | Glu | Leu |
| 65 | Ala | Leu | Asp | Phe | Ala | Val | Gly | Glu |
| 66 | Phe | Gln | Ile | Tyr | Ala | Val | Pro | Trp |
| 67 | Lys | Asp | Val | Leu | Asp | Ser | Val | Leu |
| 68 | Val | Glu | Asp | Leu | Glu | Ser | Val | Gly |
| 69 | Gly | Asn | Phe | Lys | Ser | Gln | Leu | Gln |
| 70 | Trp | Gly | Thr | Phe | Glu | Glu | Val | Ser |
| 71 | Leu | Gly | Glu | Phe | Val | Ser | Glu | Thr |
| 72 | Ser | His | Cys | Leu | Leu | Val | Thr | Leu |
| 73 | Leu | Val | Thr | Leu | Ala | Ala | His | Leu |
| 74 | Ser | Thr | Val | Leu | Thr | Ser | Lys | Tyr |
| 75 | Ala | Glu | Ala | Leu | Glu | Arg | Met | Phe |
| 76 | Leu | Glu | Arg | Met | Phe | Leu | Ser | Phe |
| 77 | Leu | Asp | Lys | Phe | Leu | Ala | Ser | Val |
| 78 | Glu | Arg | Met | Phe | Leu | Ser | Phe | Pro |
| 79 | Phe | Leu | Ser | Phe | Pro | Thr | Thr | Lys |
| 80 | Ala | Asn | Val | Ser | Thr | Val | Leu | Thr |
| 81 | Ala | Ser | Val | Ser | Thr | Val | Leu | Thr |
| 82 | Leu | Leu | Val | Thr | Leu | Ala | Ser | His |
| 83 | Leu | Leu | Val | Thr | Leu | Ala | Ala | His |
| 84 | Ala | Ala | Glu | Tyr | Gly | Ala | Glu | Ala |
| 85 | - | - | - | Val | Leu | Ser | Ala | Ala |
| 86 | Val | Gln | Ala | Ala | Tyr | Gln | Lys | Val |
| 87 | Thr | Ala | Glu | Glu | Lys | Ala | Ala | Val |
| 88 | Val | Thr | Ala | Leu | Trp | Gly | Lys | Val |
| 89 | - | Val | His | Leu | Thr | Pro | Glu | Glu |
| 90 | Leu | Gly | Arg | Leu | Leu | Val | Val | Tyr |
| 91 | Leu | Gly | Arg | Leu | Leu | Val | Val | Tyr |
| 92 | Gly | Arg | Leu | Leu | Val | Val | Tyr | Pro |
| 93 | Gly | Arg | Leu | Leu | Val | Val | Tyr | Pro |
| 94 | Val | Thr | Ala | Phe | Trp | Gly | Lys | Val |
| 95 | Thr | Gln | Arg | Phe | Phe | Glu | Ser | Phe |
| 96 | Thr | Gln | Arg | Phe | Phe | Glu | Ser | Phe |
| 97 | Phe | Glu | Ser | Phe | Gly | Asp | Leu | Ser |
| 98 | Phe | Glu | Ser | Phe | Gly | Asp | Leu | Ser |
| 99 | Lys | Gly | Thr | Phe | Ala | Thr | Leu | Ser |
| 100 | Thr | Ala | Leu | Trp | Gly | Lys | Val | Asn |
| 101 | Ala | Asp | Ala | Val | Met | Asn | Asn | Pro |
| 102 | Val | Glu | Ala | Leu | Tyr | Leu | Val | Cya |
| 103 | Ala | Leu | Tyr | Leu | Val | Cya | Gly | Glu |
| 104 | Glu | Arg | Gly | Phe | Phe | Tyr | Thr | Pro |
| 105 | Arg | Leu | Arg | Ala | Tyr | Leu | Leu | Pro |
| 106 | Leu | Lys | Phe | Leu | Asn | Val | Leu | Ser |
| 107 | Ser | Gln | Arg | Tyr | Lys | Val | Asp | Tyr |
| 108 | Lys | Val | Asp | Tyr | Glu | Ser | Gin | Ser |
| 109 | Ser | Gly | Gly | Lys | Met | Lys | Val | Asn |
| 110 | Arg | Pro | Phe | Leu | Val | Val | Ile | Phe |
| 111 | Ala | Ile | Lys | Phe | Phe | Ser | Ala | Gln |
| 112 | Ile | Lys | Phe | Phe | Ser | Ala | Gln | Thr |
| 113 | Ile | Thr | Lys | Leu | Asn | Ala | Glu | Asn |
| 114 | Ala | Gly | Lys | Lys | Tyr | Phe | Ile | Asp |
| 115 | Phe | Ile | Asp | Phe | Val | Ala | Arg | Glu |
| 116 | Pro | Tyr | Ile | Leu | Lys | Arg | Gly | Ser |
| 117 | Phe | Gln | Glu | Ala | Tyr | Arg | Arg | Phe |
| 118 | Leu | Leu | Lys | Glu | Ala | Gin | Leu | Pro |
| 119 | Val | Val | Leu | Leu | Pro | Asp | Val | Glu |
| 120 | Asp | Val | Val | Leu | Phe | Glu | Lys | Lys |
| 121 | Gly | Met | Glu | Leu | Ile | Val | Ser | Gln |
| 122 | Tyr | Pro | Val | Trp | Ser | Gly | Leu | Pro |
| 123 | Asn | Glu | Ile | Tyr | Pro | Val | Trp | Ser |
| 124 | Phe | Ile | Val | Gly | Phe | Thr | Arg | Gln |
| 125 | Ala | Asn | Pro | Lys | Gln | Thr | Trp | Val |
| 126 | His | Pro | Lys | Phe | Ile | Val | Gly | Phe |
| 127 | Lys | Gln | Thr | Trp | Val | Lys | Tyr | Ile |
| 128 | Trp | Val | Lys | Tyr | Ile | Val | Arg | Leu |
| 129 | Pro | Lys | Glu | Leu | Trp | Val | Gln | Gln |
| 130 | Leu | Arg | Tyr | Asp | Thr | Glu | Tyr | Tyr |
| 131 | Lys | Ile | Leu | Gly | Cys | Asp | Trp | Tyr |
| 132 | Asp | Val | Gln | Leu | Lys | Asn | Ile | Thr |
| 133 | Phe | Asn | Asn | Leu | Asp | Arg | Ile | Leu |
| 134 | Gln | Leu | Lys | Leu | Tyr | Asp | Asp | Lys |
| 135 | Ser | Leu | Gly | Leu | Val | Gly | Thr | His |
| 136 | Arg | Asp | Ile | Leu | Ile | Ala | Ser | Asn |
| 137 | Thr | Asp | Tyr | Met | Tyr | Leu | Thr | Asn |
| 138 | Ser | Ile | Thr | Phe | Leu | Arg | Asp | Phe |
| 139 | Gly | Leu | Lys | Phe | Ile | Ile | Lys | Arg |
| 140 | Ile | Asp | Ser | Phe | Val | Lys | Ser | Gly |
| 141 | Glu | Ile | Asp | Ser | Phe | Val | Lys | Ser |
| 142 | Lys | Thr | Tyr | Ser | Val | Gln | Leu | Lys |
| 143 | Ala | Ser | Asn | Trp | Tyr | Phe | Asn | His |
| 144 | Gly | Cys | Asp | Trp | Tyr | Phe | Val | Pro |
| 145 | Asp | Thr | Glu | Tyr | Tyr | Leu | Ile | Pro |
| 146 | Ile | Thr | Asp | Tyr | Met | Tyr | Leu | Thr |
| 147 | Asp | Tyr | Met | Tyr | Leu | Thr | Asn | Ala |
| 148 | Leu | Asn | Ile | Tyr | Tyr | Arg | Arg | Leu |
| 149 | Ile | Pro | Leu | Tyr | Lys | Lys | Met | Glu |
| 150 | Lys | Phe | Leu | Ala | Ser | Leu | Leu | Glu |
| 151 | Thr | Thr | Glu | Leu | Phe | Ser | Pro | Val |
| 152 | Asp | Gly | His | Phe | Leu | Arg | Glu | Pro |
| 153 | Phe | Ser | His | Phe | Ile | Arg | Ser | Gly |

**Table 3: Cathepsin L substrates**

| SEQ ID NO: | P4 | P3 | P2 | P1 | P1' | P2' | P3' | P4' |
|---|---|---|---|---|---|---|---|---|
| 154 | - | Abz | Phe | Arg | Ala | Lyd | NH2 | - |
| 155 | Met | Phe | Leu | Glu | Ala | Ile | Pro | Met |
| 156 | Ala | Ile | Pro | Met | Ser | Ile | Pro | Pro |
| 157 | Cys | Pro | Val | Thr | Tyr | Gly | Gln | Cys |
| 158 | Gin | Ala | Ser | Arg | Ser | Phe | Asn | Gln |
| 159 | Lys | Val | Phe | Gln | Glu | Pro | Leu | Phe |
| 160 | Leu | Phe | Tyr | Glu | Ala | Pro | Arg | Ser |
| 161 | Ala | Thr | Leu | Thr | Phe | Asp | His | Ser |
| 162 | Pro | Leu | Phe | Tyr | Glu | Ala | Pro | Arg |
| 163 | Gin | Gly | Phe | Gin | Gly | Pro | Hyp | Gly |
| 164 | Gly | Pro | Arg | Gly | Leu | Hyp | Gly | Pro |
| 165 | Gly | Pro | Hyp | Gly | Ala | Hyp | Gly | Pro |
| 166 | Arg | Leu | Val | Gly | Gly | Pro | Met | Asp |
| 167 | Thr | Gly | Leu | Arg | Asp | Pro | Phe | Asn |
| 168 | Lys | Ile | Leu | His | Leu | Pro | Thr | Ser |
| 169 | Ala | His | Leu | Lys | Asn | Ser | Gln | Glu |
| 170 | Ile | Gln | Gln | Lys | Ile | Leu | His | Leu |
| 171 | Ala | Pro | Leu | Thr | Ala | Glu | Ile | Gln |
| 172 | Ile | Met | Phe | Thr | Ser | Leu | Pro | Leu |
| 173 | - | Cap | Leu | CyB | AMC | - | - | - |
| 174 | - | Cap | Leu | Phe | AMC | - | - | - |
| 175 | - | Cap | Leu | ThB | AMC | - | - | - |
| 176 | Glu | His | Tyr | Gln | Lys | Lys | Phe | Lys |
| 177 | - | Phe | Val | Asn | Gln | His | Leu | Cya |
| 178 | His | Leu | Val | Glu | Ala | Leu | Tyr | Leu |
| 179 | Ala | Leu | Tyr | Leu | Val | Cya | Gly | Glu |
| 180 | Arg | Gly | Phe | Phe | Tyr | Thr | Pro | Lys |
| 181 | Gly | Phe | Phe | Tyr | Thr | Pro | Lys | Ala |
| 182 | His | Ser | Lys | Ile | Ile | Ile | Ile | Lys |
| 183 | Val | Leu | Pro | Arg | Ser | Ala | Lys | Glu |
| 184 | Glu | Ala | Tyr | Arg | Arg | Phe | Tyr | Gly |
| 185 | Gln | Trp | Leu | Gly | Ala | Pro | Val | Pro |
| 186 | Leu | Ser | Leu | Ala | His | Thr | His | Gln |
| 187 | Lys | Leu | Leu | Ala | Val | Ser | Gly | Pro |
| 188 | Gin | Leu | Phe | Arg | Arg | Ala | Val | Leu |
| 189 | Glu | Phe | Ser | Arg | Lys | Val | Pro | Thr |
| 190 | Leu | Leu | Ile | Gly | Ser | Ser | Gln | Asp |
| 191 | Pro | Arg | Phe | Lys | Ile | Ile | Gly | Gly |
| 192 | - | Z | Leu | Arg | AMC | - | - | - |
| 193 | - | Z | Phe | Arg | AMC | - | - | - |
| 194 | - | - | Tyr | Gly | Gly | Phe | Met | - |

In some variations, a releasable form of this disclosure includes a condensate particle of a peptide and a nucleic acid and an endosomolytic compound. In these variations, an endosomolytic compound can assist in release of the core particle and active agent into the cell from an endosome, while the peptide component can include crosslinks that may be cleaved to effect release and dissociation of the nucleic acid from the core condensate particle within the cell.

Examples of endosomolytic compounds include Chloroquin, 4-aminoquinoline, aminoquinoline, Amodiaquine, cell penetrating peptides, Transportan, Penetratin, a hemagglutinin fusion peptide from influenza virus (see for example Han et al., Nat. Struct. Biol. Vol. 8, 715-720, 2001), and influenza-based peptide diINF7.

In certain embodiments, carrier particles or constructs can be formulated with a targeting agent for cellular or sub-cellular delivery. In some variations, a carrier particle may be combined with a synthetic polymer such as polyethylene glycol (PEG) to reduce non-specific effects or interaction with blood components. A suitable synthetic polymer includes a polyethylene glycol chain (PEG), or a PEG copolymer such as PEG-polyurethane or PEG-polypropylene. See, e.g., J. Milton Harris, Poly(ethylene glycol) chemistry: biotechnical and biomedical applications (1992).

### Methods of use

This disclosure includes a method for delivering a therapeutic nucleic acid to a cell comprising preparing a composition containing a carrier particle containing a nucleic acid agent and treating a cell with the composition.

This disclosure includes a method for inhibiting expression of a gene in a cell comprising preparing a composition containing a carrier particle containing a nucleic acid agent and treating a cell with the composition.

This disclosure includes a method for inhibiting expression of a gene in a mammal comprising preparing a composition containing a carrier particle containing a nucleic acid agent and administering the composition to the mammal.

This disclosure includes a method for treating a disease in a human, the disease being selected from inflammatory diseases including rheumatoid arthritis, metabolic diseases including hypercholesterolemia, liver disease, encephalitis, bone fracture, heart disease, viral disease including hepatitis and influenza, and cancer, comprising preparing a liposomal composition and administering the composition to the human.

### Active agents

In some aspects, this disclosure provides methods for making compositions suitable for delivery of therapeutic agents. The methods of this disclosure may provide compositions of nucleic acid agents, such as condensed RNA nanoparticles, two- or three-stranded RNA structures, RNA peptide conjugates, dicer substrate RNAs, dsRNAs, siRNAs, microRNAs, hairpin RNAs, other active and regulatory RNA forms, antisense therapeutic forms including antisense RNA and DNA, and DNA and DNA-containing forms.

The active agent of this disclosure may be a single-stranded or double-stranded nucleic acid. The active agent of this disclosure may be an antigenic or immunogenic protein or polypeptide.

The active agent of this disclosure may be a peptide condensate of an active agent. For example, an active agent may be composed of nanoparticles formed by condensing an active agent with a peptide or other biomolecule, or a condensate or complex of an active agent with a peptide, biomolecule, or polymeric molecule. Nanoparticles or condensates may be crosslinked. Nanoparticles or condensates can be loaded as cargo into a liposomal composition.

The active agent of this disclosure may be an antisense or sense, DNA or RNA oligonucleotide, or a modified DNA or RNA oligonucleotide which binds to target nucleic acid sequence to block transcription or translation of the target sequence by various interactions. An antisense or sense agent may form a triple helix with a nucleotide double helix, or may be a ribozyme, or may encode transcriptional or translational regulatory sequences including promoter sequences or enhancer sequences. An antisense or sense oligonucleotide may be used to block expression of a protein and may have modified nucleobases or sugar groups, or other groups, or may be a conjugate with a biomolecule, peptide, or protein, for enhanced stability or activity. An antisense or sense oligonucleotide may be delivered into a cell containing its target nucleic acid by the compositions and methods described herein. An antisense or sense oligonucleotide may be delivered into a cell containing its target nucleic acid using an oligonucleotide-carrier complex, or a liposomal formulation, as described herein.

### Crosslinkable and cleavable peptides

Crosslinkable peptides of this invention include those having the structure shown in Formula I:

A-B Formula I

where A is a peptide of from two to about 16 amino acid residues which may contain a cationic binding region, and B is a crosslinkable group, wherein A contains one or more positively charged residues at pH 7.

Examples of B include cysteine.

Other examples of B include organic groups having up to 1000 atoms, a bifunctional linker, a bifunctional crosslinker, a heterobifunctional linker, a carbamate, and an ester.

Examples of A include cationic peptides.

Examples of A include cationic peptides having the structure shown in Formula II:

(Xaa¹)ₘ-(Xaa²)ₙ-(Xaa³)ₒ-(Xaa⁴)ₚ Formula II

where Xaa is an amino acid residue, each of Xaa¹, Xaa², Xaa³, and Xaa⁴ are independently selected amino acid residues which are the same or different, each of m, n, o, and p is from zero to four provided that the sum of m, n, o, and p is two or more, wherein one or more of Xaa¹, Xaa², Xaa³, and Xaa⁴ is a positively charged residue at pH 7.

Cationic peptides can be prepared where, for example, a residue of A has a basic side chain. Examples of amino acids having a basic side chain include arginine (Arg), homoarginine (homoArg) (side chain -(CH₂)₄NH(C=NH)NH₂), norarginine (norArg) (side chain -(CH₂)₂NH(C=NH)NH₂), nor-norarginine (nornorArg) (side chain -(CH₂)NH(C=NH)NH₂), ornithine, lysine, homolysine, histidine, 1-methylhistidine, pyridylalanine (Pal), asparagine, N-ethylasparagine, glutamine, and 4-aminophenylalanine, and side chain modified derivatives thereof.

As used herein, the term "homo," when referring to an amino acid, means that an additional carbon is added to the side chain, while the term "nor," when referring to an amino acid, means that a carbon is subtracted from the side chain. Thus, homolysine refers to side chain-(CH₂)₅NH₂.

Cationic peptides can also be prepared where the side chain of a residue contains an ionizable group or substituent.

In some embodiments, the cationic residue is N^{G}-methylarginine, symmetric or asymmetric N^{G},N^{G}-dimethylarginine, N^{G}-methyl-homoarginine, symmetric or asymmetric N^{G},N^{G}-dimethyl-homoarginine, N^{G}-methyl-norarginine, symmetric or asymmetric N^{G},N^{G}-dimethyl-norarginine, or N^{G}-methyl-nor-norarginine, symmetric or asymmetric N^{G},N^{G}-dimethyl-nor-norarginine.

In some embodiments, the cationic residue is N^{G}-ethylarginine, symmetric or asymmetric N^{G},N^{G}-diethylarginine, N^{G}-ethyl-homoarginine, symmetric or asymmetric N^{G},N^{G}-diethyl-homoarginine, N^{G}-ethyl-norarginine, symmetric or asymmetric N^{G},N^{G}-diethyl-norarginine, or N^{G}-ethyl-nor-norarginine, symmetric or asymmetric N^{G},N^{G}-diethyl-nor-norarginine.

In certain embodiments, the cationic residue is N^{G}-alkylarginine, symmetric or asymmetric N^{G},N^{G}-dialkylarginine, N^{G}-alkyl-homoarginine, symmetric or asymmetric N^{G},N^{G}-dialkyl-homoarginine, N^{G}-alkyl-norarginine, symmetric or asymmetric N^{G},N^{G}-dialkyl-norarginine, or N^{G}-alkyl-nor-norarginine, symmetric or asymmetric N^{G},N^{G}-dialkyl-nor-norarginine.

In some embodiments, the cationic residue is an amino acid having a guanidine-or amidine-containing side chain. For example, the side chain of the Xaa residue may contain a group such as guanido, amidino, dihydroimidazole, 4-guanido-phenyl, 4-amidino-phenyl, N-amidino-piperidine, N-amidino-piperazine, 4,5-dihydroimidazole, 2-(N-amidino)-pyrrolidinyl, or 4-[(2-aminopyrimidinyl)]ethyl.

Examples of cationic residues may have side chains that include the following structures, as well as their salt forms:

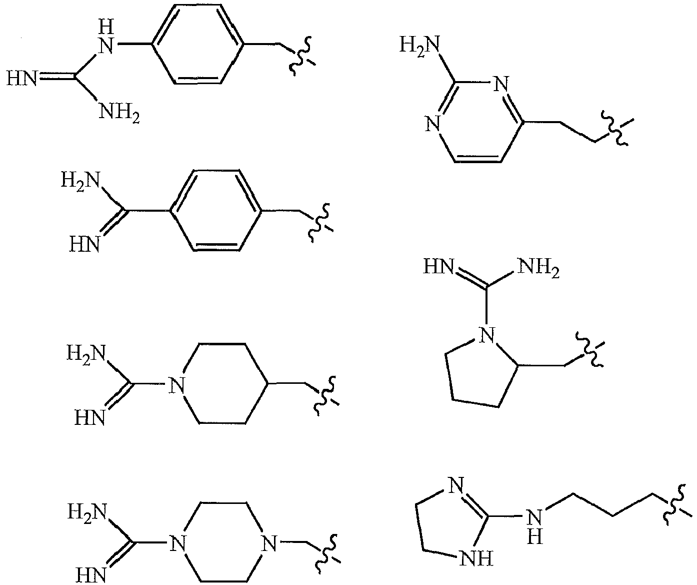

Cleavable peptides of this invention include those which are dimers of the structure shown in Formula I, for example, the dimer A-B-B-A, wherein the linker groups B are capable of linking to each other, and where the linkage -B-B- can be cleaved.

For example, the dimer A-B-B-A may be A-B-(S-S)-B-A where (S-S) is a disulfide linkage.

Other examples of linkage -B-B- include organic groups having up to 1000 atoms, a linkage formed with a bifunctional linker, a linkage formed with a bifunctional crosslinker, a linkage formed with a heterobifunctional linker, a hydrzone linker, a carbamate linkage, and an ester linkage.

Crosslinkable peptides of this invention include those having the structure shown in Formula II:

B-A-B Formula II

where A is a peptide of from about two to about 16 amino acid residues, and B is a crosslinkable group as defined above, wherein A contains one or more positively charged residues at pH 7.

Examples of B include cysteine.

Examples of A include cationic peptides.

Cleavable peptides of this invention include those which are dimers, trimers, or multimers of the structure shown in Formula II, for example, the dimer B-A-B-B-A-B, and the multimer -(B-A-B)ₙ-, wherein the linker groups B are capable of linking to each other, and where the linkage -B-B- can be cleaved. Some of these cleavable peptides remain crosslinkable because they retain a crosslinkable group at each terminus.

Examples of cationic binding regions suitable for preparation of peptides of this disclosure are shown in Table 4. A crosslinkable peptide of this disclosure may have a binding region shown in Table 4 with a cysteine attached at either the N-terminus or the C-terminus of the peptide shown in Table 4. A crosslinkable peptide of this disclosure may form a dimer.

**Table 4: Binding regions for preparation of peptides**

| SEQ ID NO: | BINDING REGION |
|---|---|
| 195 | GRKKRRQRRRPPQ |
| 196 | KKKRKV |
| 197 | KKKRKVKKKRKV |
| 198 | GRKKRR |
| 199 | RRRPPQ |
| 200 | WKKKK |
| 201 | RRRPPQH |
| 202 | KKRRQH |
| 203 | RRR |
| 204 | RRRR |
| 205 | RRRRR |
| 206 | KKK |
| 207 | RRRRWW |
| 208 | RRRWW |
| 209 | RRWW |
| 210 | KKWW |
| 211 | KKKWW |
| 212 | WHHRRKK |
| 213 | RRKKHHWW |
| 214 | KKRRW |
| 215 | KKRRHW |
| 216 | KKRRHHW |
| 217 | KKRRQ |
| 218 | KKRRQ |
| 219 | GRKKRRQ |
| 220 | QGRKKRR |
| 221 | RRH |
| 222 | RRRH |
| 223 | RRRRH |
| 224 | RRRRRH |
| 225 | KKH |
| 226 | KKKH |
| 227 | HWKKRR |
| 228 | HWKKRR |
| 229 | PPHRRR |
| 230 | PPHRRR |
| 231 | GRKKRRVRRRPPQ |
| 232 | WWHHKKRRGGRRKKHHWW |
| 233 | WWHHKKRR |
| 234 | YYHHKKRR |
| 235 | RRKKHHYY |
| 236 | VQAAIDYING |
| 237 | WWRRHH |
| 238 | HHRRWW |
| 239 | YYRRHH |
| 240 | HHRRYY |
| 241 | WWRRR |
| 242 | RRRWW |
| 243 | YYRRR |
| 244 | RRRYY |
| 245 | WWRRRHH |
| 246 | HHRRRWW |
| 247 | YYRRRHH |
| 248 | HHRRRYY |
| 249 | WWRRRR |
| 250 | RRRRWW |
| 251 | YYRRRR |
| 252 | RRRRYY |
| 253 | WWRRRRHH |
| 254 | HHRRRRWW |
| 255 | YYRRRRHH |
| 256 | HHRRRRYY |
| 257 | WWHH-Orn-Orn-RR |
| 258 | WWHHHRRR |
| 259 | WWHHHRRR |
| 260 | WWWHHHHRRR |
| 261 | WWWKKRRR |
| 262 | KKKWRRW |
| 263 | WRRRWRR |
| 264 | WWHHKKRR |
| 265 | WWCHHKKCRR |
| 266 | WWHHHRRR |
| 267 | WWHHCKKRR |
| 268 | WWHHKKCRR |
| 269 | RRWWKKHH |
| 270 | WWHHKKKK |
| 271 | WWHHRRRR |
| 272 | RRRRHH |
| 273 | HHKKKK |
| 274 | HHRRRR |
| 275 | YYRRRRHH |
| 276 | YYKKKKHH |

Examples of cleavable peptides of this disclosure are shown in Table 5.

**Table 5: Cleavable Peptides**

| SEQ ID NO: | PEPTIDE |
|---|---|
| 277 | GRKKRRV-Cit-RRRPPQ |
| 278 | GRKKRRV-Cit-RRKKRG |
| 279 | RRRPPQV-Cit-PPRRR |
| 280 | RRKKRGV-Cit-GRKKRR |
| 281 | QPPRRRV-Cit-RRRPPQ |
| 282 | WKKKKV-Cit-KKKKW |
| 283 | KKKKWV-Cit-WKKKK |
| 284 | HQPPRRRV-Cit-RRRPPQH |
| 285 | QPPRRRV-Cit-RRRPPQ |
| 286 | HQRRKKV-Cit-KKRRQH |
| 287 | RRV-Cit-RR |
| 288 | RRRV-Cit-RRR |
| 289 | RRRRV-Cit-RRRR |
| 290 | RRRRRV-Cit-RRRRR |
| 291 | KKV-Cit-KK |
| 292 | KKKV-Cit-KKK |
| 293 | KKKKV-Cit-KKKK |
| 294 | KKKKKV-Cit-KKKKK |
| 295 | WWRRRRV-Cit-RRRRWW |
| 296 | WWRRRV-Cit-RRRWW |
| 297 | WWRRV-Cit-RRWW |
| 298 | WWKKV-Cit-KKWW |
| 299 | WWKKKV-Cit-KKKWW |
| 300 | WWKKKKV-Cit-KKKKWW |
| 301 | KKRRHHWV-Cit-WHHRRKK |
| 302 | WWHHKKRRV-Cit-RRKKHHWW |
| 303 | WRRKKV-Cit-KKRRW |
| 304 | WHRRKKV-Cit-KKRRHW |
| 305 | WHHRRKKV-Cit-KKRRHHW |
| 306 | QRRKKV-Cit-KKRRQ |
| 307 | KKRRQV-Cit-QRRKK |
| 308 | RRKKRGV-Cit-GRKKRR |
| 309 | GRKKRRV-Cit-RRKKRG |
| 310 | QRRKKRGV-Cit-GRKKRRQ |
| 311 | QGRKKRRV-Cit-RRKKRGQ |
| 312 | HRRV-Cit-RRH |
| 313 | HRRRV-Cit-RRRH |
| 314 | HRRRRV-Cit-RRRRH |
| 315 | HRRRRRV-Cit-RRRRRH |
| 316 | HKKV-Cit-KKH |
| 317 | HKKKV-Cit-KKKH |
| 318 | HKKKKV-Cit-KKKKH |
| 319 | HKKKKKV-Cit-KKKKKH |
| 320 | HWKKRRV-Cit-RRKKWH |
| 321 | RRKKWHV-Cit-HWKKRR |
| 322 | PPHRRRV-Cit-RRRHPP |
| 323 | RRRHPPV-Cit-PPHRRR |
| 324 | YYHHKKRRC-disulfide-CRRKKHHYY |
| 325 | YYHHKKRRV-Cit-RRKKHHYY |
| 326 | WWRRC-disulfide-CRRWW |
| 327 | WWRRV-Cit-RRWW |
| 328 | YYRRC-disulfide-CRRYY |
| 329 | YYRRV-Cit-RRYY |
| 330 | WWRRHHC-disulfide-CHHRRWW |
| 331 | WWRRHHV-Cit-HHRRWW |
| 332 | YYRRHHC-disulfide-CRRHHYY |
| 333 | YYRRHHV-Cit-RRHHYY |
| 334 | WWRRRC-disulfide-CRRRWW |
| 335 | WWRRRV-Cit-RRRWW |
| 336 | YYRRRC-disulfide-CRRRYY |
| 337 | YYRRRV-Cit-RRRYY |
| 338 | WWRRRHHC-disulfide-CHHRRRWW |
| 339 | WWRRRHHV-Cit-HHRRRWW |
| 340 | YYRRRHHC-disulfide-CRRRHHYY |
| 341 | YYRRRHHV-Cit-RRRHHYY |
| 342 | WWRRRRC-disulfide-CRRRRWW |
| 343 | WWRRRRV-Cit-RRRRWW |
| 344 | YYRRRRC-disulfide-CRRRRYY |
| 345 | YYRRRRV-Cit-RRRRYY |
| 346 | WWRRRRHHC-disulfide-CHHRRRRWW |
| 347 | WWRRRRHHV-Cit-HHRRRRWW |
| 348 | YYRRRRHHC-disulfide-CRRRRHHYY |
| 349 | YYRRRRHHV-Cit-RRRRHHYY |
| 350 | WWHHKKRRWV-Cit-WRRKKHHWW |
| 351 | WWHH-Orn-Orn-RRV-Cit-RR-Orn-Orn-HHWW |
| 352 | WWHHC-disulfide-CKKRR |

As used herein, amino acid names and designations refer to any stereoisomer of the corresponding amino acid.

In Table 5, a group which is internal to the peptide sequence may provide a cleavage site. For example, an internal cleavage site can be a disulfide bond or a Val-Cit linkage.

Examples of cleavable linkages include Phe-Lys, Val-Cit, Ala-Leu, Leu-Ala-Leu, and Ala-Leu-Ala-Leu (SEQ ID NO: 376), as described in U.S. Pat. Publ. No. 20080166363.

### Routes of administration

The active agent compositions of this disclosure may be used in pharmaceutical compositions. Administration of liposomal formulations of this disclosure to a subject may be parenteral, oral, by inhalation, topical, mucosal, rectal, or buccal. Parenteral use includes subcutaneous, intracutaneous, intravenous, intramuscular, intraarticular, intrasynovial, intrastemal, intrathecal, intralesional, and intracranial injection or infusion techniques.

### Effective amount

An effective amount of an active agent composition of this disclosure for treating a particular disease is generally an amount sufficient to ameliorate or reduce a symptom of the disease. An effective amount of an active agent composition of this disclosure may be an amount sufficient to cause any biological effect attributed to the agent. The composition may be administered as a single dosage, or may be administered by repeated dosing.

### DILA2 amino acid liposome-forming compounds

Liposomal compositions of this disclosure may include one or more DILA2 amino acid compounds which are disclosed in US 2008-0317839 A1.

DILA2 amino acid compounds are synthetic organic compounds that may form liposomal structures under certain conditions. DILA2 amino acid compounds may be formed by substituting a delivery-enhancing or lipophilic tail at either the N-terminus or the C-terminus of an amino acid, or at both termini. In some embodiments, the amino acid core may include one or more amino acids, or may be a peptide of 2-20 amino acid residues.

DILA2 amino acid compounds can be cationic or non-cationic, where non-cationic includes neutral and anionic. As used herein, the physical state or ionicity of a species refers to an environment having pH about 7, unless otherwise specified.

In some aspects, DILA2 amino acid compounds may provide delivery of a therapeutic agent in a releasable form. Releasable forms and compositions are designed to provide sufficient uptake of an agent by a cell to provide a therapeutic effect.

Releasable forms include DILA2 amino acid compounds that bind and release an active agent. In some embodiments, release of the active agent may be provided by an acid-labile linker.

Examples of acid-labile linkers include linkers containing an orthoester group, a hydrazone, a cis-acetonyl, an acetal, a ketal, a silyl ether, a silazane, an imine, a citriconic anhydride, a maleic anhydride, a crown ether, an azacrown ether, a thiacrown ether, a dithiobenzyl group, a cis-aconitic acid, a cis-carboxylic alkatriene, methacrylic acid, and mixtures thereof.

Examples of acid-labile groups and linkers are given in U.S. Patent Nos. 7,098,032; 6,897,196; 6,426,086; 7,138,382; 5,563,250; and 5,505,931.

Releasable forms of compounds and compositions of this disclosure include molecules that bind an active agent and discharge a moiety that assists in release of the agent. In some embodiments, a DILA2 amino acid compound may include a group which releases a small molecule such as ethanol that assists in delivering an agent to a cell. A DILA2 amino acid compound may bind an active agent and, subsequent to contact with a cell, or subsequent to transport within a biological compartment having a local pH lower than physiological pH, be hydrolyzed in an acidic environment to release ethanol to assist in delivery of the agent. In some embodiments, a small molecule such as ethanol, which assists in delivery of the agent, may be bound to a lipophilic component.

In some embodiments, a DILA2 amino acid compound may be admixed with a compound that releases a small molecule such as ethanol to assists in delivering an agent to a cell.

Releasable forms of compounds and compositions of this disclosure include DILA2 amino acid compounds which may bind an active agent and, subsequent to contact with a cell, or subsequent to transport within a biological compartment having a local pH lower than physiological pH, be modulated in an acidic environment into a cationic form to assist in release of the agent.

In some embodiments, a DILA2 amino acid compound may bind an active agent, and may be admixed with a compound that can be modulated in an acidic environment into a cationic form to assist in release of an active agent.

Examples of hydrolysable and modulatable groups are given in U.S. Patent Nos. 6,849,272; 6,200,599; as well as Z. H. Huang and F. C. Szoka, "Bioresponsive liposomes and their use for macromolecular delivery," in: G. Gregoriadis (ed.), Liposome Technology, 3rd ed. (CRC Press 2006).

In some embodiments, releasable forms of compounds and compositions of this disclosure include DILA2 amino acid compounds which can bind an active agent, and may be admixed with a lipid or compound that can be modulated in an acidic environment into a neutral form to assist in release of an active agent. The acidic environment may be entered subsequent to contact with a cell, or subsequent to transport within a biological compartment having a local pH lower than physiological pH.

Examples of compounds which are modulatable from anionic to neutral forms include cholesteryl hemisuccinate (CHEMS) as described in U.S. Patent Nos. 6,897,196; 6,426,086; and 7,108,863. In some examples, CHEMS exhibits pH sensitive polymorphism as described in Cullis, 1463 Biochimica et Biophysica Acta 107-14 (2000).

In some embodiments, releasable forms of compounds and compositions of this disclosure include DILA2 amino acid compounds which can bind an active agent, and may be admixed with a pH-sensitive polymeric material.

Examples of pH-sensitive polymeric materials are given in U.S. Patent No. 6,835,393.

In some embodiments, release of the active agent may be provided by an enzyme-cleavable peptide.

In some aspects, this disclosure provides a range of DILA2 amino acid compounds as shown in Formula I:

R³-(C=O)-Xaa-Z-R⁴ Formula I

wherein
Xaa is any D- or L-amino acid residue having the general formula
-NR^{N}-CR¹R²-(C=O)-, or a peptide of 2-20 amino acid residues, wherein
   R¹ is a non-hydrogen, substituted or unsubstituted side chain of an amino acid;
   R² is hydrogen, or an organic group consisting of carbon, oxygen, nitrogen, sulfur, and hydrogen atoms, and having from 1 to 20 carbon atoms, or C(1-5)alkyl, cycloalkyl, cycloalkylalkyl, C(3-5)alkenyl, C(3-5)alkynyl, C(1-5)alkanoyl, C(1-5)alkanoyloxy, C(1-5)alkoxy, C(1-5)alkoxy-C(1-5)alkyl, C(1-5)alkoxy-C(1-5)alkoxy, C(1-5)alkyl-amino-C(1-5)alkyl-, C(1-5)dialkyl-amino-C(1-5)alkyl-, nitro-C(1-5)alkyl, cyano-C(1-5)alkyl, aryl-C(1-5)alkyl, 4-biphenyl-C(1-5)alkyl, carboxyl, or hydroxyl,
   R^{N} is hydrogen, or an organic group consisting of carbon, oxygen, nitrogen, sulfur, and hydrogen atoms, and having from 1 to 20 carbon atoms, or C(1-5)alkyl, cycloalkyl, cycloalkylalkyl, C(3-5)alkenyl, C(3-5)alkynyl, C(1-5)alkanoyl, C(1-5)alkanoyloxy, C(1-5)alkoxy, C(1-5)alkoxy-C(1-5)alkyl, C(1-5)alkoxy-C(1-5)alkoxy, C(1-5)alkyl-amino-C(1-5)alkyl-, C(1-5)dialkyl-amino-C(1-5)alkyl-, nitro-C(1-5)alkyl, cyano-C(1-5)alkyl, aryl-C(1-5)alkyl, 4-biphenyl-C(1-5)alkyl, carboxyl, or hydroxyl,
R³ is a lipophilic tail derived from a naturally-occurring or synthetic phospholipid, glycolipid, triacylglycerol, glycerophospholipid, sphingolipid, ceramide, sphingomyelin, cerebroside, or ganglioside; or a substituted or unsubstituted C(3-22)alkyl, C(6-12)cycloalkyl, C(6-12)cycloalkyl-C(3-22)alkyl, C(3-22)alkenyl, C(3-22)alkynyl, C(3-22)alkoxy, or C(6-12)alkoxy-C(3-22)alkyl; or a lipophilic tail of any other naturally-occurring or synthetic lipid, or a lipophilic tail of any one of the lipids described hereinbelow, and may contain a steroid;
R⁴ is a lipophilic tail derived from a naturally-occurring or synthetic phospholipid, glycolipid, triacylglycerol, glycerophospholipid, sphingolipid, ceramide, sphingomyelin, cerebroside, or ganglioside; or substituted or unsubstituted C(3-22)alkyl, C(6-12)cycloalkyl, C(6-12)cycloalkyl-C(3-22)alkyl, C(3-22)alkenyl, C(3-22)alkynyl, C(3-22)alkoxy, or C(6-12)alkoxy-C(3-22)alkyl; or a lipophilic tail of any other naturally-occurring or synthetic lipid, or a lipophilic tail of any one of the lipids described hereinbelow, and may contain a steroid;
Z is NH, O, S, -CH₂S-, -CH₂S(O)-, or an organic linker consisting of 1-40 atoms selected from hydrogen, carbon, oxygen, nitrogen, and sulfur atoms;
and salts thereof.

In some embodiments, R³ is independently a substituted or unsubstituted C(6-22)alkyl or C(6-22)alkenyl; R⁴ is independently a substituted or unsubstituted C(6-22)alkyl or C(6-22)alkenyl.

The residue Xaa may be a D- or L-stereocenter.

In some embodiments, R¹ is a non-hydrogen, substituted or unsubstituted side chain of an amino acid wherein a substituent of a side chain is an organic group consisting of 1 to 40 atoms selected from hydrogen, carbon, oxygen, nitrogen, and sulfur atoms.

In some embodiments, Z is an alkyl or an organic linker synthetic polymer such as a polyethylene glycol chain (PEG), or a PEG copolymer such as PEG-polyurethane or PEG-polypropylene. See, e.g., J. Milton Harris, Poly(ethylene glycol) chemistry: biotechnical and biomedical applications (1992).

In some embodiments, this invention provides a range of DILA2 amino acid compounds as shown in Formula I above wherein:
Xaa is any D- or L-amino acid having the general formula -NR^{N}-CR¹R²-(C=O)-,
   wherein
   R¹ is a non-hydrogen, substituted or unsubstituted basic side chain of an amino acid;
   R² is hydrogen, or C(1-5)alkyl,
   R^{N} is hydrogen, or C(1-5)alkyl,
R³ is a lipophilic tail derived from a naturally-occurring or synthetic phospholipid, glycolipid, triacylglycerol, glycerophospholipid, sphingolipid, ceramide, sphingomyelin, cerebroside, or ganglioside; or a substituted or unsubstituted C(3-22)alkyl, C(6-12)cycloalkyl, C(6-12)cycloalkyl-C(3-22)alkyl, C(3-22)alkenyl, C(3-22)alkynyl, C(3-22)alkoxy, or C(6-12)alkoxy-C(3-22)alkyl; or a lipophilic tail of any other naturally-occurring or synthetic lipid, or a lipophilic tail of any one of the lipids described hereinbelow, and may contain a steroid;
R⁴ is a lipophilic tail derived from a naturally-occurring or synthetic phospholipid, glycolipid, triacylglycerol, glycerophospholipid, sphingolipid, ceramide, sphingomyelin, cerebroside, or ganglioside; or substituted or unsubstituted C(3-22)alkyl, C(6-12)cycloalkyl, C(6-12)cycloalkyl-C(3-22)alkyl, C(3-22)alkenyl, C(3-22)alkynyl, C(3-22)alkoxy, or C(6-12)alkoxy-C(3-22)alkyl; or a lipophilic tail of any other naturally-occurring or synthetic lipid, or a lipophilic tail of any one of the lipids described hereinbelow, and may contain a steroid;
Z is NH, O, S, -CH₂S-, -CH₂S(O)-, or an organic linker consisting of 1-40 atoms selected from hydrogen, carbon, oxygen, nitrogen, and sulfur atoms.

In some embodiments, this invention provides a range of DILA2 amino acid compounds as shown in Formula I above wherein:
Xaa is any D- or L-amino acid having the general formula -NR^{N}-CR¹R²-(C=O)-,
   wherein
   R¹ is a non-hydrogen, substituted or unsubstituted basic side chain of an amino acid;
   R² is hydrogen, or C(1-5)alkyl,
   R^{N} is hydrogen, or C(1-5)alkyl,
R³ is a substituted or unsubstituted C(3-22)alkyl, C(6-12)cycloalkyl, C(6-12)cycloalkyl-C(3-22)alkyl, C(3-22)alkenyl, C(3-22)alkynyl, C(3-22)alkoxy, or C(6-12)alkoxy-C(3-22)alkyl;
R⁴ is a substituted or unsubstituted C(3-22)alkyl, C(6-12)cycloalkyl, C(6-12)cycloalkyl-C(3-22)alkyl, C(3-22)alkenyl, C(3-22)alkynyl, C(3-22)alkoxy, or C(6-12)alkoxy-C(3-22)alkyl;
Z is NH, O, S, -CH₂S-, -CH₂S(O)-, or an organic linker consisting of 1-40 atoms selected from hydrogen, carbon, oxygen, nitrogen, and sulfur atoms.

In some embodiments, this invention provides a range of DILA2 amino acid compounds as shown in Formula I above wherein:
Xaa is any D- or L-amino acid having the general formula -NR^{N}-CR¹R²-(C=O)-,
   wherein
   R¹ is a non-hydrogen, substituted or unsubstituted basic side chain of an amino acid;
   R² is hydrogen, or C(1-5)alkyl,
   R^{N} is hydrogen, or C(1-5)alkyl,
R³ is a substituted or unsubstituted C(3-22)alkyl, C(6-12)cycloalkyl, C(6-12)cycloalkyl-C(3-22)alkyl, C(3-22)alkenyl, C(3-22)alkynyl, C(3-22)alkoxy, or C(6-12)alkoxy-C(3-22)alkyl;
R⁴ is a substituted or unsubstituted C(3-22)alkyl, C(6-12)cycloalkyl, C(6-12)cycloalkyl-C(3-22)alkyl, C(3-22)alkenyl, C(3-22)alkynyl, C(3-22)alkoxy, or C(6-12)alkoxy-C(3-22)alkyl;
Z is NH.

In some embodiments, this invention provides a range of DILA2 amino acid compounds as shown in Formula I above wherein:
Xaa is any D- or L-amino acid having the general formula -NR^{N}-CR¹R²-(C=O)-,
   wherein
   R¹ is a non-hydrogen, substituted or unsubstituted basic side chain of an amino acid;
   R² is hydrogen, or C(1-5)alkyl,
   R^{N} is hydrogen, or C(1-5)alkyl,
R³ is a substituted or unsubstituted C(3-22)alkyl, C(6-12)cycloalkyl, C(6-12)cycloalkyl-C(3-22)alkyl, C(3-22)alkenyl, C(3-22)alkynyl, C(3-22)alkoxy, or C(6-12)alkoxy-C(3-22)alkyl;
R⁴ is a substituted or unsubstituted C(3-22)alkyl, C(6-12)cycloalkyl, C(6-12)cycloalkyl-C(3-22)alkyl, C(3-22)alkenyl, C(3-22)alkynyl, C(3-22)alkoxy, or C(6-12)alkoxy-C(3-22)alkyl;
Z is O.

Cationic DILA2 amino acid compounds can be prepared where, for example, Xaa has a basic side chain. Examples of amino acids having a basic side chain include arginine (Arg), homoarginine (homoArg) (side chain -(CH₂)₄NH(C=NH)NH₂), norarginine (norArg) (side chain -(CH₂)₂NH(C=NH)NH₂), nor-norarginine (nornorArg) (side chain -(CH₂)NH(C=NH)NH₂), ornithine, lysine, homolysine, histidine, 1-methylhistidine, pyridylalanine (Pal), asparagine, N-ethylasparagine, glutamine, and 4-aminophenylalanine, and side chain modified derivatives thereof.

As used herein, the term "homo," when referring to an amino acid, means that an additional carbon is added to the side chain, while the term "nor," when referring to an amino acid, means that a carbon is subtracted from the side chain. Thus, homolysine refers to side chain-(CH₂)₅NH₂.

Anionic DILA2 amino acid compounds can be prepared where, for example, Xaa is glutamate or aspartate.

Cationic and anionic DILA2 amino acid compounds can also be prepared where the amino acid side chain contains an ionizable group or substituent.

Non-cationic DILA2 amino acid compounds can be prepared where, for example, Xaa is leucine, valine, alanine, or serine.

In some embodiments, Xaa is N^{G}-methylarginine, symmetric or asymmetric N^{G},N^{G}-dimethylarginine, N^{G}-methyl-homoarginine, symmetric or asymmetric N^{G},N^{G}-dimethyl-homoarginine, N^{G}-methyl-norarginine, symmetric or asymmetric N^{G},N^{G}-dimethyl-norarginine, or N^{G}-methyl-nor-norarginine, symmetric or asymmetric N^{G},N^{G}-dimethyl-nor-norarginine.

In some embodiments, Xaa is N^{G}-ethylarginine, symmetric or asymmetric N^{G},N^{G}-diethylarginine, N^{G}-ethyl-homoarginine, symmetric or asymmetric N^{G},N^{G}-diethyl-homoarginine, N^{G}-ethyl-norarginine, symmetric or asymmetric N^{G},N^{G}-diethyl-norarginine, or N^{G}-ethyl-nor-norarginine, symmetric or asymmetric N^{G},N^{G}-diethyl-nor-norarginine.

In certain embodiments, Xaa is N^{G}-alkylarginine, symmetric or asymmetric N^{G},N^{G}-dialkylarginine, N^{G}-alhyl-homoarginine, symmetric or asymmetric N^{G},N^{G}-dialkyl-homoarginine, N^{G}-alkyl-norarginine, symmetric or asymmetric N^{G},N^{G}-dialkyl-norarginine, or N^{G}-alkyl-nor-norarginine, symmetric or asymmetric N^{G},N^{G}-dialkyl-nornorarginine.

In some embodiments, Xaa is an amino acid having a guanidine- or amidine-containing side chain. For example, the side chain of the Xaa residue may contain a group such as guanido, amidino, dihydroimidazole, 4-guanido-phenyl, 4-amidino-phenyl, N-amidino-piperidine, N-amidino-piperazine, 4,5-dihydroimidazole, 2-(N-amidino)-pyrrolidinyl, or 4-[(2-aminopyrimidinyl)]ethyl.

Examples of Xaa side chains include the following structures, as well as their salt forms:

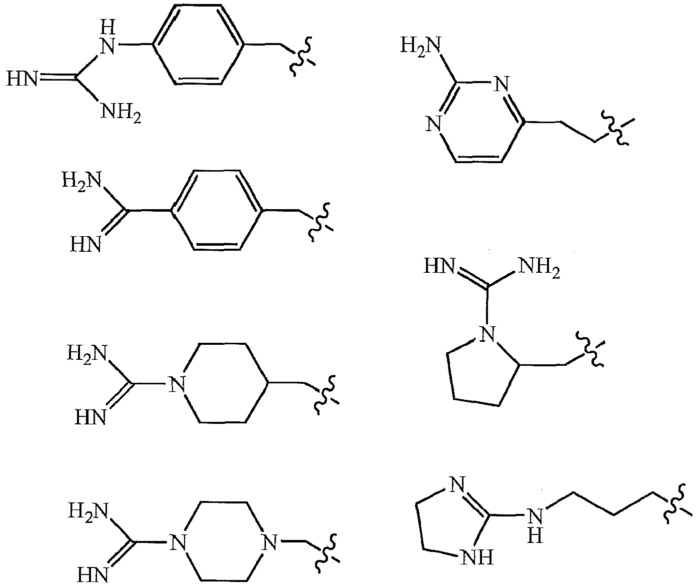

Examples of a substituted side chain of an amino acid suitable for a releasable form of a DILA2 amino acid compound include a releasing functional group having a pKa from about 5 to about 7.5, or from about 6 to about 7. In general, a releasing functional group which is a weak base may exhibit a predominant neutral form at a local pH above pKa, and may exhibit a predominant ionic form at a local pH below pKa. A releasing functional group which is a weak acid may exhibit an ionic form at a local pH above pKa, and may exhibit a neutral form at a local pH below pKa. See, e.g., P. Heinrich Stahl, Handbook of Pharmaceutical Salts (2002).

In some embodiments, Xaa may have a side chain containing a functional group having a pKa from 5 to 7.5.

Examples of a substituted side chain of an amino acid suitable for a releasable form of a DILA2 amino acid compound include 1-methylhistidine.

Examples of a substituted side chain of an amino acid suitable for a releasable form of a DILA2 amino acid compound include 3,5-diiodo-tyrosine.

Examples of a substituted side chain of an amino acid suitable for a releasable form of a DILA2 amino acid compound include the following structures:

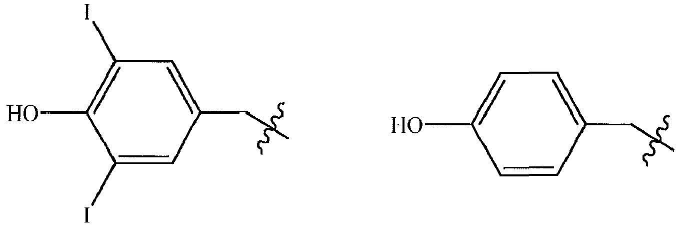

Examples of DILA2 amino acid compounds include the structures:

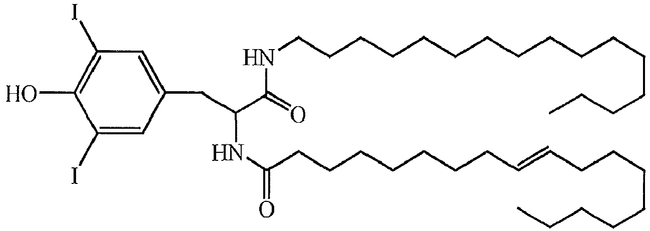

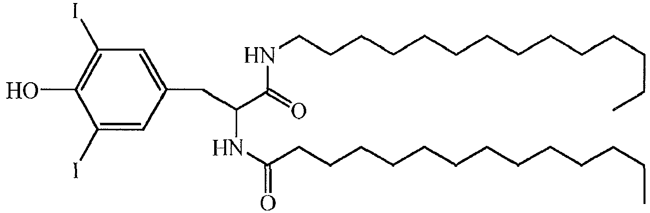

Examples of a substituent on a side chain of an amino acid suitable for a releasable form of a DILA2 amino acid compound include releasing functional groups derived from 3,5-diiodo-tyrosine, 1-methylhistidine, 2-Methylbutanoic acid, 2-*o*-Anisylpropanoic acid, *meso*-Tartaric acid, 4,6-Dimethylpyrimidinamine, *p*-Phthalic acid, Creatinine, Butanoic acid, *N*,*N*-Dimethyl-1-naphthylamine, Pentanoic acid, 4-Methylpentanoic acid, N-Methylaniline, 1,10-Phenanthroline, 3-Pyridinecarboxylic acid, Hexanoic acid, Propanoic acid, 4-Animobenzoic acid, 2-Methylpropanoic acid, Heptanoic acid, Octanoic acid, Cyclohexanecarboxylic acid, Quinoline, 3-Quinolinamine, 2-Aminobenzoic acid, 4-Pyridinecarboxylic acid, Nonanic acid, Melamine, 8-Quinolinol, Trimethylacetic acid, 6-Methoxyquinoline, 4-(Methylamino)benzoic acid, *p-*Methylaniline, 3-(Methylamino)benzoic acid, Malic acid, *N*-Ethylaniline, 2-Benzylpyridine, 3,6-Dinitrophenol, *N*,*N*-Dimethylaniline, 2,5-Dimethylpiperazine, *p-*Phenetidine, 5-Methylquinoline, 2-Phenylbenzimidazole, Pyridine, Picolinic acid, 3,5-Diiodityrosine, p-Anisidine, 2-(Methylamino)benzoic acid, 2-Thiazolamine, Glutaric acid, Adipic acid, Isoquinoline, Itaconic acid, *o*-Phthalic acid, Benzimidazole, Piperazine, Heptanedioic acid, Acridine, Phenanthridine, Succinic acid, Methylsuccinic acid, 4-Methylquinoline, 3-Methylpyridine, 7-Isoquinolinol, Malonic acid, Methymalonic acid, 2-Methylquinoline, 2-Ethylpyridine, 2-Methylpyridine, 4-Methylpyridine, Histamine, Histidine, Maleic acid, *cis*-1,2-Cyclohexanediamine, 3,5-Dimethylpyridine, 2-Ethylbenzimidazole, 2-Methylbenzimidazole, Cacodylic acid, Perimidine, Citric acid, Isocitric acid, 2,5-Dimethylpyridine, Papaverine, 6-Hydroxy-4-methylpteridine, *L-*Thyroxine, 3,4-Dimethylpyridine, Methoxypyridine, *trans*-1,2-Cyclohexanediamine, 2,5-Pyridinediamine, *l*-1-Methylhistidine, *l*-3-Methylhistidine, 2,3-Dimethylpyridine, Xanthopterin, 1,2-Propanediamine, *N*,*N-*Diethylaniline, Alloxanic acid, 2,6-Dimethylpyridine, L-Carnosine, 2-Pyridinamine, N-b-Alanylhistidine, Pilocarpine, 1-Methylimidazol, 1H-Imidazole, 2,4-Dimethylpyridine, 4-Nitrophenol, 2-Nitrophenol, Tyrosineamide, 5-Hydoxxyquinazoline, 1,1-Cyclopropanedicarboxylic acid, 2,4,6-Trimethylpyridine, Veronal, 2,3-Dichlorophenol, 1,2-Ethanediamine, 1-Isoquinolinamine, and combinations thereof.

In some embodiments, a range of DILA2 amino acid compounds corresponding to Formula I are represented by the structures 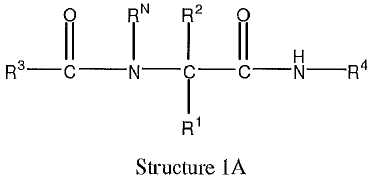 and 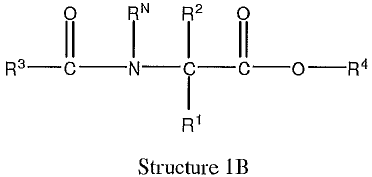 where R¹, R², R^{N}, R³, and R⁴ are defined as above.

In some embodiments, R³ and R⁴ are independently selected lipophilic tails which impart sufficient lipophilic character or lipophilicity, such as defined by water/octanol partitioning, to provide delivery across a membrane or uptake by a cell. These tails provide, when used in a DILA2 amino acid compound, an amphipathic molecule. Lipophilic tails may be derived from phospholipids, glycolipids, triacylglycerols, glycerophospholipids, sphingolipids, ceramides, sphingomyelins, cerebrosides, or gangliosides, among others, and may contain a steroid.

In certain embodiments, R³ and R⁴ may independently be a lipophilic tail having a glycerol backbone.

In some embodiments, R³ and R⁴ may independently be C10alkyl, C11alkyl, C12alkyl, C13alkyl, C14alkyl, C15alkyl, C16alkyl, C17alkyl, C18alkyl, C19alkyl, C20alkyl, C21alkyl, or C22alkyl.

In some embodiments, R³ and R⁴ may independently be lipophilic tails having one of the following structures: 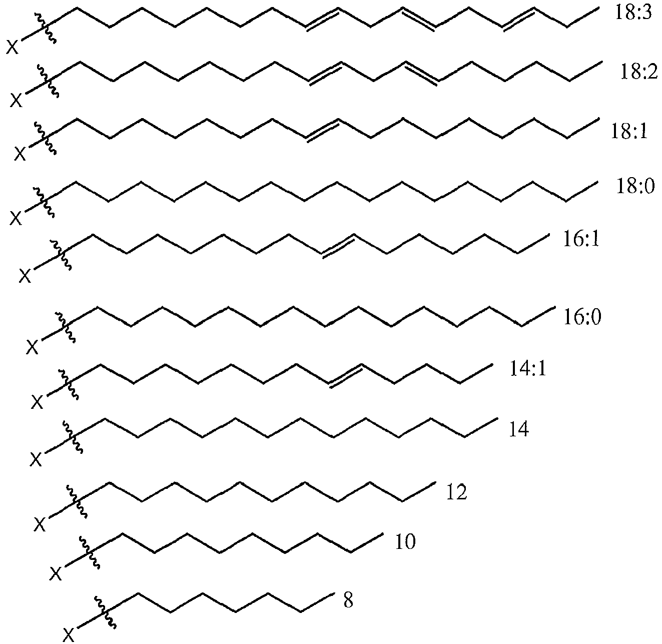 In the structures above, X represents the atom of the tail that is directly attached to the amino acid residue terminus, and is counted as one of the atoms in the numerical designation, for example, "18:3." In some embodiments, X may be a carbon, nitrogen, or oxygen atom.

In some embodiments, R³ and R⁴ may independently be lipophilic tails having one of the following structures: 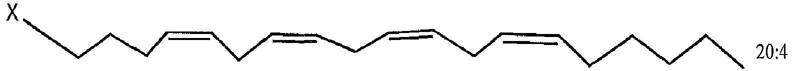 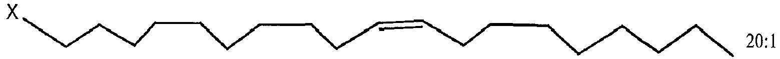 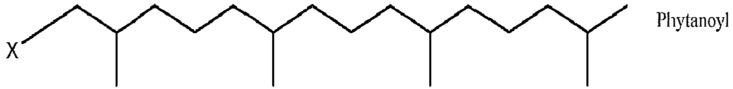 where X is as defined above.

In some embodiments, R³ and R⁴ are independently selected lipophilic tails which may contain a cholesterol, a sterol, or a steroid such as gonanes, estranes, androstanes, pregnanes, cholanes, cholestanes, ergostanes, campestanes, poriferastanes, stigmastanes, gorgostanes, lanostanes, cycloartanes, as well as sterol or zoosterol derivatives of any of the foregoing, and their biological intermediates and precursors, which may include, for example, cholesterol, lanosterol, stigmastanol, dihydrolanosterol, zymosterol, zymostenol, desmosterol, 7-dehydrocholesterol, and mixtures and derivatives thereof.

In certain embodiments, R³ and R⁴ may independently be derived from fatty acid-like tails such as tails from myristic acid (C14:0)alkenyl, palmitic acid (C16:0)alkenyl, stearic acid (C18:0)alkenyl, oleic acid (C18:1, double bond at carbon 9)alkenyl, linoleic acid (C18:2, double bond at carbon 9 or 12)alkenyl, linonenic acid (C18:3, double bond at carbon 9, 12, or 15)alkenyl, arachidonic acid (C20:4, double bond at carbon 5, 8, 11, or 14)alkenyl, and eicosapentaenoic acid (C20:5, double bond at carbon 5, 8, 11, 14, or 17)alkenyl. Other examples of fatty acid-like tails are found at Donald Voet and Judith Voet, Biochemistry, 3rd Edition (2005), p. 383.

In some embodiments, R³ and R⁴ may independently be derived from an isoprenoid.

As used herein, the term "amino acid" includes naturally-occurring and non-naturally occurring amino acids. Thus, a DILA2 amino acid compound can be made from a genetically encoded amino acid, a naturally occurring non-genetically encoded amino acid, or a synthetic amino acid.

Examples of amino acids include Ala, Arg, Asn, Asp, Cys, Gln, Glu, Gly, His, Ile, Leu, Lys, Met, Phe, Pro, Ser, Thr, Trp, Tyr, and Val.

Examples of amino acids include azetidine, 2-aminooctadecanoic acid, 2-aminoadipic acid, 3-aminoadipic acid, 2,3-diaminopropionic acid, 2-aminobutyric acid, 4-aminobutyric acid, 2,3-diaminobutyric acid, 2,4-diaminobutyric acid, 2-aminoisobutyric acid, 4-aminoisobutyric acid, 2-aminopimelic acid, 2,2'-diaminopimelic acid, 6-aminohexanoic acid, 6-aminocaproic acid, 2-aminoheptanoic acid, desmosine, ornithine, citrulline, N-methylisoleucine, norleucine, tert-leucine, phenylglycine, t-butylglycine, N-methylglycine, sacrosine, N-ethylglycine, cyclohexylglycine, 4-oxo-cyclohexylglycine, N-ethylasparagine, cyclohexylalanine, t-butylalanine, naphthylalanine, pyridylalanine, 3-chloroalanine, 3-benzothienylalanine, 4-halophenylalanine, 4-chlorophenylalanine, 2-fluorophenylalanine, 3-fluorophenylalanine, 4-fluorophenylalanine, penicillamine, 2-thienylalanine, methionine, methionine sulfoxide, homoarginine, norarginine, nor-norarginine, N-acetyllysine, 4-aminophenylalanine, N-methylvaline, homocysteine, homoserine, hydroxylysine, allo-hydroxylysine, 3-hydroxyproline, 4-hydroxyproline, isodesmosine, allo-isoleucine, 6-N-methyllysine, norvaline, O-allyl-serine, O-allyl-threonine, alpha-aminohexanoic acid, alpha-aminovaleric acid, and pyroglutamic acid.

As used herein, the term "amino acid" includes alpha- and beta- amino acids.

Other amino acid residues can be found in Fasman, CRC Practical Handbook of Biochemistry and Molecular Biology, CRC Press, Inc. (1989).

In general, a compound may contain one or more chiral centers. Compounds containing one or more chiral centers may include those described as an "isomer," a "stereoisomer," a "diastereomer," an "enantiomer," an "optical isomer," or as a "racemic mixture." Conventions for stereochemical nomenclature, for example the stereoisomer naming rules of Cahn, Ingold and Prelog, as well as methods for the determination of stereochemistry and the separation of stereoisomers are known in the art. See, for example, Michael B. Smith and Jerry March, March's Advanced Organic Chemistry, 5th edition, 2001. The compounds and structures of this disclosure are meant to encompass all possible isomers, stereoisomers, diastereomers, enantiomers, and/or optical isomers that would be understood to exist for the specified compound or structure, including any mixture, racemic or otherwise, thereof.

Examples of DILA2 amino acid compounds include R³-(C=O)-Arg-NH-R⁴ wherein Arg is D- or L-arginine, and R³ and R⁴ are independently alkyl or alkenyl.

Examples of DILA2 amino acid compounds include the following structures:

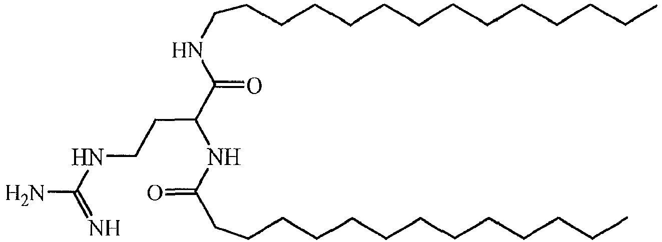

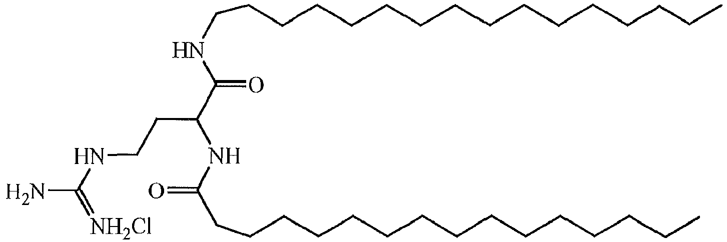

Examples of DILA2 amino acid compounds include the following structures:

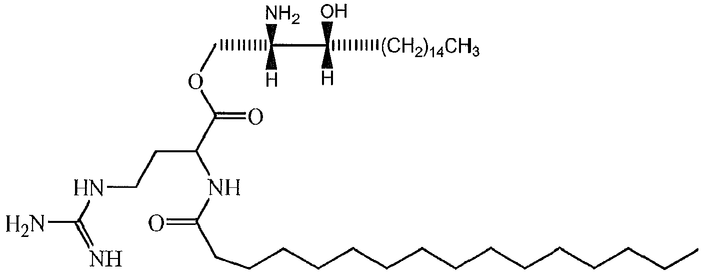

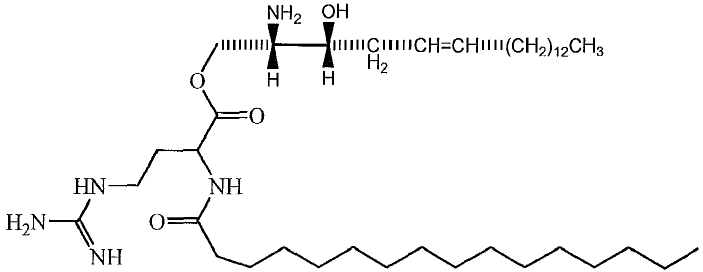

Examples of DILA2 amino acid compounds include the following structures:

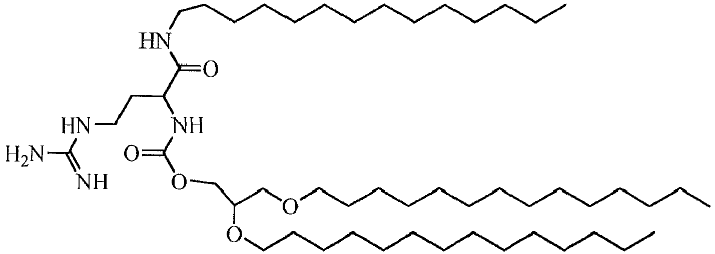

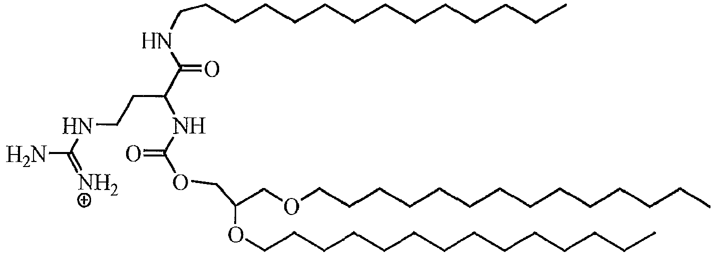

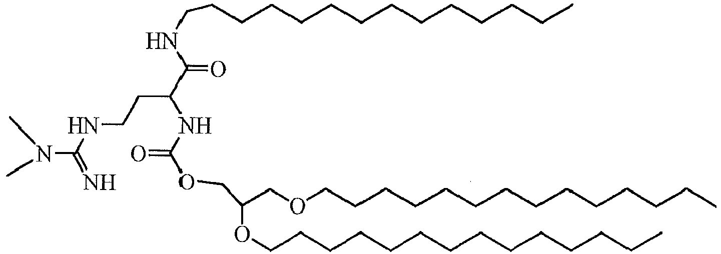

Examples of DILA2 amino acid compounds include R³-(C=O)-norArg-NH-R⁴ wherein norArg is D- or L-norarginine, and R³ and R⁴ are independently alkyl or alkenyl.

Examples of DILA2 amino acid compounds include the following structures:

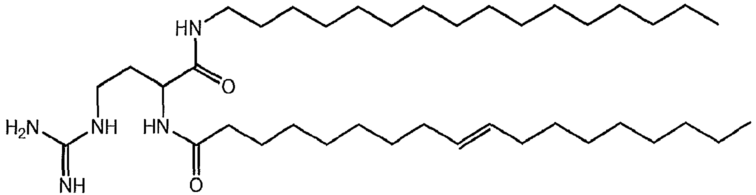

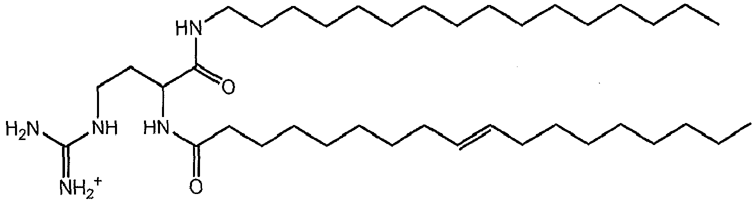

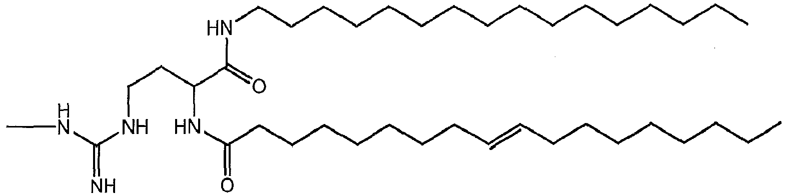

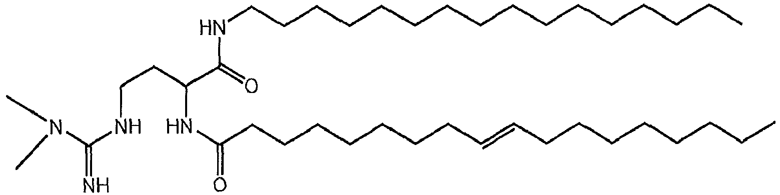

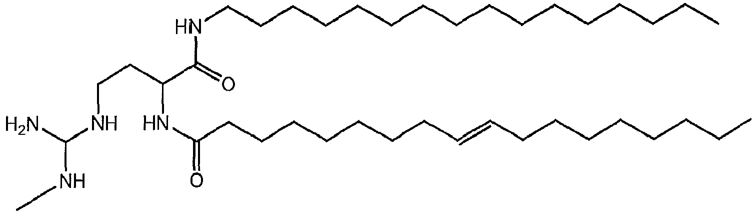

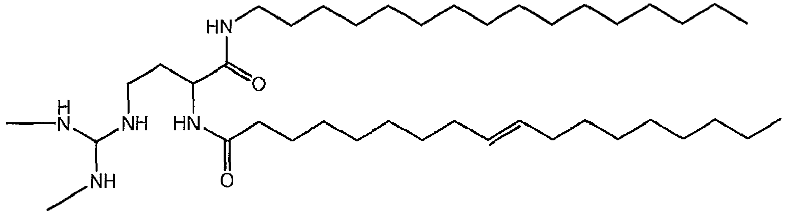

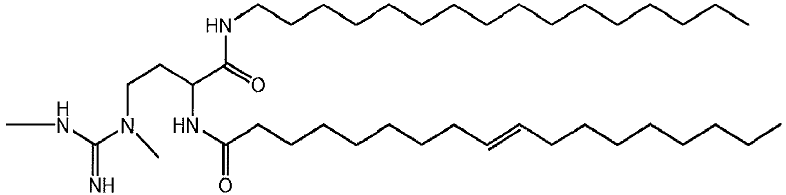

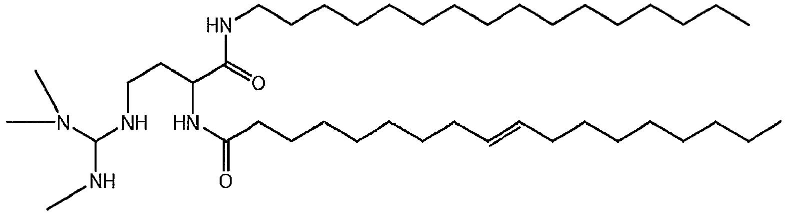

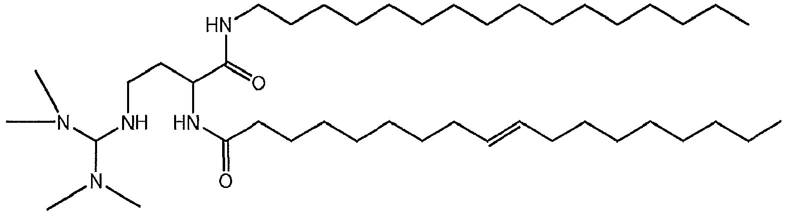

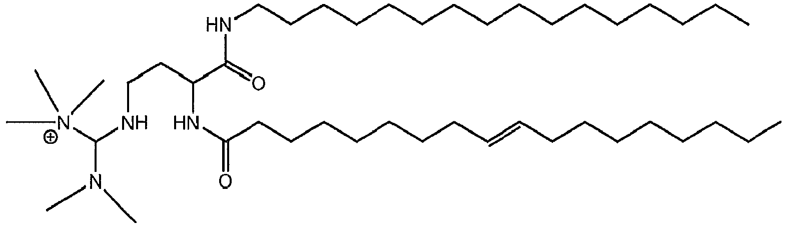

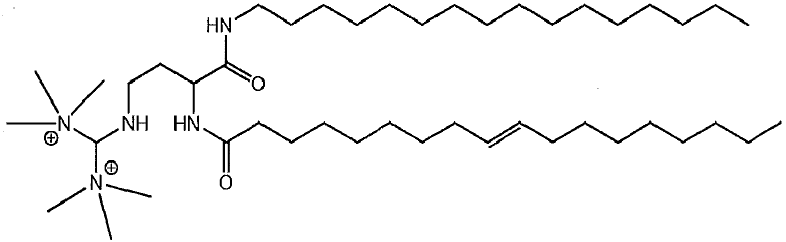

Examples of DILA2 amino acid compounds include R³-(C=O)-nornorArg-NH-R⁴ wherein nornorArg is D- or L-nor-norarginine, and R³ and R⁴ are independently alkyl such as heptyl, octyl, nonyl, decyl, and undecyl.

Examples of DILA2 amino acid compounds include the following structures:

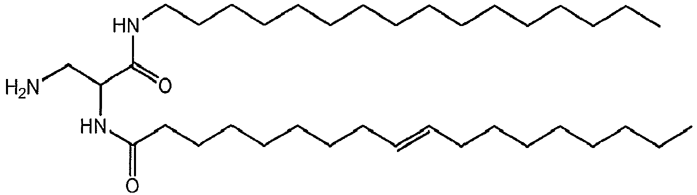

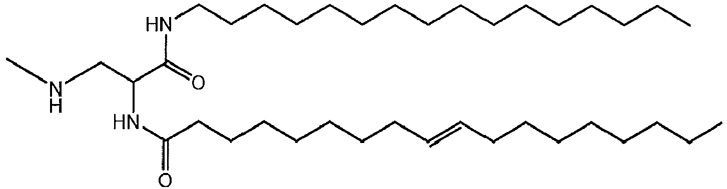

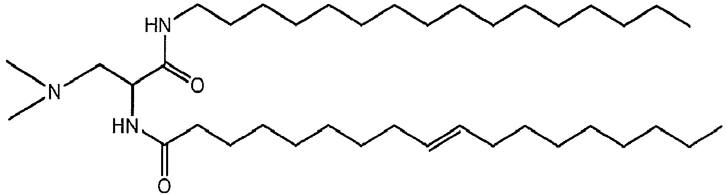

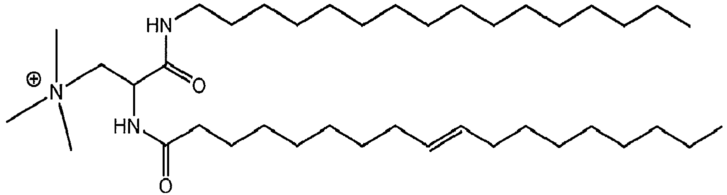

Examples of DILA2 amino acid compounds include R³-(C=O)-homoArg-NH-R⁴ wherein homoArg is D- or L-homoarginine, and R³ and R⁴ are independently alkyl such as heptyl, octyl, nonyl, decyl, and undecyl.

Examples of DILA2 amino acid compounds include R³-(C=O)-4-pyridylalanine-NH-R⁴ wherein the pyridylalanine is D- or L-pyridylalanine, and R³ and R⁴ are independently alkyl such as heptyl, octyl, nonyl, decyl, and undecyl. Examples of R³-(C=O)-pyridylalanine-NH-R⁴ DILA2 amino acid compounds include pharmaceutically-acceptable pyridyl salts, such as 4-[N-methylpyridyl]alanine chloride. Examples of pyridylalanine DILA2 amino acid compounds include the following structures:

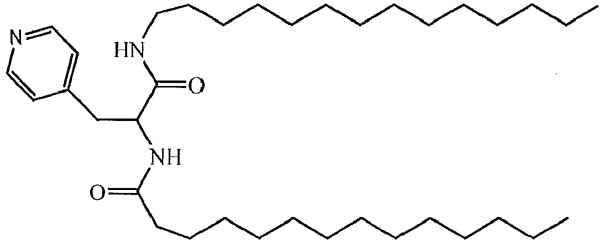

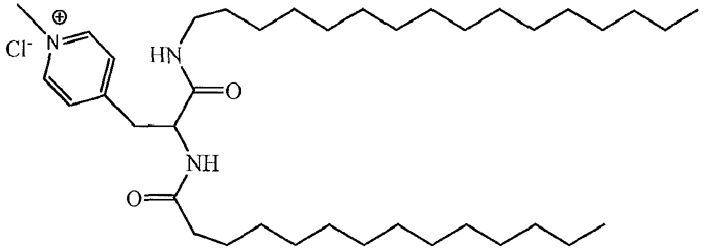

Examples of DILA2 amino acid compounds include R³-(C=O)-Lys-NH-R⁴ wherein R³ and R⁴ are independently alkyl or alkenyl.

Examples of DILA2 amino acid compounds include the following structures:

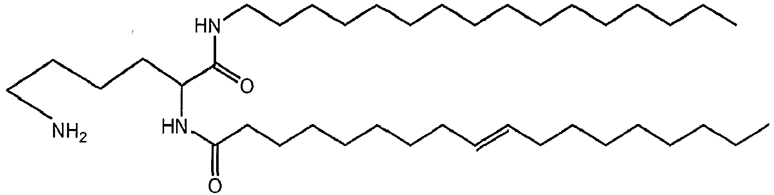

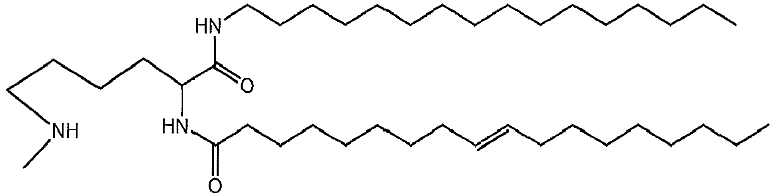

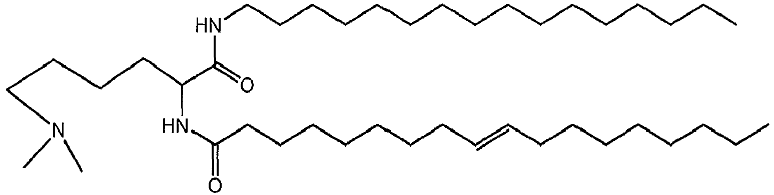

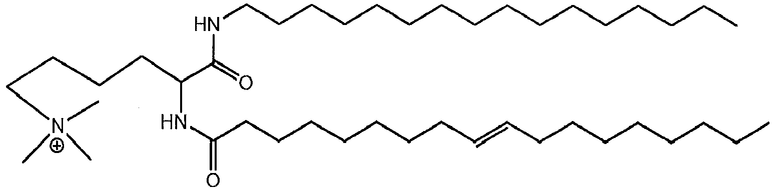

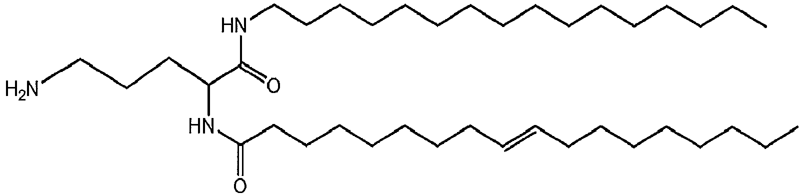

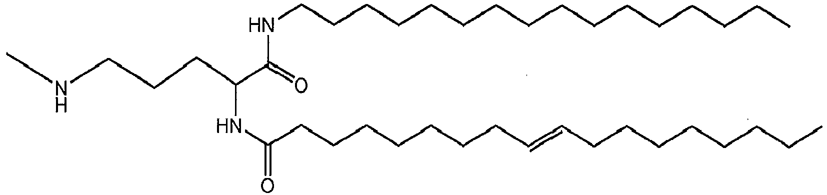

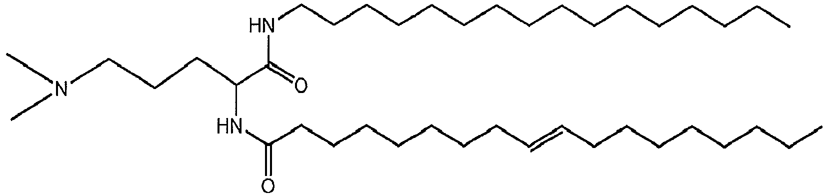

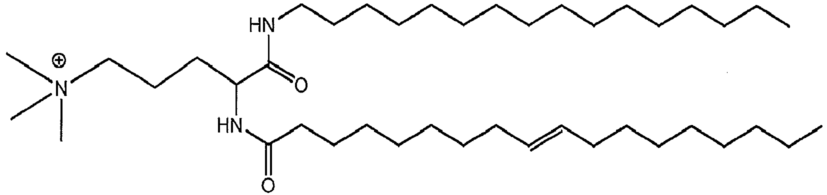

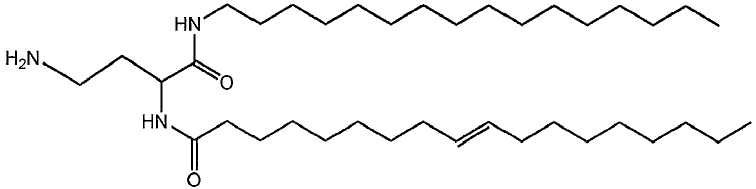

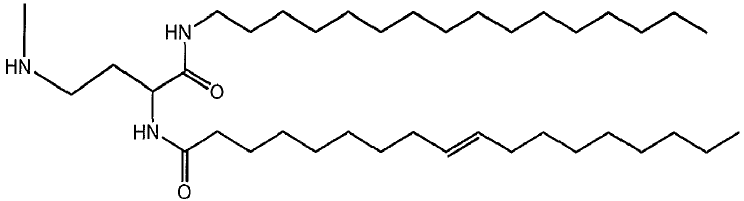

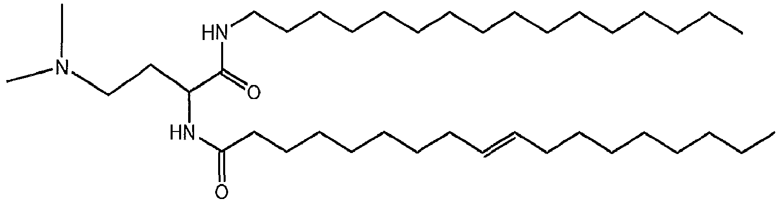

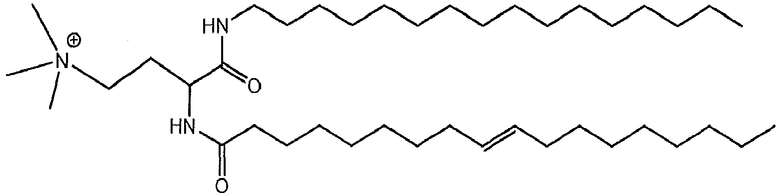

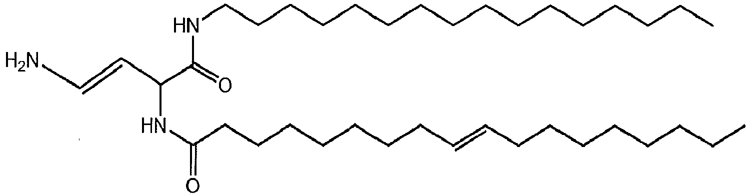

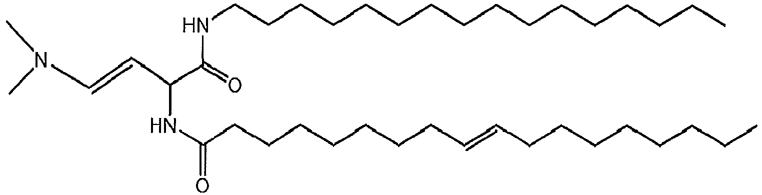

Examples of DILA2 amino acid compounds include R³-(C=O)-His-NH-R⁴ wherein R³ and R⁴ are independently alkyl or alkenyl. Examples of His DILA2 amino acid compounds include the following structures:

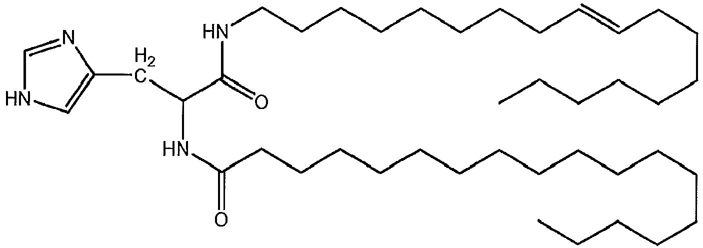

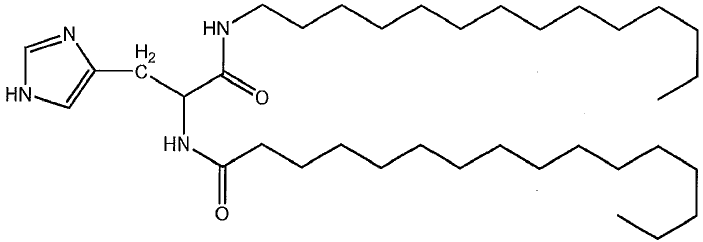

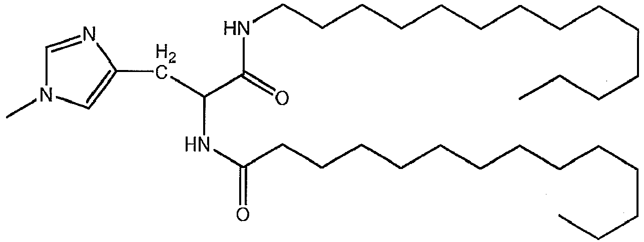

Examples of DILA2 amino acid compounds include R³-(C=O)-Xaa-O-R⁴ wherein R³ is alkyl and R⁴ is a sphingoid.

Examples of DILA2 amino acid compounds include the following structures:

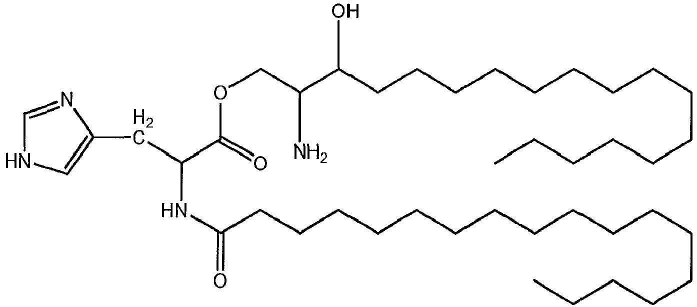

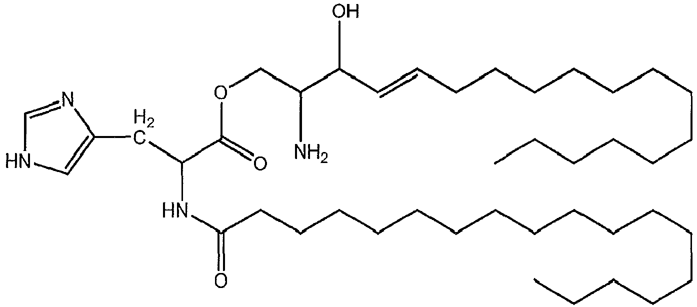

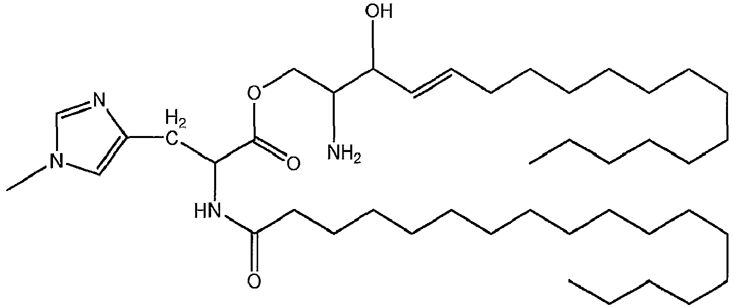

Examples of DILA2 amino acid compounds include R³-(C=O)-Xaa-NH-R⁴ wherein R³ and R⁴ are alkyl or alkenyl. Examples of DILA2 amino acid compounds include the following structure:

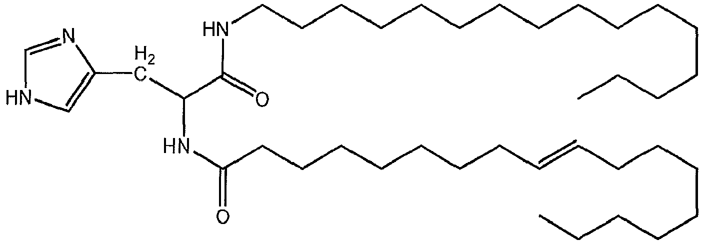

Examples of DILA2 amino acid compounds include the following structure:

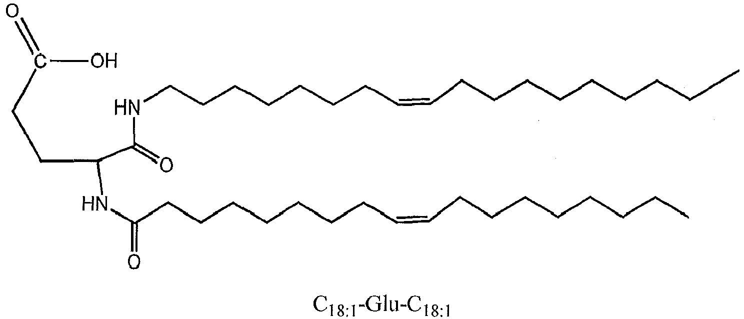

Examples of DILA2 amino acid compounds include the following structure:

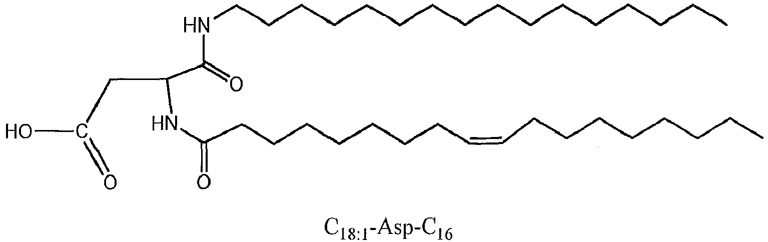

Examples of DILA2 amino acid compounds include the following structure:

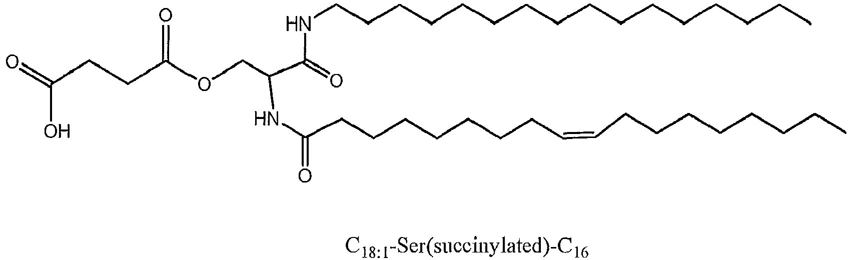

Examples of DILA2 amino acid compounds include (C10acyl)-Arg-NH-(C10alkyl) (SEQ ID NO: 11), (C12acyl)-Arg-NH-(C12alkyl) (SEQ ID NO: 11), (C14acyl)-Arg-NH-(C14alkyl) (SEQ ID NO: 11), (C16acyl)-Arg-NH-(C16alkyl) (SEQ ID NO: 11), (C18acyl)-Arg-NH-(C18alkyl) (SEQ ID NO: 11), (C10acyl)-homoArg-NH-(C10alkyl), (C12acyl)-homoArg-NH-(C12alkyl), (C14acyl)-homoArg-NH-(C14alkyl), (C16acyl)-homoArg-NH-(C16alkyl), (C18acyl)-homoArg-NH-(C18alkyl), (C10acyl)-norArg-NH-(C10alkyl), (C12acyl)-norArg-NH-(C12alkyl), (C14acyl)-norArg-NH-(C14alkyl), (C16acyl)-norArg-NH-(C16alkyl), (C18acyl)-norArg-NH-(C18alkyl), (C10acyl)-nornorArg-NH-(C10alkyl), (C12acyl)-nornorArg-NH-(C12alkyl), (C14acyl)-nornorArg-NH-(C14alkyl), (C16acyl)-nornorArg-NH-(C16alkyl), (C18acyl)-nornorArg-NH-(C18alkyl), (C10acyl)-4-Pal-NH-(C10alkyl), (C12acyl)-4-Pal-NH-(C12alkyl), (C14acyl)-4-Pal-NH-(C14alkyl), (C16acyl)-4-Pal-NH-(C16alkyl), (C18acyl)-4-Pal-NH-(C18alkyl), (C10acyl)-4-Pal(Me)-NH-(C10alkyl), (C12acyl)-4-Pal(Me)-NH-(C12alkyl), (C14acyl)-4-Pal(Me)-NH-(C14alkyl), (C16acyl)-4-Pal(Me)-NH-(C16alkyl), and (C18acyl)-4-Pal(Me)-NH-(C18alkyl).

In general, the designation "C14-norArg-C14," for example, refers to (C13alkyl)-(C=O)-norArg-NH-(C14alkyl) which is the same as (C14acyl)-norArg-NH-(C14alkyl).

Examples of DILA2 amino acid compounds include (C10acyl)-D-Arg-L-Arg-NH-(C10alkyl), (C12acyl)-D-Arg-L-Arg-NH-(C12alkyl), (C14acyl)-D-Arg-L-Arg-NH-(C14alkyl), (C16acyl)-D-Arg-L-Arg-NH-(C16alkyl), (C18acyl)-D-Arg-L-Arg-NH-(C18alkyl), (C10acyl)-D-homoArg-L-homoArg-NH-(C10alkyl), (C12acyl)-D-homoArg-L-homoArg-NH-(C12alkyl), (C14acyl)-D-homoArg-L-homoArg-NH-(C14alkyl), (C16acyl)-D-homoArg-L-homoArg-NH-(C16alkyl), (C18acyl)-D-homoArg-L-homoArg-NH-(C18alkyl), (C10acyl)-D-norArg-L-norArg-NH-(C10alkyl), (C12acyl)-D-norArg-L-norArg-NH-(C12alkyl), (C14acyl)-D-norArg-L-norArg-NH-(C 14alkyl), (C16acyl)-D-norArg-L-norArg-NH-(C16alkyl), (C18acyl)-D-norArg-L-norArg-NH-(C18alkyl), (C10acyl)-D-nornorArg-L-nornorArg-NH-(C10alkyl), (C12acyl)-D-nornorArg-L-nornorArg-NH-(C12alkyl), (C14acyl)-D-nornorArg-L-nornorArg-NH-(C14alkyl), (C16acyl)-D-nornorArg-L-nornorArg-NH-(C 16alkyl), (C18acyl)-D-nornorArg-L-nornorArg-NH-(C18alkyl).

Examples of DILA2 amino acid compounds include (C10acyl)-His-Arg-NH-(C10alkyl), (C12acyl)-His-Arg-NH-(C12alkyl), (C14acyl)-His-Arg-NH-(C14alkyl), (C16acyl)-His-Arg-NH-(C16alkyl), (C18acyl)-His-Arg-NH-(C18alkyl), (C10acyl)-His-Arg-NH-(C10alkyl), (C12acyl)-His-Arg-NH-(C12alkyl), (C14acyl)-His-Arg-NH-(C14alkyl), (C16acyl)-His-Arg-NH-(C16alkyl), (C18acyl)-His-Arg-NH-(C18alkyl), (C10acyl)-His-Arg-(C10alkyl), (C12acyl)-His-Arg-NH-(C12alkyl), (C14acyl)-His-Arg-NH-(C14alkyl), (C16acyl)-His-Arg-NH-(C16alkyl), (C18acyl)-His-Arg-NH-(C18alkyl), (C10acyl)-His-Arg-NH-(C10alkyl), (C12acyl)-His-Arg-NH-(C12alkyl), (C14acyl)-His-Arg-NH-(C14alkyl), (C16acyl)-His-Arg-NH-(C16alkyl), (C18acyl)-His-Arg-NH-(C18alkyl).

Examples of DILA2 amino acid compounds include (C10acyl)-His-Asp-NH-(C10alkyl), (C12acyl)-His-Asp-NH-(C12alkyl), (C14acyl)-His-Asp-NH-(C14alkyl), (C16acyl)-His-Asp-NH-(C16alkyl), (C18acyl)-His-Asp-NH-(C18alkyl), (C10acyl)-His-Asp-NH-(C10alkyl), (C12acyl)-His-Asp-NH-(C12alkyl), (C14acyl)-His-Asp-NH-(C14alkyl), (C16acyl)-His-Asp-NH-(C16alkyl), (C18acyl)-His-Asp-NH-(C18alkyl), (C10acyl)-His-Asp-(C10alkyl), (C12acyl)-His-Asp-NH-(C12alkyl), (C14acyl)-His-Asp-NH-(C14alkyl), (C16acyl)-His-Asp-NH-(C16alkyl), (C18acyl)-His-Asp-NH-(C18alkyl), (C10acyl)-His-Asp-NH-(C10alkyl), (C12acyl)-His-Asp-NH-(C12alkyl), (C14acyl)-His-Asp-NH-(C14alkyl), (C16acyl)-His-Asp-NH-(C16alkyl), (C18acyl)-His-Asp-NH-(C18alkyl).

Examples of DILA2 amino acid compounds include (C10acyl)-Pal-Arg-NH-(C10alkyl), (C12acyl)-Pal-Arg-NH-(C12alkyl), (C14acyl)-Pal-Arg-NH-(C14alkyl), (C16acyl)-Pal-Arg-NH-(C16alkyl), (C18acyl)-Pal-Arg-NH-(C18alkyl), (C10acyl)-Pal-Arg-NH-(C10alkyl), (C12acyl)-Pal-Arg-NH-(C12alkyl), (C14acyl)-Pal-Arg-NH-(C14alkyl), (C16acyl)-Pal-Arg-NH-(C16alkyl), (C18acyl)-Pal-Arg-NH-(C18alkyl), (C10acyl)-Pal-Arg-(C10alkyl), (C12acyl)-Pal-Arg-NH-(C12alkyl), (C14acyl)-Pal-Arg-NH-(C14alkyl), (C16acyl)-Pal-Arg-NH-(C16alkyl), (C18acyl)-Pal-Arg-NH-(C18alkyl), (C10acyl)-Pal-Arg-NH-(C10alkyl), (C12acyl)-Pal-Arg-NH-(C12alkyl), (C14acyl)-Pal-Arg-NH-(C14alkyl), (C16acyl)-Pal-Arg-NH-(C16alkyl), (C18acyl)-Pal-Arg-NH-(C18alkyl).

DILA2 amino acid compounds can be prepared as poly-mer or multi-mer species, such as dimers, trimers, or tetramers. The poly-mer or multi-mer species can be prepared from a single DILA2 amino acid compound, or from more than one species. Poly-mer or multi-mer DILA2 amino acid compounds can be prepared in some embodiments by providing a sulfhydryl group or other cross-linkable group on a side chain of the amino acid, or with linked or tethered amino acid structures such as desmosine or citrulline. In other embodiments, a poly-mer or multi-mer DILA2 amino acid compound can be prepared with bioconjugate linker chemistries.

Examples of DILA2 amino acid compounds include the following structures:

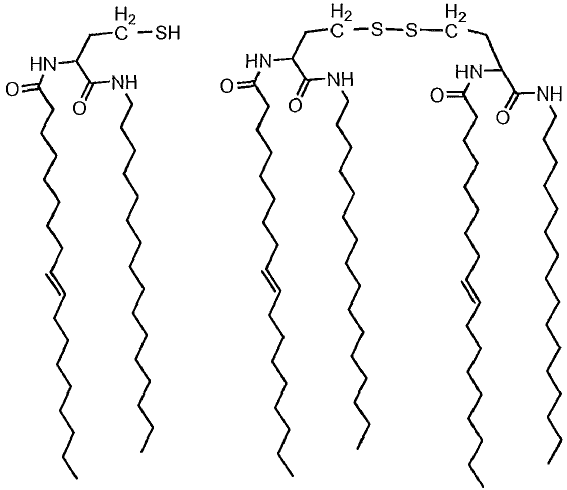

A DILA2 amino acid compound can be prepared as a conjugate having a peptide or polymer chain covalently attached to the amino acid side chain. The peptide or polymer chain can be attached using a reactive group of the amino acid side chain, for example, using the thiol or methylmercaptan group of cysteine or methionine, respectively, or the alcohol group of serine, or the amino group of lysine. The peptide or polymer chain can be attached using any reactive group of a substituted or modified amino acid side chain. Various linker groups such as NHS, maleimido, and bioconjugate techniques and linkers can be used.

DILA2 amino acid compounds can be prepared as constructs attached to an oligomeric or polymeric framework. For example, a DILA2 amino acid compound can be attached to polyethylene glycol, polypropylene glycol, an oilgonucleotide network or lattice, a poly(amino acid), a carbohydrate, a dextran, a hydrogel, or a starch.

DILA2 amino acid compounds can be prepared as constructs attached to a pharmaceutical drug compound or composition, or a biologically active agent. For example, a DILA2 amino acid compound can be conjugated to a nucleic acid drug such as a regulatory or interfering RNA.

Examples of DILA2 amino acid compounds include the following structures: 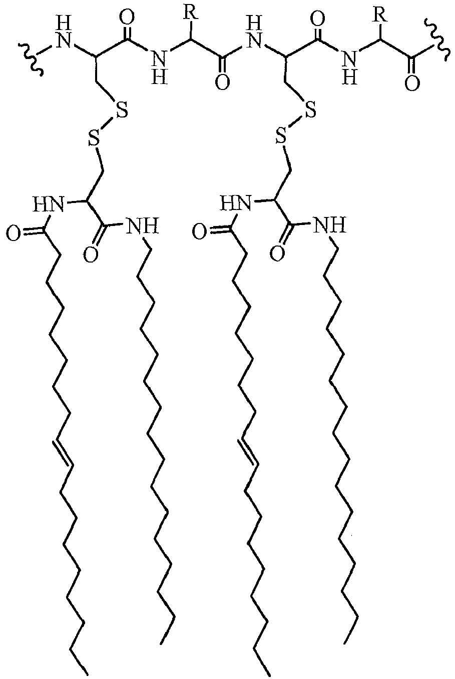 where R is any amino acid side chain.

The compounds and compositions of this disclosure may incorporate solubilizing or functionalizing groups or structures including polymeric structures. See, e.g., R. L. Dunn and R. M. Ottenbrite, Polymeric Drugs and Drug Delivery Systems, ACS Symp. Ser. 469 (1991). DILA2 amino acid compounds can be derivatized to enhance solubility such as, for example, to attach a diol, to prepare a quaternary ammonium or charged group, to attach hydroxyl or amine groups such as alcohols, polyols, or polyethers, or to attach a polyethyleneimine, a polyethyleneglycol or a polypropyleneglycol. The molecular mass of an attached polymeric component such as a polyethyleneglycol can be any value, for example, 200, 300, 400, 500, 750, 1000, 1250, 1500, 2000, 3000, 4000, 5000, 7500, 10,000, 15,000, 20,000, 25,000, or 30,000 Da, or greater. For example, a polyethyleneglycol chain can be attached through an amino group or other reactive group of an amino acid side chain.

In general, as used herein, general chemical terms refer to all groups of a specified type, including groups having any number and type of atoms, unless otherwise specified. For example "alkenyl" refers broadly to alkyls having 2 to 22 carbon atoms, as defined below, while (C18:1)alkenyl refers to alkenyls having 18 carbon atoms and one double bond.

The term "alkyl" as used herein refers to a saturated, branched or unbranched, substituted or unsubstituted aliphatic group containing from 1-22 carbon atoms. This definition applies to the alkyl portion of other groups such as, for example, alkoxy, alkanoyl, aralkyl, and other groups defined below. The term "cycloalkyl" as used herein refers to a saturated, substituted or unsubstituted cyclic alkyl ring containing from 3 to 12 carbon atoms.

The term "alkenyl" as used herein refers to an unsaturated, branched or unbranched, substituted or unsubstituted alkyl or cycloalkyl having 2 to 22 carbon atoms and at least one carbon-carbon double bond. The term "alkynyl" as used herein refers to an unsaturated, branched or unbranched, substituted or unsubstituted alkyl or cycloalkyl having 2 to 22 carbon atoms and at least one carbon-carbon triple bond.

The term "alkoxy" as used herein refers to an alkyl, cycloalkyl, alkenyl, or alkynyl group covalently bonded to an oxygen atom. The term "alkanoyl" as used herein refers to -C(=O)-alkyl, which may alternatively be referred to as "acyl." The term "alkanoyloxy" as used herein refers to -O-C(=O)-alkyl groups. The term "alkylamino" as used herein refers to the group -NRR', where R and R' are each either hydrogen or alkyl, and at least one of R and R' is alkyl. Alkylamino includes groups such as piperidino wherein R and R' form a ring. The term "alkylaminoalkyl" refers to -alkyl-NRR'.

The term "aryl" as used herein refers to any stable monocyclic, bicyclic, or polycyclic carbon ring system of from 4 to 12 atoms in each ring, wherein at least one ring is aromatic. Some examples of an aryl include phenyl, naphthyl, tetrahydronaphthyl, indanyl, and biphenyl. Where an aryl substituent is bicyclic and one ring is non-aromatic, it is understood that attachment is to the aromatic ring. An aryl may be substituted or unsubstituted.

The term "heteroaryl" as used herein refers to any stable monocyclic, bicyclic, or polycyclic carbon ring system of from 4 to 12 atoms in each ring, wherein at least one ring is aromatic and contains from 1 to 4 heteroatoms selected from oxygen, nitrogen and sulfur. Some examples of a heteroaryl include acridinyl, quinoxalinyl, pyrazolyl, indolyl, benzotriazolyl, furanyl, thienyl, benzothienyl, benzofuranyl, quinolinyl, isoquinolinyl, oxazolyl, isoxazolyl, pyrazinyl, pyridazinyl, pyridinyl, pyrimidinyl, pyrrolyl, and tetrahydroquinolinyl. A heteroaryl includes the N-oxide derivative of a nitrogen-containing heteroaryl.

The term "heterocycle" or "heterocyclyl" as used herein refers to an aromatic or nonaromatic ring system of from five to twenty-two atoms, wherein from 1 to 4 of the ring atoms are heteroatoms selected from oxygen, nitrogen, and sulfur. Thus, a heterocycle may be a heteroaryl or a dihydro or tetrathydro version thereof.

The term "aroyl" as used herein refers to an aryl radical derived from an aromatic carboxylic acid, such as a substituted benzoic acid. The term "aralkyl" as used herein refers to an aryl group bonded to an alkyl group, for example, a benzyl group.

The term "carboxyl" as used herein represents a group of the formula -C(=O)OH or -C(=O)O⁻. The terms "carbonyl" and "acyl" as used herein refer to a group in which an oxygen atom is double-bonded to a carbon atom >C=O. The term "hydroxyl" as used herein refers to -OH or -O⁻. The term "nitrile" or "cyano" as used herein refers to -CN. The term "halogen" or "halo" refers to fluoro (-F), chloro (-Cl), bromo (-Br), and iodo (-I).

The term "substituted" as used herein refers to an atom having one or more substitutions or substituents which can be the same or different and may include a hydrogen substituent. Thus, the terms alkyl, cycloalkyl, alkenyl, alkynyl, alkoxy, alkanoyl, alkanoyloxy, alkylamino, alkylaminoalkyl, aryl, heteroaryl, heterocycle, aroyl, and aralkyl as used herein refer to groups which include substituted variations. Substituted variations include linear, branched, and cyclic variations, and groups having a substituent or substituents replacing one or more hydrogens attached to any carbon atom of the group. Substituents that may be attached to a carbon atom of the group include alkyl, cycloalkyl, alkenyl, alkynyl, alkoxy, alkanoyl, alkanoyloxy, alkylamino, alkylaminoalkyl, aryl, heteroaryl, heterocycle, aroyl, aralkyl, acyl, hydroxyl, cyano, halo, haloalkyl, amino, aminoacyl, alkylaminoacyl, acyloxy, aryloxy, aryloxyalkyl, mercapto, nitro, carbamyl, carbamoyl, and heterocycle. For example, the term ethyl includes without limitation -CH₂CH₃, -CHFCH₃, -CF₂CH₃, -CHFCH₂F, -CHFCHF₂, -CHFCF₃, -CF₂CH₂F, -CF₂CHF₂, -CF₂CF₃, and other variations as described above. In general, substituents may be further substituted with any atom or group of atoms.

DILA2 amino acid compounds can be synthesized by methods known in the art.

Methods to prepare various organic groups and protective groups are known in the art and their use and modification is generally within the ability of one of skill in the art. See, e.g., Stanley R. Sandler and Wolf Karo, Organic Functional Group Preparations (1989); Greg T. Hermanson, Bioconjugate Techniques (1996); Leroy G. Wade, Compendium Of Organic Synthetic Methods (1980); examples of protective groups are found in T. W. Greene and P. G. M. Wuts, Protective Groups In Organic Synthesis (3rd ed. 1991).

For example, the DILA2 amino acid compound PONA can be synthesized according to the following scheme:

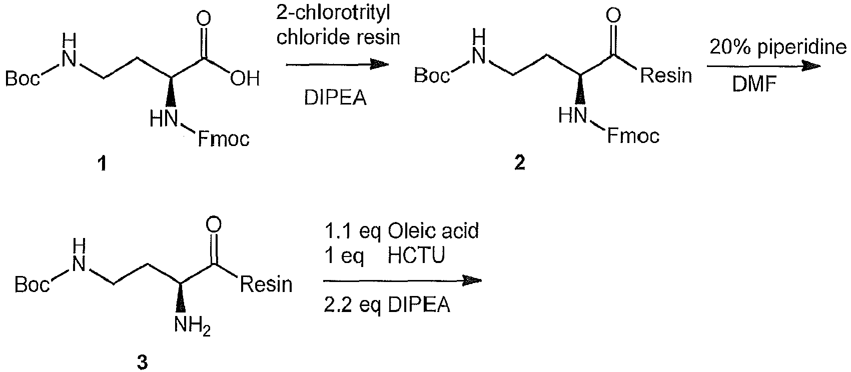

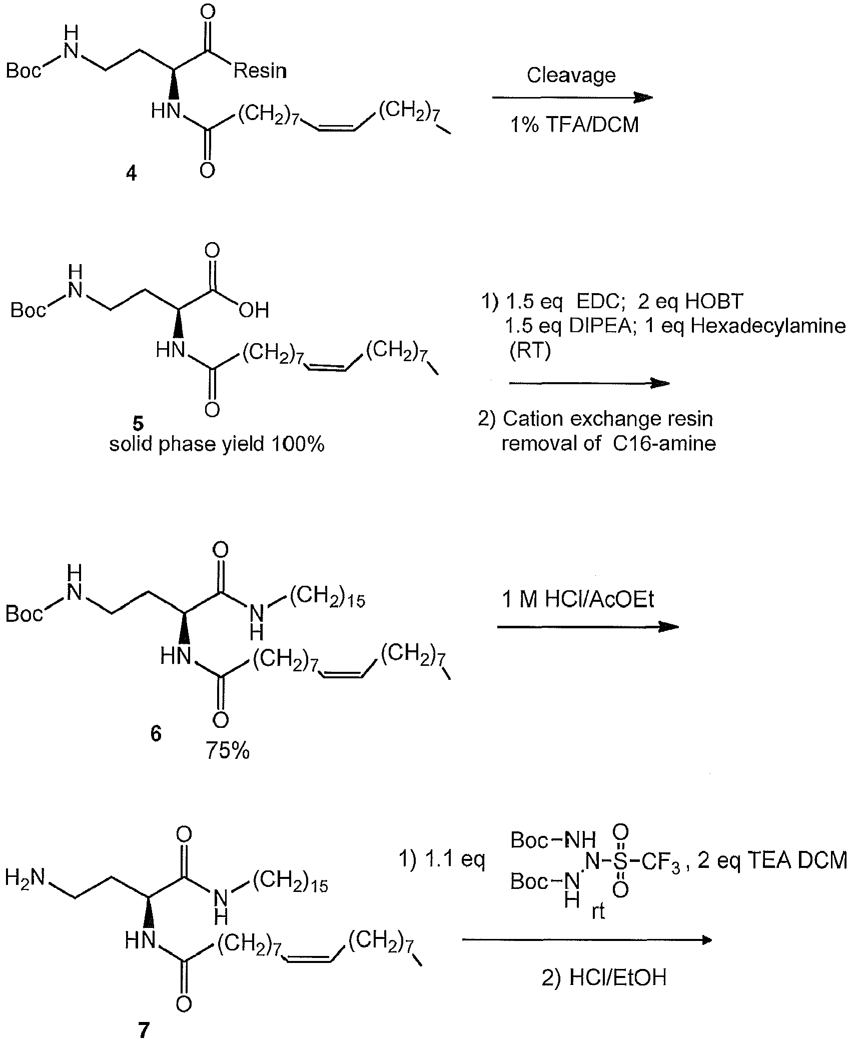

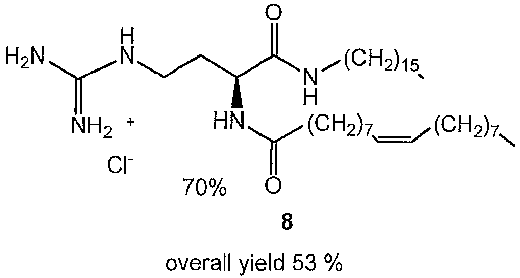

A pharmaceutically acceptable salt of a peptide or protein composition of this invention which is sufficiently basic may be an acid-addition salt with, for example, an inorganic or organic acid such as hydrochloric, hydrobromic, sulfuric, nitric, phosphoric, chlorosulfonic, trifluoroacetic, citric, maleic, acetic, propionic, oxalic, malic, maleic, malonic, fumaric, or tartaric acids, and alkane- or arenesulfonic acids such as methanesulfonic, ethanesulfonic, benzenesulfonic, chlorobenzenesulfonic, toluenesulfonic, naphthalenesulfonic, naphthalenedisulfonic, and camphorsulfonic acids.

A pharmaceutically acceptable salt of a peptide or protein composition of this invention which is sufficiently acidic may be an alkali metal salt, for example, a sodium or potassium salt, or an alkaline earth metal salt, for example, a calcium or magnesium salt, or a zinc or manganese salt, or an ammonium salt or a salt with an organic base which provides a physiologically-acceptable cation, for example, a salt with methylamine, dimethylamine, trimethylamine, triethylamine, ethanolamine, diethanolamine, triethanolamine, ethylenediamine, tromethamine, N-methylglucamine, piperidine, morpholine or tris-(2-hydroxyethyl)amine, and including salts of amino acids such as arginate, and salts of organic acids such as glucuronic or galactunoric acids. *See,* for example, Berge et al., J. Pharm. Sci. 66:1-19, 1977.

A salt or pharmaceutically-acceptable salt of a composition of this disclosure which contains an interfering-RNA agent and a DILA2 amino acid compound, a lipid, a peptide, or protein, among other components, may contain a salt complex of the interfering-RNA agent and the DILA2 amino acid compound, lipid, peptide, or protein. A salt complex of the interfering-RNA agent and the DILA2 amino acid compound, lipid, peptide, or protein may be formed from a pharmaceutically-acceptable salt of an interfering-RNA agent, or from a pharmaceutically-acceptable salt of the DILA2 amino acid compound, lipid, peptide, or protein.

Some compounds of this disclosure may contain both basic and acidic functionalities that may allow the compounds to be made into either a base or acid addition salt.

Some compounds, peptides and/or protein compositions of this invention may have one or more chiral centers and/or geometric isomeric centers (E- and Z-isomers), and it is to be understood that the invention encompasses all such optical isomers, diastereoisomers, geometric isomers, and mixtures thereof.

This disclosure encompasses any and all tautomeric, solvated or unsolvated, hydrated or unhydrated forms, as well as any atom isotope forms of the compounds, peptides and/or protein compositions disclosed herein.

### Lipids

In some aspects of this invention, one or more DILA2 amino acid compounds and one or more lipids may be employed for delivery and administration of regulatory RNA components, RNA antagonists, interfering RNA, or nucleic acids. More particularly, a composition of this invention may include one or more DILA2 amino acid compounds along with cationic lipids and non-cationic lipids.

Cationic lipids may be monocationic or polycationic. Some cationic lipids include neutral lipids and lipids having approximately zero net charge at a particular pH, for example, a zwitterionic lipid. Non-cationic lipids also include anionic lipids.

In some embodiments, a composition is a mixture or complex of an RNA component with a DILA2 amino acid compound and a cationic lipid. In some embodiments, a composition may be a mixture or complex of one or more regulatory or interfering RNA agents with one or more DILA2 amino acid compounds and one or more cationic lipids.

The compounds and compositions of this disclosure can be admixed with, or attached to various targeting ligands or agents to deliver an active agent to a cell, tissue, organ or region of an organism. Examples of targeting agents include antibodies, ligands for receptors, peptides, proteins, lectins, (poly)saccharides, galactose, mannose, cyclodextrins, nucleic acids, DNA, RNA, aptamers, and polyamino acids.

Examples of cationic lipids include N-[1-(2,3-dioleoyloxy)propyl]-N,N,N-trimethylammonium chloride (DOTMA); 1,2-bis(oleoyloxy)-3-3-(trimethylammonium)propane (DOTAP), 1,2-bis(dimyrstoyloxy)-3-3-(trimethylammonia)propane (DMTAP); 1,2-dimyristyloxypropyl-3-dimethylhydroxyethylammonium bromide (DMRIE); dimethyldioctadecylammonium bromide (DDAB); 3-(N-(N',N'-dimethylaminoethane)carbamoyl)cholesterol (DC-Chol); 3β-[N',N'-diguanidinoethyl-aminoethane)carbamoyl cholesterol (BGTC); 2-(2-(3-(bis(3-aminopropyl)amino)propylamino)acetamido)-*N*,*N*-ditetradecylacetamide (RPR209120); pharmaceutically acceptable salts thereof, and mixtures thereof.

Examples of cationic lipids include 1,2-dialkenoyl-*sn*-glycero-3-ethylphosphocholines (EPCs), such as 1,2-dioleoyl-*sn*-glycero-3-ethylphosphocholine, 1,2-distearoyl-*sn*-glycero-3-ethylphosphocholine, 1,2-dipalmitoyl-*sn*-glycero-3-ethylphosphocholine, pharmaceutically acceptable salts thereof, and mixtures thereof.

Examples of cationic lipids include 1,2-distearyloxy-N,N-dimethyl-3-aminopropane (DSDMA), 1,2-dioleyloxy-N,Ndimethyl-3-aminopropane (DODMA), 1,2-dilinoleyloxy-N,N-dimethyl-3-aminopropane (DLinDMA), and 1,2-dilinolenyloxy-N,N-dimethyl-3-aminopropane (DLenDMA).

Examples of polycationic lipids include tetramethyltetrapalmitoyl spermine (TMTPS), tetramethyltetraoleyl spermine (TMTOS), tetramethlytetralauryl spermine (TMTLS), tetramethyltetramyristyl spermine (TMTMS), tetramethyldioleyl spermine (TMDOS), pharmaceutically acceptable salts thereof, and mixtures thereof.

Examples of polycationic lipids include 2,5-bis(3-aminopropylamino)-N-(2-(dioctadecylamino)-2-oxoethyl) pentanamide (DOGS); 2,5-bis(3-aminopropylamino)-N-(2-(di(Z)-octadeca-9-dienylamino)-2-oxoethyl) pentanamide (DOGS-9-en); 2,5-bis(3-aminopropylamino)-N-(2-(di(9Z,12Z)-octadeca-9,12-dienylamino)-2-oxoethyl) pentanamide (DLinGS); 3-beta-(N⁴-(N¹,N⁸-dicarbobenzoxyspermidine)carbamoyl)cholesterol (GL-67); (9Z,9'Z)-2-(2,5-bis(3-aminopropylamino)pentanamido)propane-1,3-diyl-dioctadec-9-enoate (DOSPER); 2,3-dioleyloxy-N-[2(sperminecarboxamido)ethyl]-N,N-dimethyl-1-propanaminium trifluoro-acetate (DOSPA); pharmaceutically acceptable salts thereof, and mixtures thereof.

Examples of cationic lipids include DS404-28 BGTC (CAS 182056-06-0), DOSPER (CAS 178532-92-8), GL-67 (179075-30-0), RPR209120 (CAS 433292-13-8), DOGS (12050-77-7), DOGS (9-en, C18:1), DLinGS (C18:2), and DOTMA (104162-48-3).

Examples of cationic lipids are described in U.S. Patent Nos. 4,897,355; 5,279,833; 6,733,777; 6,376,248; 5,736,392; 5,334,761; 5,459,127; 2005/0064595; 5,208,036; 5,264,618; 5,279,833; 5,283,185; 5,753,613; and 5,785,992.

In some embodiments, the composition is a mixture or complex of an RNA component with a DILA2 amino acid compound and a non-cationic lipid. In some embodiments, the composition is a mixture or complex of one or more RNA components with one or more DILA2 amino acid compounds and one or more non-cationic lipids.

Non-cationic lipids include neutral, zwitterionic, and anionic lipids. Thus, a non-cationic zwitterionic lipid may contain a cationic head group.

Examples of non-cationic lipids include 1,2-Dilauroyl-sn-glycerol (DLG); 1,2-Dimyristoyl-sn-glycerol (DMG); 1,2-Dipalmitoyl-sn-glycerol (DPG); 1,2-Distearoyl-sn-glycerol (DSG); 1,2-Dilauroyl-sn-glycero-3-phosphatidic acid (sodium salt; DLPA); 1,2-Dimyristoyl-sn-glycero-3-phosphatidic acid (sodium salt; DMPA); 1,2-Dipalmitoyl-sn-glycero-3-phosphatidic acid (sodium salt; DPPA); 1,2-Distearoyl-sn-glycero-3-phosphatidic acid (sodium salt; DSPA); 1,2-Diarachidoyl-sn-glycero-3-phosphocholine (DAPC); 1,2-Dilauroyl-sn-glycero-3-phosphocholine (DLPC); 1,2-Dimyristoyl-sn-glycero-3-phosphocholine (DMPC); 1,2-Dipalmitoyl-sn-glycero-0-ethyl-3-phosphocholine (chloride or triflate; DPePC); 1,2-Dipalmitoyl-sn-glycero-3-phosphocholine (DPPC); 1,2-Distearoyl- sn-glycero-3-phosphocholine (DSPC); 1,2-Dilauroyl-sn-glycero-3-phosphoethanolamine (DLPE); 1,2-Dimyristoyl-sn-glycero-3-phosphoethanolamine (DMPE); 1,2-Dipalmitoyl-sn-glycero-3-phosphoethanolamine (DPPE); 1,2-Distearoyl-sn-glycero-3-phosphoethanolamine (DSPE); 1,2-Dilauroyl-sn-glycero-3-phosphoglycerol (sodium salt; DLPG); 1,2-Dimyristoyl-sn-glycero-3-phosphoglycerol (sodium salt; DMPG); 1,2-Dimyristoyl-sn-glycero-3-phospho-sn-1-glycerol (ammonium salt; DMP-sn-1-G); 1,2-Dipalmitoyl-sn-glycero-3-phosphoglycerol (sodium salt; DPPG); 1,2-Distearoyl-sn-glycero-3-phosphoglycero (sodium salt; DSPG); 1,2-Distearoyl-sn-glycero-3-phospho-sn-1-glycerol (sodium salt; DSP-sn-1-G); 1,2-Dipalmitoyl-sn-glycero-3-phospho-L-serine (sodium salt; DPPS); 1-Palmitoyl-2-linoleoyl-sn-glycero-3-phosphocholine (PLinoPC); 1-Palmitoyl-2-oleoyl-sn-glycero-3-phosphocholine (POPC); 1-Palmitoyl-2-oleoyl-sn-glycero-3-phosphoglycerol (sodium salt; POPG); 1-Palmitoyl-2-oleoyl-sn-glycero-3-phosphoglycerol (sodium salt; POPG); 1-Palmitoyl-2-oleoyl-sn-glycero-3-phosphoglycerol (ammonium salt; POPG); 1-Palmitoyl-2-4o-sn-glycero-3-phosphocholine (P-lyso-PC); 1-Stearoyl-2-lyso-sn-glycero-3-phosphocholine (S-lyso-PC); and mixtures thereof.

Examples of non-cationic lipids include polymeric compounds and polymer-lipid conjugates or polymeric lipids, such as pegylated lipids having PEG regions of 300, 500, 1000, 1500, 2000, 3500, or 5000 molecular weight, including polyethyleneglycols, N-(Carbonyl-methoxypolyethyleneglycol-2000)-1,2-dimyristoyl-sn-glycero-3-phosphoethanolamine (sodium salt; DMPE-MPEG-2000); N-(Carbonyl-methoxypolyethyleneglycol-5000)-1,2-dimyristoyl-sn-glycero-3-phosphoethanolamine (sodium salt; DMPE-MPEG-5000); N-(Carbonyl-methoxypolyethyleneglycol 2000)-1,2-dipalmitoyl-sn-glycero-3-phosphoethanolamine (sodium salt; DPPE-MPEG-2000); N-(Carbonyl-methoxypolyethyleneglycol 5000)-1,2-dipalmitoyl-sn-glycero-3-phosphoethanolamine (sodium salt; DPPE-MPEG-5000); N-(Carbonyl-methoxypolyethyleneglycol 750)-1,2-distearoyl-sn-glycero-3-phosphoethanolamine (sodium salt; DSPE-MPEG-750); N-(Carbonyl-methoxypolyethyleneglycol 2000)-1,2-distearoyl-sn-glycero-3-phosphoethanolamine (sodium salt; DSPE-MPEG-2000); N-(Carbonyl-methoxypolyethyleneglycol 5000)-1,2-distearoyl-sn-glycero-3-phosphoethanolamine (sodium salt; DSPE-MPEG-5000); sodium cholesteryl sulfate (SCS); pharmaceutically acceptable salts thereof, and mixtures thereof.

Examples of non-cationic lipids include polymeric lipids such as DOPE-PEG, DLPE-PEG, DDPE-PEG DLinPE-PEG, and diacylglycerol-PEG-2000 or -5000.

Examples of non-cationic lipids include polymeric lipids such as multi-branched pegylated compounds, for example DSPE-PTE020 and DSPE-AM0530K.

Examples of non-cationic lipids include polymeric lipids such as DSPE-PG8G polyglycerine lipids.

Examples of non-cationic lipids include dioleoylphosphatidylethanolamine (DOPE), diphytanoylphosphatidylethanolamine (DPhPE), 1,2-Dioleoyl-*sn*-Glycero-3-Phosphocholine (DOPC), and 1,2-Diphytanoyl-*sn*-Glycero-3-Phosphocholine (DPhPC).

Examples of non-cationic lipids include cholesterols, sterols, and steroids such as gonanes, estranes, androstanes, pregnanes, cholanes, cholestanes, ergostanes, campestanes, poriferastanes, stigmastanes, gorgostanes, lanostanes, cycloartanes, as well as sterol or zoosterol derivatives of any of the foregoing, and their biological intermediates and precursors, which may include, for example, cholesterol, lanosterol, stigmastanol, dihydrolanosterol, zymosterol, zymostenol, desmosterol, 7-dehydrocholesterol, and mixtures and derivatives thereof.

Examples of non-cationic lipids include pegylated cholesterols, and cholestane 3-oxo(C1-22acyl) derivatives such as cholesteryl acetate, cholesteryl arachidonate, cholesteryl butyrate, cholesteryl hexanoate, cholesteryl caprylate, cholesteryl n-decanoate, cholesteryl dodecanoate, cholesteryl myristate, cholesteryl palmitate, cholesteryl behenate, cholesteryl stearate, cholesteryl nervonate, cholesteryl pelargonate, cholesteryl n-valerate, cholesteryl oleate, cholesteryl elaidate, cholesteryl erucate, cholesteryl heptanoate, cholesteryl linolelaidate, cholesteryl linoleate, and mixtures and derivatives thereof.

Examples of non-cationic lipids include compounds derived from plant sterols including phytosterols, beta-sitosterol, campesterol, ergosterol, brassicasterol, delta-7-stigmasterol, delta-7-avenasterol, and mixtures and derivatives thereof.

Examples of non-cationic lipids include bile acids, cholic acid, chenodeoxycholic acid, glycocholic acid, taurocholic acid, deoxycholic acid, lithocholic acid, methyl-lithocholic acid, and mixtures and derivatives thereof.

Examples of non-cationic lipids include compounds derived from steroids including glucocorticoids, cortisol, hydrocortisone, corticosterone, Δ⁵-pregnenolone, progesterone, deoxycorticosterone, 17-OH-pregnenolone, 17-OH-progesterone, 11-dioxycortisol, dehydroepiandrosterone, dehydroepiandrosterone sulfate, androstenedione, aldosterone, 18-hydroxycorticosterone, tetrahydrocortisol, tetrahydrocortisone, cortisone, prednisone, 6α-methylpredisone, 9α-fluoro-16α-hydroxyprednisolone, 9α-fluoro-16α-methylprednisolone, 9α-fluorocortisol, and mixtures and derivatives thereof.

Examples of non-cationic lipids include compounds derived from steroids including adrogens, testosterone, dihydrotestosterone, androstenediol, androstenedione, androstenedione, 3α,5α-androstanediol, and mixtures and derivatives thereof.

Examples of non-cationic lipids include compounds derived from steroids including estrogens, estriols, estrones, estradiols, and mixtures and derivatives thereof.

Examples of non-cationic lipids include compounds derived from lumisterol and vitamin D compounds.

Examples of non-cationic lipids include lipids having tails ranging from C10:0 to C22:6, for example, DDPE (C10:0) (CAS 253685-27-7), DLPE (C12:0) (CAS 59752-57-7), DSPE (C18:0) (CAS 1069-79-0), DOPE (C18:1) (CAS 4004-05-1), DLinPE (C18:2) (CAS 20707-71-5), DLenPE (C18:3) (CAS 34813-40-6), DARAPE (C20:4) (CAS 5634-86-6), DDHAPE (C22:6) (CAS 123284-81-1), DPhPE (16:0[(CH₃)₄]) (CAS 201036-16-0).

Examples of anionic lipids include phosphatidylserine, phosphatidic acid, phosphatidylcholine, platelet-activation factor (PAF), phosphatidylethanolamine, phosphatidyl-DL-glycerol, phosphatidylinositol, phosphatidylinositol (pi(4)p, pi(4,5)p2), cardiolipin (sodium salt), lysophosphatides, hydrogenated phospholipids, sphingoplipids, gangliosides, phytosphingosine, sphinganines, pharmaceutically acceptable salts thereof, and mixtures thereof.

### Uses for delivering RNA therapeutics

In some aspects, this disclosure relates generally to the fields of regulatory RNA and RNA interference, antisense therapeutics, and delivery of RNA therapeutics. More particularly, this invention relates to compositions and formulations for ribonucleic acids, and their uses for medicaments and for delivery as therapeutics. This invention relates generally to methods of using ribonucleic acids in RNA interference for gene-specific inhibition of gene expression in cells, or in mammals to alter a disease state or a phenotype.

RNA interference refers to methods of sequence-specific post-transcriptional gene silencing which is mediated by a double-stranded RNA (dsRNA) called a short interfering RNA (siRNA). *See* Fire, et al., Nature 391:806, 1998, and Hamilton, et al., Science 286:950-951, 1999. RNAi is shared by diverse flora and phyla and is believed to be an evolutionarily-conserved cellular defense mechanism against the expression of foreign genes. *See* Fire, et al., Trends Genet. 15:358, 1999.

RNAi is therefore a ubiquitous, endogenous mechanism that uses small noncoding RNAs to silence gene expression. *See* Dykxhoorn, D.M. and J. Lieberman, Annu. Rev. Biomed. Eng. 8:377-402, 2006. RNAi can regulate important genes involved in cell death, differentiation, and development. RNAi may also protect the genome from invading genetic elements, encoded by transposons and viruses. When a siRNA is introduced into a cell, it binds to the endogenous RNAi machinery to disrupt the expression of mRNA containing complementary sequences with high specificity. Any disease-causing gene and any cell type or tissue can potentially be targeted. This technique has been rapidly utilized for gene-function analysis and drug-target discovery and validation. Harnessing RNAi also holds great promise for therapy, although introducing siRNAs into cells in vivo remains an important obstacle.

The mechanism of RNAi, although not yet fully characterized, is through cleavage of a target mRNA. The RNAi response involves an endonuclease complex known as the RNA-induced silencing complex (RISC), which mediates cleavage of a single-stranded RNA complementary to the antisense strand of the siRNA duplex. Cleavage of the target RNA takes place in the middle of the region complementary to the antisense strand of the siRNA duplex (Elbashir, et al., Genes Dev. 15:188, 2001).

One way to carry out RNAi is to introduce or express a siRNA in cells. Another way is to make use of an endogenous ribonuclease III enzyme called dicer. One activity of dicer is to process a long dsRNA into siRNAs. *See* Hamilton, et al., Science 286:950-951, 1999; Berstein, et al., Nature 409:363, 2001. A siRNA derived from dicer is typically about 21-23 nucleotides in overall length with about 19 base pairs duplexed. *See* Hamilton, *et al., supra;* Elbashir, et al., Genes Dev. 15:188, 2001. In essence, a long dsRNA can be introduced in a cell as a precursor of a siRNA.

This invention provides a range of compositions, formulations and methods which include a regulatory RNA, an interfering nucleic acid or a precursor thereof in combination with various components including DILA2 amino acid compounds, lipids, and natural or synthetic polymers.

The term "dsRNA" as used herein refers to any double-stranded RNA molecule capable of inhibiting or down regulating gene expression, for example, by promoting RNA interference ("RNAi" or "iRNA") or gene silencing in a sequence-specific manner. The dsRNAs of this disclosure may be suitable substrates for Dicer or for association with RISC to mediate gene silencing by RNAi. One or both strands of the dsRNA can further comprise a terminal phosphate group, such as a 5'-phosphate or 5', 3'-diphosphate. As used herein, dsRNA molecules, in addition to at least one ribonucleotide, can further include substitutions, chemically-modified nucleotides, and non-nucleotides.

Examples of dsRNA molecules can be found in, for example, U.S. Patent Application No. 11/681,725, U.S. Patent Nos. 7,022,828 and 7,034,009, and PCT International Application Publication No. WO/2003/070897.

Examples of a nucleic acid agent of this disclosure may contain one or more acyclic monomers described in PCT International Application Publication No. WO2008/147824. Examples of an acyclic monomer include monomers D through J shown in Figs. 1 and 2 of WO2008/147824.

Examples of an active agent of this disclosure include nucleic acid molecules containing an acyclic monomer of WO2008/147824.

Examples of an active agent of this disclosure include UsiRNAs. A UsiRNA is a UNA-containing siRNA, where a UNA is an "unlocked nucleobase analog." The acyclic monomers D through J of WO2008/147824 are examples of UNAs.

Examples of modified nucleosides are found in U.S. Patent Nos. 6,403,566, 6,509,320, 6,479,463, 6,191,266, 6,083,482, 5,712,378, and 5,681,940. A modified nucleoside may have the following structure: 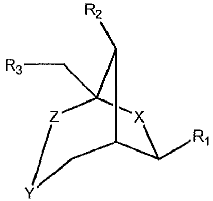 wherein, X is O or CH₂, Y is O, and Z is CH₂; R₁ is selected from the group of adenine, cytosine, guanine, hypoxanthine, uracil, thymine, and a heterocycle wherein the heterocycle is selected from the group of a substituted 1,3-diazine, an unsubstituted 1,3-diazine, and an unsubstituted 7H imidazo[4,5]1,3 diazine; and R₂, R₃ are independently selected from the group of H, OH, DMTO, TBDMSO, BnO, THPO, AcO, BzO, OP(NiPr₂)O(CH₂)₂CN, OPO₃ H, diphosphate, and triphosphate, wherein R₂ and R₃ together may be PhCHO₂, TIPDSO₂ or DTBSO₂. As used herein, the abbreviation "Ac" refers to acetyl; the abbreviation "Bn" refers to benzyl; the abbreviation "Bz" refers to benzoyl; the abbreviation "DMT" refers to dimethoxytrityl; the abbreviation "THP" refers to tetrahydropyranyl; the abbreviation "TBDMS" refers to t-butyldimethylsilyl; the abbreviation "TIPDS" refers to tetraisopropyldisilyl; and the abbreviation "DTBS" refers to di(t-butyl)silyl.

In addition, as used herein, the terms "dsRNA," "RNAi-inducing agent, "and "RNAi-agent" are meant to be synonymous with other terms used to describe nucleic acid molecules that are capable of mediating sequence specific RNAi including meroduplex RNA (mdRNA), nicked dsRNA (ndsRNA), gapped dsRNA (gdsRNA), short interfering nucleic acid (siRNA), siRNA, microRNA (miRNA), single strand RNA, short hairpin RNA (shRNA), short interfering oligonucleotide, short interfering substituted oligonucleotide, short interfering modified oligonucleotide, chemically-modified dsRNA, and post-transcriptional gene silencing RNA (ptgsRNA), as well as precursors of any of the above.

The term "large double-stranded (ds) RNA" refers to any double-stranded RNA longer than about 40 base pairs (bp) to about 100 bp or more, particularly up to about 300 bp to about 500 bp. The sequence of a large dsRNA may represent a segment of an mRNA or an entire mRNA. A double-stranded structure may be formed by self-complementary nucleic acid molecule or by annealing of two or more distinct complementary nucleic acid molecule strands.

In some aspects, a dsRNA comprises two separate oligonucleotides, comprising a first strand (antisense) and a second strand (sense), wherein the antisense and sense strands are self-complementary *(i.e.,* each strand comprises a nucleotide sequence that is complementary to a nucleotide sequence in the other strand and the two separate strands form a duplex or double-stranded structure, for example, wherein the double-stranded region is about 15 to about 24 base pairs or about 26 to about 40 base pairs); the antisense strand comprises a nucleotide sequence that is complementary to a nucleotide sequence in a target nucleic acid molecule or a portion thereof *(e.g.,* a human mRNA); and the sense strand comprises a nucleotide sequence corresponding *(i.e.,* homologous) to the target nucleic acid sequence or a portion thereof *(e.g.,* a sense strand of about 15 to about 25 nucleotides or about 26 to about 40 nucleotides corresponds to the target nucleic acid or a portion thereof).

In some embodiments, the dsRNA may be assembled from a single oligonucleotide in which the self-complementary sense and antisense strands of the dsRNA are linked by together by a nucleic acid based-linker or a non-nucleic acid-based linker. In some embodiments, the first (antisense) and second (sense) strands of the dsRNA molecule are covalently linked by a nucleotide or non-nucleotide linker as described herein and known in the art. In some embodiments, a first dsRNA molecule is covalently linked to at least one second dsRNA molecule by a nucleotide or non-nucleotide linker known in the art, wherein the first dsRNA molecule can be linked to a plurality of other dsRNA molecules that can be the same or different, or any combination thereof. In some embodiments, the linked dsRNA may include a third strand that forms a meroduplex with the linked dsRNA.

In some respects, dsRNA molecules described herein form a meroduplex RNA (mdRNA) having three or more strands, for example, an 'A' (first or antisense) strand, 'S1' (second) strand, and 'S2' (third) strand in which the 'S1' and 'S2' strands are complementary to and form base pairs (bp) with non-overlapping regions of the 'A' strand *(e.g.,* an mdRNA can have the form of A:S1S2). The S1, S2, or more strands together essentially comprise a sense strand to the 'A' strand. The double-stranded region formed by the annealing of the 'S1' and 'A' strands is distinct from and non-overlapping with the double-stranded region formed by the annealing of the 'S2' and 'A' strands. An mdRNA molecule is a "gapped" molecule, meaning a "gap" ranging from 0 nucleotides up to about 10 nucleotides. In some embodiments, the A:S1 duplex is separated from the A:S2 duplex by a gap resulting from at least one unpaired nucleotide (up to about 10 unpaired nucleotides) in the 'A' strand that is positioned between the A:S1 duplex and the A:S2 duplex and that is distinct from any one or more unpaired nucleotide at the 3'-end of one or more of the 'A', 'S1', or 'S2' strands. In some embodiments, the A:S1 duplex is separated from the A:B2 duplex by a gap of zero nucleotides (*i.e.,* a nick in which only a phosphodiester bond between two nucleotides is broken or missing in the polynucleotide molecule) between the A:S1 duplex and the A:S2 duplex - which can also be referred to as nicked dsRNA (ndsRNA). For example, A:S1S2 may be comprised of a dsRNA having at least two double-stranded regions that combined total about 14 base pairs to about 40 base pairs and the double-stranded regions are separated by a gap of about 0 to about 10 nucleotides, optionally having blunt ends, or A:S1S2 may comprise a dsRNA having at least two double-stranded regions separated by a gap of up to 10 nucleotides wherein at least one of the double-stranded regions comprises between about 5 base pairs and 13 base pairs.

As described herein, a dsRNA molecule which contains three or more strands may be referred to as a "meroduplex" RNA (mdRNA). Examples of mdRNA molecules can be found in U.S. Provisional Patent Application Nos. 60/934,930 and 60/973,398.

A dsRNA or large dsRNA may include a substitution or modification in which the substitution or modification may be in a phosphate backbone bond, a sugar, a base, or a nucleoside. Such nucleoside substitutions can include natural non-standard nucleosides (*e.g*., 5-methyluridine or 5-methylcytidine or a 2-thioribothymidine), and such backbone, sugar, or nucleoside modifications can include an alkyl or heteroatom substitution or addition, such as a methyl, alkoxyalkyl, halogen, nitrogen or sulfur, or other modifications known in the art.

In addition, as used herein, the term "RNAi" is meant to be equivalent to other terms used to describe sequence specific RNA interference, such as post transcriptional gene silencing, translational inhibition, or epigenetics. For example, dsRNA molecules of this disclosure can be used to epigenetically silence genes at the post-transcriptional level or the pre-transcriptional level or any combination thereof.

In some aspects, this invention provides compositions containing one or more RNAi-inducing agents which are targeted to one or more genes or target transcripts, along with one or more delivery components. Examples of delivery components include DILA2 amino acid compounds, lipids, peptides, polymers, polymeric lipids, and conjugates thereof.

The compositions and formulations of this disclosure may be used for delivery of RNAi-inducing entities such as dsRNA, siRNA, mdRNA, miRNA, shRNA, or RNAi-inducing vectors to cells in intact mammalian subjects, and may also be used for delivery of these agents to cells in culture.

This disclosure also provides methods for the delivery of one or more RNAi-inducing agents or entities to cells, organs and tissues within the body of a mammal. In some respects, compositions containing an RNAi-inducing entity may be introduced by various routes to be transported within the body and taken up by cells in one or more organs or tissues, where expression of a target transcript is modulated.

In general, this disclosure encompasses RNAi-inducing agents that are useful therapeutics to prevent and treat diseases or disorders characterized by various aberrant processes. For instance, viruses that infect mammals can replicate by taking control of cellular machinery of the host cell. See, e.g., Fields Virology (2001). Thus, dsRNAs are useful to disrupt viral pathways which control virus production or replication.

This disclosure includes methods for treating or preventing a viral infection in a subject by use of one or more therapeutic RNAi-inducing agents having a broad spectrum of efficacy against strains of a target virus. An RNAi-inducing agent of this invention can be targeted to a sequence of a viral gene in a known variant strain or variants of a virus, and exhibit sequence-specific gene silencing of the targeted viral gene in those variants. For example, an RNAi-inducing agent may be targeted to, and exhibit efficacy against a seasonal strain of influenza virus, as well as variant strains of influenza.

Compositions and formulations of this disclosure may be used for delivery of drug agents or biologically active agents to a variety of cells *in vitro.* Examples of cells for which *in vitro* delivery is encompassed include epithelial cells such as A549, immortal cell lines such as HeLa, hepatoma cells such as HepG2, rat gliosarcoma cells such as 9L/LacZ, human monocyte cells such as THP-1, Madin-Darby canine kidney cells (MDCK), various fibroblast cell lines, and primary cells in culture in the presence or absence of various sera, among others.

Compositions and formulations of this disclosure may be used for delivery of drug agents or biologically active agents to a variety of cells, tissues or organs *in vivo.* Modalities for delivering an agent *in vivo* include topical, enteral, and parenteral routes. Examples of modalities for delivering an agent *in vivo* include inhalation of particles or droplets, delivery of nasal or nasal-pharngyl drops, particles, or suspensions, transdermal and transmucosal routes, as well as injection or infusion by intramuscular, subcutaneous, intravenous, intraarterial, intracardiac, intrathecal, intraosseus, intraperitoneal, and epidural routes.

In some embodiments, an agent can be administered *ex vivo* by direct exposure to cells, tissues or organs originating from a mammalian subject.

A drug agent or biologically active agent to be delivered using a composition or formulation of this disclosure may be found in any form including, for example, a pure form, a crystalline form, a solid form, a nanoparticle, a condensed form, a complexed form, or a conjugated form.

This invention also provides methods for the delivery of one or more RNAi-inducing entities to organs and tissues within the body of a mammal. In some embodiments, compositions containing an RNAi-inducing entity, one or more DILA2 amino acid compounds, and one or more lipid components are introduced by various routes to be transported within the body and taken up by cells in one or more organs or tissues, where expression of a target transcript is modulated.

This disclosure provides pharmaceutically acceptable nucleic acid compositions with various DILA2 amino acid compounds or lipids useful for therapeutic delivery of nucleic acids and gene-silencing RNAs. In particular, this invention provides compositions and methods for *in vitro* and *in vivo* delivery of dsRNAs for decreasing, downregulating, or silencing the translation of a target nucleic acid sequence or expression of a gene. These compositions and methods may be used for prevention and/or treatment of diseases in a mammal. In exemplary methods of this invention, a ribonucleic acid molecule such as an siRNA or shRNA is contacted with a DILA2 amino acid compound to formulate a composition which can be administered to cells or subjects such as mammals. In some embodiments, this invention provides methods for delivering an siRNA or shRNA intracellularly by contacting a nucleic acid-containing composition with a cell.

In exemplary embodiments, this invention includes compositions containing a nucleic acid molecule, such as a double-stranded RNA (dsRNA), a short interfering RNA (siRNA), or a short hairpin RNA (shRNA), admixed or complexed with a DILA2 amino acid compound, and a polymeric lipid to form a composition that enhances intracellular delivery of the nucleic acid molecule. In some embodiments, a delivery composition of this invention may contain a dsRNA and one, two, or more DILA2 amino acid compounds, which may be cationic or non-cationic. In some variations, a delivery composition may contain a dsRNA, DILA2 amino acid compounds, and one or more polymeric lipids. In some embodiments, a delivery composition may contain a dsRNA, one or more DILA2 amino acid compounds, one or more lipids, and one or more polymeric lipids. The compositions of this invention can form stable particles which may incorporate a dsRNA as an interfering RNA agent. Compositions and formulations of this invention may include further delivery-enhancing components or excipients.

In some embodiments, compositions of this invention contain stable RNA-containing particles having diameters from about 5 nm to about 400 nm. In some embodiments, the particles may have a uniform diameter of from about 10 nm to about 300 nm. In some embodiments, the particles may have a uniform diameter of from about 50 nm to about 150 nm.

Within exemplary compositions of this invention, a double-stranded RNA may be admixed or complexed with DILA2 amino acid compounds to form a composition that enhances intracellular delivery of the dsRNA as compared to contacting target cells with naked dsRNA.

In some embodiments, a composition of this invention may contain one or more DILA2 amino acid compounds which are from about 0.5% to about 70% (mol%) of the total amount of DILA2 amino acid compounds and lipids, if any, and delivery-enhancing components, including any polymeric component, but not including the RNA component. In some embodiments, a composition of this invention may contain one or more DILA2 amino acid compounds from about 10% to about 55%. In some embodiments, a composition of this invention may contain one or more DILA2 amino acid compounds from about 15% to about 35%.

In certain embodiments, a composition of this invention may contain one or more non-cationic lipids, where the non-cationic lipids are from about 2% to about 95% (mol%) of the total amount of DILA2 amino acid compounds and lipids, if any, and delivery-enhancing components, including any polymeric component, but not including the RNA component. In some embodiments, a composition of this invention may contain one or more non-cationic lipids from about 20% to about 75%, or from about 45% to about 75%, or from about 45% to about 55%. In some embodiments, a composition of this invention may contain one or more non-cationic lipids from about 10% to about 50%.

In some embodiments, a composition of this invention may contain one or more polymeric lipids, where the polymeric lipids are from about 0.2% to about 20% (mol%) of the total amount of DILA2 amino acid compounds and lipids, if any, and delivery-enhancing components, including any polymeric component, but not including the RNA component. In some embodiments, a composition of this invention may contain one or more polymeric lipids from about 0.5% to about 10%. In some embodiments, a composition of this invention may contain one or more polymeric lipids from about 1 % to about 5% of the composition.

### Compositions and uses for nucleic acid therapeutics

In some embodiments, this invention provides a method of treating a disease or disorder in a mammalian subject. A therapeutically effective amount of a composition of this invention containing an interfering RNA, a DILA2 amino acid compound, a non-cationic lipid, a polymeric lipid, and one or more delivery-enhancing components or excipients may be administered to a subject having a disease or disorder associated with expression or overexpression of a gene that can be reduced, decreased, downregulated, or silenced by the composition.

This invention encompasses methods for treating a disease of the lung such as respiratory distress, asthma, cystic fibrosis, pulmonary fibrosis, chronic obstructive pulmonary disease, bronchitis, or emphysema, by administering to the subject a therapeutically effective amount of a composition.

This invention encompasses methods for treating a disease including cancer, bladder cancer, liver cancer, liver disease, hypercholesterolemia, an inflammatory disease, a metabolic disease, inflammation, arthritis, rheumatoid arthritis, encephalitis, bone fracture, heart disease, viral disease, hepatitis, and influenza.

Methods for making liposomes are given in, for example, G. Gregoriadis, Liposome Technology (CRC Press 1984), and M. J. Ostro, Liposomes (Marcel Dekker 1987).

The nucleic acid component, DILA2 amino acid compounds, and other components may be mixed together first in a suitable medium such as a cell culture medium, after which one or more lipids or compounds may be added to the mixture. Alternatively, the DILA2 amino acid compounds can be mixed together first in a suitable medium such as a cell culture medium, after which the nucleic acid component can be added.

Within certain embodiments of the invention, a dsRNA is admixed with one or more DILA2 amino acid compounds, or a combination of one or more DILA2 amino acid compounds and non-cationic lipids.

The interfering RNA agent may also be complexed with, or conjugated to a DILA2 amino acid compound or polymeric lipid, and admixed with one or more non-cationic lipids, or a combination of one or more non-cationic and cationic lipids.

An interfering RNA agent and a DILA2 amino acid compound may be mixed together first, followed by the addition of one or more non-cationic lipids, or a combination of non-cationic and cationic lipids added in a suitable medium such as a cell culture medium. Alternatively, the DILA2 amino acid compounds and lipid components may be mixed first, followed by the addition of the RNA agent in a suitable medium.

In some embodiments, this disclosure includes micellar dispersion compositions containing a drug or active agent admixed or complexed with an DILA2 amino acid compounds and a dispersant to form a composition that provides intracellular delivery of the drug or active agent.

In certain embodiments, a dispersion composition of this disclosure may contain one or more drugs or active agents, one or more DILA2 amino acid compounds, and one or more dispersants. In some variations, a delivery composition may contain a drug or active agent, a dispersant, a DILA2 amino acid compound, and an optional polymeric lipid. The dispersion compositions of this disclosure can form stable particles which may incorporate the drug or active agent.

In some aspects, a dispersion composition of this disclosure may contain stable nucleic acid dispersion particles having diameters from about 5 nm to about 400 nm. In some embodiments, the particles may have a uniform diameter of from about 10 nm to about 300 nm. In some embodiments, the particles may have a uniform diameter of from about 50 nm to about 150 nm.

A micellar dispersion can be used to formulate and improve the bioavailability of a drug or active agent, including RNAi therapeutics. A micellar dispersion can provide dispersion droplets or nanoparticles having a hydrophobic oil-like core. The dispersion nanoparticles can be suspended in a continuous aqueous phase. A dispersion structure can avoid some disadvantages inherent in using a liposomal structure for delivery of active agents, and can provide advantages in delivery because of the lipophilic core.

This disclosure provides a range of micellar dispersion compositions containing DILA2 amino acid compounds or lipids and dispersants for drugs or medicaments, and for delivery and administration of RNA agents.

Examples of dispersants include synthetic compounds including polyoxyglycerides such as polyglycolated capryl glycerides, ethoxy diglycol, pegylated fatty glycerides, diethylene glycol monoethyl ethers, and mixtures thereof. Examples of dispersants include LABRAFIL, LABRASOL, ARLATONE, TRANSCUTOL, and mixtures thereof. Examples of dispersants include synthetic compounds such as alkylphospho-N-methylethanolamines and alkoylsarcosines. Examples of dispersants include FOS-MEA and CRODASINIC.

In some embodiments, a delivery composition of this disclosure may contain a drug or active agent, one or more oils, one or more DILA2 amino acid compounds, and emulsifier and stabilizer lipids. In some variations, a delivery composition may contain a drug or active agent, an oil, a lipid emulsifier, a DILA2 amino acid compound, a non-cationic lipid, and a polymeric lipid.

The compositions of this disclosure can form stable particles which may incorporate a drug or active agent. In some aspects, compositions of this disclosure contain stable drug or active agent emulsion particles having diameters from about 5 nm to about 400 nm. In some embodiments, the particles may have a uniform diameter of from about 10 nm to about 300 nm. In some embodiments, the particles may have a uniform diameter of from about 50 nm to about 150 nm.

In some embodiments, a drug or active agent may be admixed or complexed with an oil, an emulsifier, a DILA2 amino acid compound, and a polymeric stabilizing lipid, to form a composition that enhances intracellular delivery of the drug or active agent.

An oil-in-water emulsion can be used to formulate and improve the bioavailability of a drug or active agent, including RNAi therapeutics.

An oil-in-water emulsion can provide emulsion droplets or nanoparticles having a DILA2 amino acid compound or lipid layer surrounding a hydrophobic oil core. The emulsion droplets or nanoparticles can be suspended in a continuous aqueous phase. An emulsion structure can avoid some disadvantages inherent in using a liposomal structure for delivery of active agents, and can provide advantages in delivery because of the lipophilic core.

A range of novel emulsion compositions are provided in this disclosure including novel compositions and uses of oils, emulsifiers, DILA2 amino acid compounds and lipid components with interfering-RNA agents.

Examples of oils include synthetic oils, fatty acid esters of propylene glycols, ethers of ethylene glycols, glyceryl oils, cholesteryl oils, vegetable oils, nut oils, essential oils, mineral oil, lipid-soluble compounds such as tocopherols and Vitamin E, and mixtures thereof. Examples of oils include synthetic oils such as CAPRYOL 90

(propylene glycol monoester), CAPRYOL PGMC (propylene glycol monoester), LABRAFAC PC (propylene glycol monoester), LABRAFAC PG (propylene glycol diester), LAUROGLYCOL 90 (propylene glycol monoester), LAUROGLYCOL FCC (propylene glycol monoester), PLUROL OLEIQUE CC 497 (propylene glycol monoester), LABRAFAC LIPOPHILE WL 1349 (triglyceride), PECEOL (glyceryl monoester), MAISINE 35-1 (glyceryl monoester), and mixtures thereof.

### Compositions and methods for RNA therapeutics

This invention provides compositions and methods for modulating gene expression using regulatory RNA such as by RNA interference. A composition of this invention can deliver a ribonucleic acid agent to a cell which can produce the response of RNAi. Examples of nucleic acid agents useful for this invention include double-stranded nucleic acids, modified or degradation-resistant nucleic acids, RNA, siRNA, siRNA, shRNA, miRNA, piRNA, RNA antagonists, single-stranded nucleic acids, DNA-RNA chimeras, antisense nucleic acids, and ribozymes. As used herein, the terms siRNA, siRNA, and shRNA include precursors of siRNA, siRNA, and shRNA, respectively. For example, the term siRNA includes an RNA or double-stranded RNA that is suitable as a substrate of dicer enzyme.

Ribonucleic acid agents useful for this invention may be targeted to various genes. Examples of human genes suitable as targets include TNF, FLT1, the VEGF family, the ERBB family, the PDGFR family, BCR-ABL, and the MAPK family, among others. Examples of human genes suitable as targets and nucleic acid sequences thereto include those disclosed in PCT/US08/55333, PCT/US08/55339, PCT/US08/55340, PCT/US08/55341, PCT/US08/55350, PCT/US08/55353, PCT/US08/55356, PCT/US08/55357, PCT/US08/55360, PCT/US08/55362, PCT/US08/55365, PCT/US08/55366, PCT/US08/55369, PCT/US08/55370, PCT/US08/55371, PCT/US08/55372, PCT/US08/55373, PCT/US08/55374, PCT/US08/55375, PCT/US08/55376, PCT/US08/55377, PCT/US08/55378, PCT/US08/55380, PCT/US08/55381, PCT/US08/55382, PCT/US08/55383, PCT/LTS08/55385, PCT/US08/55386, PCT/US08/55505, PCT/US08/55511, PCT/US08/55515, PCT/US08/55516, PCT/US08/55519, PCT/US08/55524, PCT/US08/55526, PCT/US08/55527, PCT/US08/55532, PCT/US08/55533, PCT/US08/55542, PCT/US08/55548, PCT/US08/55550, PCT/US08/55551, PCT/US08/55554, PCT/US08/55556, PCT/US08/55560, PCT/US08/55563, PCT/US08/55597, PCT/US08/55599, PCT/LTS08/55601, PCT/US08/55603, PCT/US08/55604, PCT/US08/55606, PCT/US08/55608, PCT/US08/55611, PCT/US08/55612, PCT/US08/55615, PCT/US08/55618, PCT/US08/55622, PCT/US08/55625, PCT/US08/55627, PCT/US08/55631, PCT/US08/55635, PCT/US08/55644, PCT/US08/55649, PCT/US08/55651, PCT/US08/55662, PCT/US08/55672, PCT/US08/55676, PCT/US08/55678, PCT/US08/55695, PCT/US08/55697, PCT/US08/55698, PCT/US08/55701, PCT/US08/55704, PCT/US08/55708, PCT/US08/55709, and PCT/US08/55711.

An RNA of this disclosure to be delivered may have a sequence that is complementary to a region of a viral gene. For example, some compositions and methods of this invention are useful to regulate expression of the viral genome of an influenza virus. In some embodiments, this invention provides compositions and methods for modulating expression and infectious activity of an influenza by RNA interference. Expression and/or activity of an influenza can be modulated by delivering to a cell, for example, a short interfering RNA molecule having a sequence that is complementary to a region of a RNA polymerase subunit of an influenza. Examples of RNAs targeted to an influenza virus are given in U.S. Patent Publication No. 20070213293 A1.

In some embodiments, this invention provides compositions and methods for inhibiting expression of a target transcript in a subject by administering to the subject a composition containing an effective amount of an RNAi-inducing compound such as a short interfering oligonucleotide molecule, or a precursor thereof. RNAi uses small interfering RNAs (siRNAs) to target messenger RNA (mRNAs) and attenuate translation. A siRNA as used in this invention may be a precursor for dicer processing such as, for example, a long dsRNA processed into a siRNA. This invention provides methods of treating or preventing diseases or conditions associated with expression of a target transcript or activity of a peptide or protein encoded by the target transcript.

A therapeutic strategy based on RNAi can be used to treat a wide range of diseases by shutting down the growth or function of a virus or microorganism, as well as by shutting down the function of an endogenous gene product in the pathway of the disease.

In some embodiments, this invention provides novel compositions and methods for delivery of RNAi-inducing entities such as short interfering oligonucleotide molecules, and precursors thereof. In particular, this invention provides compositions containing an RNAi-inducing entity which is targeted to one or more transcripts of a cell, tissue, and/or organ of a subject.

A siRNA can be two RNA strands having a region of complementarity about 19 nucleotides in length. A siRNA optionally includes one or two single-stranded overhangs or loops,

A shRNA can be a single RNA strand having a region of self-complementarity. The single RNA strand may form a hairpin structure with a stem and loop and, optionally, one or more unpaired portions at the 5' and/or 3' portion of the RNA.

The active therapeutic agent can be a chemically-modified RNA with improved resistance to nuclease degradation in vivo, and/or improved cellular uptake, which retains RNAi activity.

A siRNA agent of this invention may have a sequence that is complementary to a region of a target gene. A siRNA of this invention may have 29-50 base pairs, for example, a dsRNA having a sequence that is complementary to a region of a target gene. Alternately, the double-stranded nucleic acid can be a dsDNA.

In certain embodiments, the active agent can be a short interfering nucleic acid (siRNA), short interfering RNA (siRNA), double-stranded RNA (dsRNA), micro-RNA, or short hairpin RNA (shRNA) that can modulate expression of a gene product.

Comparable methods and compositions are provided that target expression of one or more different genes associated with a particular disease condition in a subject, including any of a large number of genes whose expression is known to be aberrantly increased as a causal or contributing factor associated with the selected disease condition.

The RNAi-inducing compound of this invention can be administered in conjunction with other known treatments for a disease condition.

In some embodiments, this invention features compositions containing a small nucleic acid molecule, such as short interfering nucleic acid, a short interfering RNA, a double-stranded RNA, a micro-RNA, or a short hairpin RNA, admixed or complexed with, or conjugated to, a delivery-enhancing compound.

As used herein, the terms "regulatory RNA," "short interfering nucleic acid," "siRNA," "short interfering RNA," "short interfering oligonucleotide molecule," and "chemically-modified short interfering nucleic acid molecule," refer to any nucleic acid molecule capable of regulating, inhibiting or down regulating gene expression or, for example, viral replication, by mediating RNA interference (RNAi) or gene silencing in a sequence-specific manner. Regulatory RNA includes single-stranded RNA antagonists.

In some embodiments, the siRNA is a double-stranded polynucleotide molecule comprising self-complementary sense and antisense regions, wherein the antisense region comprises a nucleotide sequence that is complementary to a nucleotide sequence in a target ribonucleic acid molecule for down regulating expression, or a portion thereof, and the sense region comprises a nucleotide sequence corresponding to (*i.e.,* which is substantially identical in sequence to) the target ribonucleic acid sequence or portion thereof.

As used herein, "siRNA" means a small interfering ribonucleic acid that is a relatively short-length double-stranded nucleic acid, or optionally a longer precursor thereof. The length of useful siRNAs within this invention will in some embodiments be preferred at a length of approximately 20 to 50 bp. However, there is no particular limitation to the length of useful siRNAs, including siRNAs. For example, siRNAs can initially be presented to cells in a precursor form that is substantially different than a final or processed form of the siRNA that will exist and exert gene silencing activity upon delivery, or after delivery, to the target cell. Precursor forms of siRNAs may, for example, include precursor sequence elements that are processed, degraded, altered, or cleaved at or after the time of delivery to yield a siRNA that is active within the cell to mediate gene silencing. In some embodiments, useful siRNAs will have a precursor length, for example, of approximately 100-200 base pairs, or 50-100 base pairs, or less than about 50 base pairs, which will yield an active, processed siRNA within the target cell. In other embodiments, a useful siRNA or siRNA precursor will be approximately 10 to 49 bp, or 15 to 35 bp, or about 21 to 30 bp in length.

In certain embodiments of this invention, polynucleotide delivery-enhancing polypeptides may be used to facilitate delivery of nucleic acid molecules, including large nucleic acid precursors of siRNAs. For example, the methods and compositions herein may be employed for enhancing delivery of larger nucleic acids that represent "precursors" to desired siRNAs, wherein the precursor amino acids may be cleaved or otherwise processed before, during or after delivery to a target cell to form an active siRNA for modulating gene expression within the target cell.

For example, a dsRNA precursor polynucleotide may be selected as a circular, single-stranded polynucleotide, having two or more loop structures and a stem comprising self-complementary sense and antisense regions, wherein the antisense region comprises a nucleotide sequence that is complementary to a nucleotide sequence in a target nucleic acid molecule or a portion thereof, and the sense region having nucleotide sequence corresponding to the target nucleic acid sequence or a portion thereof, and wherein the circular polynucleotide can be processed either in vivo or in vitro to generate an active dsRNA molecule capable of inducing RNAi.

siRNA molecules of this invention, particularly non-precursor forms, can be less than 30 base pairs, or about 17-19 bp, or 19-21 bp, or 21-23 bp.

siRNAs can mediate selective gene silencing in the mammalian system. Hairpin RNAs, with a short loop and 19 to 27 base pairs in the stem, also selectively silence expression of genes that are homologous to the sequence in the double-stranded stem. Mammalian cells can convert short hairpin RNA into siRNA to mediate selective gene silencing.

RISC mediates cleavage of single stranded RNA having sequence complementary to the antisense strand of the siRNA duplex. Cleavage of the target RNA takes place within the region complementary to the antisense strand of the siRNA duplex. siRNA duplexes of 21 nucleotides are typically most active when containing two-nucleotide 3'-overhangs.

Replacing the 3'-overhanging segments of a 21-mer siRNA duplex having 2-nucleotide 3' overhangs with deoxyribonucleotides may not have an adverse effect on RNAi activity. Replacing up to 4 nucleotides on each end of the siRNA with deoxyribonucleotides can be tolerated.

Alternatively, siRNAs can be delivered as single or multiple transcription products expressed by a polynucleotide vector encoding the single or multiple siRNAs and directing their expression within target cells. In these embodiments the double-stranded portion of a final transcription product of the siRNAs to be expressed within the target cell can be, for example, 15 to 49 bp, 15 to 35 bp, or about 21 to 30 bp long.

In some embodiments of this invention, the double-stranded region of siRNAs in which two strands are paired may contain bulge or mismatched portions, or both. Double-stranded portions of siRNAs in which two strands are paired are not limited to completely paired nucleotide segments, and may contain nonpairing portions due to, for example, mismatch (the corresponding nucleotides not being complementary), bulge (lacking in the corresponding complementary nucleotide on one strand), or overhang. Nonpairing portions can be contained to the extent that they do not interfere with siRNA formation. In some embodiments, a "bulge" may comprise 1 to 2 nonpairing nucleotides, and the double-stranded region of siRNAs in which two strands pair up may contain from about 1 to 7, or about 1 to 5 bulges. In addition, "mismatch" portions contained in the double-stranded region of siRNAs may be present in numbers from about 1 to 7, or about 1 to 5. Most often in the case of mismatches, one of the nucleotides is guanine, and the other is uracil. Such mismatching may be attributable, for example, to a mutation from C to T, G to A, or mixtures thereof, in a corresponding DNA coding for sense RNA, but other causes are also contemplated.

The terminal structure of siRNAs of this invention may be either blunt or cohesive (overhanging) as long as the siRNA retains its activity to silence expression of target genes. The cohesive (overhanging) end structure is not limited to the 3' overhang, but includes the 5' overhanging structure as long as it retains activity for inducing gene silencing. In addition, the number of overhanging nucleotides is not limited to 2 or 3 nucleotides, but can be any number of nucleotides as long as it retains activity for inducing gene silencing. For example, overhangs may comprise from 1 to about 8 nucleotides, or from 2 to 4 nucleotides.

The length of siRNAs having overhang end structure may be expressed in terms of the paired duplex portion and any overhanging portion at each end. For example, a 25/27-mer siRNA duplex with a 2-bp 3' antisense overhang has a 25-mer sense strand and a 27-mer antisense strand, where the paired portion has a length of 25 bp.

Any overhang sequence may have low specificity to a target gene, and may not be complementary (antisense) or identical (sense) to the target gene sequence. As long as the siRNA retains activity for gene silencing, it may contain in the overhang portion a low molecular weight structure, for example, a natural RNA molecule such as a tRNA, an rRNA, a viral RNA, or an artificial RNA molecule.

The terminal structure of the siRNAs may have a stem-loop structure in which ends of one side of the double-stranded nucleic acid are connected by a linker nucleic acid, for example, a linker RNA. The length of the double-stranded region (stem portion) can be, for example, 15 to 49 bp, or 15 to 35 bp, or about 21 to 30 bp long. Alternatively, the length of the double-stranded region that is a final transcription product of siRNAs to be expressed in a target cell may be, for example, approximately 15 to 49 bp, or 15 to 35 bp, or about 21 to 30 bp long.

The siRNA can contain a single stranded polynucleotide having a nucleotide sequence complementary to a nucleotide sequence in a target nucleic acid molecule, or a portion thereof, wherein the single stranded polynucleotide can contain a terminal phosphate group, such as a 5'-phosphate (*see e.g.* Martinez, et al., Cell. 110:563-574, 2002, and Schwarz, et al., Molecular Cell 10:537-568, 2002, or 5',3'-diphosphate.

As used herein, the term siRNA is not limited to molecules containing only naturally-occurring RNA or DNA, but also encompasses chemically-modified nucleotides and non-nucleotides. In some embodiments, the short interfering nucleic acid molecules of the invention lack 2'-hydroxy (2'-OH) containing nucleotides. In some embodiments, short interfering nucleic acids do not require the presence of nucleotides having a 2'-hydroxy group for mediating RNAi and as such, short interfering nucleic acid molecules of this invention optionally do not include any ribonucleotides (e.g., nucleotides having a 2'-OH group). siRNA molecules that do not require the presence of ribonucleotides within the siRNA molecule to support RNAi can, however, have an attached linker or linkers or other attached or associated groups, moieties, or chains containing one or more nucleotides with 2'-OH groups. siRNA molecules can comprise ribonucleotides in at least about 5, 10, 20, 30, 40, or 50% of the nucleotide positions.

As used herein, the term siRNA encompasses nucleic acid molecules that are capable of mediating sequence specific RNAi such as, for example, short interfering RNA (siRNA) molecules, double-stranded RNA (dsRNA) molecules, micro-RNA molecules, short hairpin RNA (shRNA) molecules, short interfering oligonucleotide molecules, short interfering nucleic acid molecules, short interfering modified oligonucleotide molecules, chemically-modified siRNA molecules, and post-transcriptional gene silencing RNA (ptgsRNA) molecules, among others.

In some embodiments, siRNA molecules comprise separate sense and antisense sequences or regions, wherein the sense and antisense regions are covalently linked by nucleotide or non-nucleotide linker molecules, or are non-covalently linked by ionic interactions, hydrogen bonding, van der waals interactions, hydrophobic interactions, and/or stacking interactions.

"Antisense RNA" is an RNA strand having a sequence complementary to a target gene mRNA, that can induce RNAi by binding to the target gene mRNA.

"Sense RNA" is an RNA strand having a sequence complementary to an antisense RNA, and anneals to its complementary antisense RNA to form a siRNA.

As used herein, the term "RNAi construct" or "RNAi precursor" refers to an RNAi-inducing compound such as small interfering RNAs (siRNAs), hairpin RNAs, and other RNA species which can be cleaved *in vivo* to form a siRNA. RNAi precursors herein also include expression vectors (also referred to as RNAi expression vectors) capable of giving rise to transcripts which form dsRNAs or hairpin RNAs in cells, and/or transcripts which can produce siRNAs *in vivo*.

A siHybrid molecule is a double-stranded nucleic acid that has a similar function to siRNA. Instead of a double-stranded RNA molecule, a siHybrid is comprised of an RNA strand and a DNA strand. Preferably, the RNA strand is the antisense strand which binds to a target mRNA. The siHybrid created by the hybridization of the DNA and RNA strands have a hybridized complementary portion and preferably at least one 3'overhanging end.

siRNAs for use within the invention can be assembled from two separate oligonucleotides, where one strand is the sense strand and the other is the antisense strand, wherein the antisense and sense strands are self-complementary (*i.e.,* each strand comprises nucleotide sequence that is complementary to nucleotide sequence in the other strand; such as where the antisense strand and sense strand form a duplex or double stranded structure, for example wherein the double stranded region is about 19 base pairs), The antisense strand may comprise a nucleotide sequence that is complementary to a nucleotide sequence in a target nucleic acid molecule or a portion thereof, and the sense strand may comprise a nucleotide sequence corresponding to the target nucleic acid sequence or a portion thereof. Alternatively, the siRNA can be assembled from a single oligonucleotide, where the self-complementary sense and antisense regions of the siRNA are linked by means of a nucleic acid-based or non-nucleic acid-based linker(s),

In some embodiments, siRNAs for intracellular delivery can be a polynucleotide with a duplex, asymmetric duplex, hairpin or asymmetric hairpin secondary structure, having self-complementary sense and antisense regions, wherein the antisense region comprises a nucleotide sequence that is complementary to a nucleotide sequence in a separate target nucleic acid molecule or a portion thereof, and the sense region comprises a nucleotide sequence corresponding to the target nucleic acid sequence or a portion thereof.

Examples of chemical modifications that can be made in an siRNA include phosphorothioate internucleotide linkages, 2'-deoxyribonucleotides, 2'-O-methyl ribonucleotides, 2'-deoxy-2'-fluoro ribonucleotides, "universal base" nucleotides, "acyclic" nucleotides, 5-C-methyl nucleotides, and terminal glyceryl and/or inverted deoxy abasic residue incorporation.

The antisense region of a siRNA molecule can include a phosphorothioate internucleotide linkage at the 3'-end of said antisense region. The antisense region can comprise about one to about five phosphorothioate internucleotide linkages at the 5'-end of said antisense region. The 3'-terminal nucleotide overhangs of a siRNA molecule can include ribonucleotides or deoxyribonucleotides that are chemically-modified at a nucleic acid sugar, base, or backbone. The 3'-terminal nucleotide overhangs can include one or more universal base ribonucleotides. The 3'-terminal nucleotide overhangs can comprise one or more acyclic nucleotides.

For example, a chemically-modified siRNA can have 1, 2, 3, 4, 5, 6, 7, 8, or more phosphorothioate internucleotide linkages in one strand, or can have 1 to 8 or more phosphorothioate internucleotide linkages in each strand. The phosphorothioate internucleotide linkages can be present in one or both oligonucleotide strands of the siRNA duplex, for example in the sense strand, the antisense strand, or both strands.

siRNA molecules can comprise one or more phosphorothioate internucleotide linkages at the 3'-end, the 5'-end, or both of the 3'- and 5'-ends of the sense strand, the antisense strand, or in both strands. For example, an exemplary siRNA molecule can include 1, 2, 3, 4, 5, or more consecutive phosphorothioate internucleotide linkages at the 5'-end of the sense strand, the antisense strand, or both strands.

In certain embodiments, a siRNA molecule includes 1, 2, 3, 4, 5, 6, 7, 8, 9, 10, or more pyrimidine phosphorothioate internucleotide linkages in the sense strand, the antisense strand, or in both strands.

In some embodiments, a siRNA molecule includes 1, 2, 3, 4, 5, 6, 7, 8, 9, 10, or more purine phosphorothioate internucleotide linkages in the sense strand, the antisense strand, or in both strands.

A siRNA molecule can include a circular nucleic acid molecule, wherein the siRNA is about 38 to about 70, for example, about 38, 40, 45, 50, 55, 60, 65, or 70 nucleotides in length, having about 18 to about 23, for example, about 18, 19, 20, 21, 22, or 23 base pairs, wherein the circular oligonucleotide forms a dumbbell-shaped structure having about 19 base pairs and 2 loops.

A circular siRNA molecule can contain two loop motifs, wherein one or both loop portions of the siRNA molecule is biodegradable. For example, the loop portions of a circular siRNA molecule may be transformed *in vivo* to generate a double-stranded siRNA molecule with 3'-terminal overhangs, such as 3'-terminal nucleotide overhangs comprising about 2 nucleotides.

Modified nucleotides in a siRNA molecule can be in the antisense strand, the sense strand, or both. For example, modified nucleotides can have a Northern conformation *(e.g.,* Northern pseudorotation cycle; *see e.g.,* Saenger, Principles of Nucleic Acid Structure, Springer-Verlag ed., 1984). Examples of nucleotides having a Northern configuration include locked nucleic acid (LNA) nucleotides (*e.g.,* 2'-O, 4'-C-methylene-(D-ribofuranosyl) nucleotides), 2'-methoxyethoxy (MOE) nucleotides, 2'-methyl-thio-ethyl, 2'-deoxy-2'-fluoro nucleotides, 2'-deoxy-2'-chloro nucleotides, 2'-azido nucleotides, and 2'-O-methyl nucleotides.

Chemically modified nucleotides can be resistant to nuclease degradation while at the same time maintaining the capacity to mediate RNAi.

The sense strand of a double stranded siRNA molecule may have a terminal cap moiety such as an inverted deoxyabasic moiety, at the 3'-end, 5'-end, or both 3' and 5'-ends of the sense strand.

Examples of conjugates include conjugates and ligands described in Vargeese, et al., U.S. Application Serial No. 10/427,160, filed April 30, 2003, incorporated by reference herein in its entirety, including the drawings.

In some embodiments of this invention, the conjugate may be covalently attached to the chemically-modified siRNA molecule via a biodegradable linker. For example, the conjugate molecule may be attached at the 3'-end of either the sense strand, the antisense strand, or both strands of the chemically-modified siRNA molecule.

In certain embodiments, the conjugate molecule is attached at the 5'-end of either the sense strand, the antisense strand, or both strands of the chemically-modified siRNA molecule. In some embodiments, the conjugate molecule is attached both the 3'-end and 5'-end of either the sense strand, the antisense strand, or both strands of the chemically-modified siRNA molecule, or any combination thereof.

In some embodiments, a conjugate molecule comprises a molecule that facilitates delivery of a chemically-modified siRNA molecule into a biological system, such as a cell.

In some embodiments, a conjugate molecule attached to the chemically-modified siRNA molecule is a polyethylene glycol, human serum albumin, or a ligand for a cellular receptor that can mediate cellular uptake. Examples of specific conjugate molecules contemplated by the instant invention that can be attached to chemically-modified siRNA molecules are described in Vargeese, et al., U.S. Patent Publication Nos. 20030130186 and 20040110296.

A siRNA may be contain a nucleotide, non-nucleotide, or mixed nucleotide/non-nucleotide linker that joins the sense region of the siRNA to the antisense region of the siRNA. In some embodiments, a nucleotide linker can be 3, 4, 5, 6, 7, 8, 9, or 10 nucleotides in length. In some embodiments, the nucleotide linker can be a nucleic acid aptamer. As used herein, the terms "aptamer" or "nucleic acid aptamer" encompass a nucleic acid molecule that binds specifically to a target molecule, wherein the nucleic acid molecule contains a sequence that is recognized by the target molecule in its natural setting. Alternately, an aptamer can be a nucleic acid molecule that binds to a target molecule where the target molecule does not naturally bind to a nucleic acid.

For example, the aptamer can be used to bind to a ligand-binding domain of a protein, thereby preventing interaction of the naturally occurring ligand with the protein, *See,* for example, Gold, et al., Annu. Rev. Biochem. 64:763, 1995; Brody and Gold, J. Biotechnol. 74:5, 2000; Sun, Curr. Opin, Mol. Ther. 2:100,2000; Kusser, J. Biotechnol, 74:27, 2000; Hermann and Patel, Science 287:820, 2000; and Jayasena, Clinical Chemistry 45:1628, 1999.

A non-nucleotide linker can be an abasic nucleotide, polyether, polyamine, polyamide, peptide, carbohydrate, lipid, polyhydrocarbon, or other polymeric compounds (*e.g*., polyethylene glycols such as those having between 2 and 100 ethylene glycol units). Specific examples include those described by Seela and Kaiser, Nucleic Acids Res. 18:6353, 1990, and Nucleic Acids Res, 15:3113, 1987; Cload and Schepartz, J. Am. Chem. Soc. 113:6324, 1991; Richardson and Schepartz, J. Am. Chem. Soc. 113:5109, 1991; Ma, et al., Nucleic Acids Res. 21:2585, 1993, and Biochemistry 32:1751, 1993; Durand, et al., Nucleic Acids Res. 18:6353, 1990; McCurdy, et al., Nucleosides & Nucleotides 10:287, 1991; Jaschke, et al., Tetrahedron Lett. 34:301-304, 1993; Ono, et al., Biochemistry 30:9914, 1991; Arnold, et al., International Publication No. WO 89/02439; Usman, et al., International Publication No. WO 95/06731; Dudycz, et al., International Publication No. WO 95/11910, and Ferentz and Verdine, J. Am. Chem. Soc. 113:4000, 1991.

A "non-nucleotide linker" refers to a group or compound that can be incorporated into a nucleic acid chain in the place of one or more nucleotide units, including either sugar and/or phosphate substitutions, and allows the remaining bases to exhibit their enzymatic activity. The group or compound can be abasic in that it does not contain a commonly recognized nucleotide base, such as adenosine, guanine, cytosine, uracil or thymine, for example at the C1 position of the sugar.

In some embodiments, modified siRNA molecule can have phosphate backbone modifications including one or more phosphorothioate, phosphorodithioate, methylphosphonate, phosphotriester, morpholino, amidate carbamate, carboxymethyl, acetamidate, polyamide, sulfonate, sulfonamide, sulfamate, formacetal, thioformacetal, and/or alkylsilyl substitutions. Examples of oligonucleotide backbone modifications are given in Hunziker and Leumann, Nucleic Acid Analogues: Synthesis and Properties, in Modern Synthetic Methods, VCH, pp. 331-417, 1995, and Mesmaeker, et al., Novel Backbone Replacements for Oligonucleotides, in Carbohydrate Modifications in Antisense Research, ACS, pp. 24-39, 1994.

siRNA molecules, which can be chemically-modified, can be synthesized by: (a) synthesis of two complementary strands of the siRNA molecule; and (b) annealing the two complementary strands together under conditions suitable to obtain a double-stranded siRNA molecule. In some embodiments, synthesis of the complementary portions of the siRNA molecule is by solid phase oligonucleotide synthesis, or by solid phase tandem oligonucleotide synthesis.

Oligonucleotides (*e.g*., certain modified oligonucleotides or portions of oligonucleotides lacking ribonucleotides) are synthesized using protocols known in the art, for example, as described in Caruthers, et al., Methods in Enzymology 211:3-19, 1992; Thompson, et al., International PCT Publication No. WO 99/54459; Wincott, et al., Nucleic Acids Res. 23:2677-2684, 1995; Wincott, et al., Methods Mol. Bio. 74:59, 1997; Brennan, et al., Biotechnol Bioeng. 61:33-45, 1998; and Brennan, U.S. Patent No. 6,001,311. Synthesis of RNA, including certain siRNA molecules of the invention, follows general procedures as described, for example, in Usman, et al., J. Am. Chem. Soc. 109:7845, 1987; Scaringe, et al., Nucleic Acids Res. 18:5433, 1990; and Wincott, et al., Nucleic Acids Res. 23:2677-2684, 1995; Wincott, et al., Methods Mol. Bio. 74:59, 1997.

An "asymmetric hairpin" as used herein is a linear siRNA molecule comprising an antisense region, a loop portion that can comprise nucleotides or non-nucleotides, and a sense region that comprises fewer nucleotides than the antisense region to the extent that the sense region has enough complementary nucleotides to base pair with the antisense region and form a duplex with loop.

An "asymmetric duplex" as used herein is a siRNA molecule having two separate strands comprising a sense region and an antisense region, wherein the sense region comprises fewer nucleotides than the antisense region to the extent that the sense region has enough complementary nucleotides to base pair with the antisense region and form a duplex.

To "modulate gene expression" as used herein is to upregulate or downregulate expression of a target gene, which can include upregulation or downregulation of mRNA levels present in a cell, or of mRNA translation, or of synthesis of protein or protein subunits, encoded by the target gene.

The terms "inhibit," "down-regulate," or "reduce expression," as used herein mean that the expression of the gene, or level of RNA molecules or equivalent RNA molecules encoding one or more proteins or protein subunits, or level or activity of one or more proteins or protein subunits encoded by a target gene, is reduced below that observed in the absence of the nucleic acid molecules *(e.g.,* siRNA) of the invention.

"Gene silencing" as used herein refers to partial or complete inhibition of gene expression in a cell and may also be referred to as "gene knockdown." The extent of gene silencing may be determined by methods known in the art, some of which are summarized in International Publication No. WO 99/32619.

As used herein, the terms "ribonucleic acid" and "RNA" refer to a molecule containing at least one ribonucleotide residue. A ribonucleotide is a nucleotide with a hydroxyl group at the 2' position of a beta-D-ribo-furanose moiety. These terms include double-stranded RNA, single-stranded RNA, isolated RNA such as partially purified RNA, essentially pure RNA, synthetic RNA, recombinantly produced RNA, as well as modified and altered RNA that differs from naturally occurring RNA by the addition, deletion, substitution, modification, and/or alteration of one or more nucleotides. Alterations of an RNA can include addition of non-nucleotide material, such as to the end(s) of a siRNA or internally, for example at one or more nucleotides of an RNA.

Nucleotides in an RNA molecule include non-standard nucleotides, such as non-naturally occurring nucleotides or chemically synthesized nucleotides or deoxynucleotides. These altered RNAs can be referred to as analogs.

By "highly conserved sequence region" is meant, a nucleotide sequence of one or more regions in a target gene does not vary significantly from one generation to the other or from one biological system to the other.

By "sense region" is meant a nucleotide sequence of a siRNA molecule having complementarity to an antisense region of the siRNA molecule. In addition, the sense region of a siRNA molecule can comprise a nucleic acid sequence having homology with a target nucleic acid sequence.

By "antisense region" is meant a nucleotide sequence of a siRNA molecule having complementarity to a target nucleic acid sequence. In addition, the antisense region of a siRNA molecule can include a nucleic acid sequence having complementarity to a sense region of the siRNA molecule.

By "target nucleic acid" is meant any nucleic acid sequence whose expression or activity is to be modulated. A target nucleic acid can be DNA or RNA.

By "complementarity" is meant that a nucleic acid can form hydrogen bond(s) with another nucleic acid sequence either by traditional Watson-Crick or by other non-traditional modes of binding.

The term "biodegradable linker" as used herein, refers to a nucleic acid or non-nucleic acid linker molecule that is designed as a biodegradable linker to connect one molecule to another molecule, for example, a biologically active molecule to a siRNA molecule or the sense and antisense strands of a siRNA molecule. The biodegradable linker is designed such that its stability can be modulated for a particular purpose, such as delivery to a particular tissue or cell type. The stability of a nucleic acid-based biodegradable linker molecule can be variously modulated, for example, by combinations of ribonucleotides, deoxyribonucleotides, and chemically-modified nucleotides, such as 2'-O-methyl, 2'-fluoro, 2'-amino, 2'-O-amino, 2'-C-allyl, 2'-O-allyl, and other 2'-modified or base modified nucleotides. The biodegradable nucleic acid linker molecule can be a dimer, trimer, tetramer or longer nucleic acid molecule, for example, an oligonucleotide of about 2, 3, 4, 5, 6, 7, 8, 9, 10, 11, 12, 13, 14, 15, 16, 17, 18, 19, or 20 nucleotides in length, or can comprise a single nucleotide with a phosphorus-based linkage, for example, a phosphoramidate or phosphodiester linkage. The biodegradable nucleic acid linker molecule can also comprise nucleic acid backbone, nucleic acid sugar, or nucleic acid base modifications.

In connection with 2'-modified nucleotides as described herein, by "amino" is meant 2'-NH₂ or 2'-O-NH₂, which can be modified or unmodified. Such modified groups are described, for example, in Eckstein, et al., U.S. Patent No. 5,672,695 and Matulic-Adamic, et al., U.S. Patent. No. 6,248,878.

Supplemental or complementary methods for delivery of nucleic acid molecules for use within then invention are described, for example, in Akhtar et al., Trends Cell Bio. 2:139, 1992; "Delivery Strategies for Antisense Oligonucleotide Therapeutics," ed. Akhtar, 1995, Maurer et al., Mol. Membr. Biol. 16:129-140, 1999; Hofland and Huang, Handb. Exp. Pharmacol. 137:165-192, 1999; and Lee et al., ACS Symp. Ser. 752:184-192, 2000. Sullivan, et al., International PCT Publication No. WO 94/02595, further describes general methods for delivery of enzymatic nucleic acid molecules.

Nucleic acid molecules can be administered within formulations that include one or more components, such as a pharmaceutically acceptable carrier, diluent, excipient, adjuvant, emulsifier, buffer, stabilizer, or preservative.

As used herein, the term "carrier" means a pharmaceutically acceptable solid or liquid diluent, solvent, filler, or encapsulating material. Examples of carriers include saline, biological and pharmaceutical buffer systems, and biologically acceptable media. A water-containing liquid carrier can contain pharmaceutically acceptable additives such as acidifying agents, alkalizing agents, antimicrobial preservatives, antioxidants, buffering agents, chelating agents, complexing agents, solubilizing agents, humectants, solvents, suspending and/or viscosity-increasing agents, tonicity agents, wetting agents or other biocompatible materials. Examples of ingredients of the above categories can be found in the U.S. Pharmacopeia National Formulary, 1990, pp. 1857-1859, as well as in Raymond C. Rowe, et al., Handbook of Pharmaceutical Excipients, 5th ed., 2006, and "Remington: The Science and Practice of Pharmacy," 21st ed., 2006, editor David B. Troy.

Examples of preservatives include phenol, methyl paraben, paraben, m-cresol, thiomersal, benzylalkonium chloride, and mixtures thereof.

Examples of surfactants include oleic acid, sorbitan trioleate, polysorbates, lecithin, phosphotidylcholines, various long chain diglycerides and phospholipids, and mixtures thereof.

Examples of phospholipids include phosphatidylcholine, lecithin, phosphatidylglycerol, phosphatidylinositol, phosphatidylserine, and phosphatidylethanolamine, and mixtures thereof.

Examples of dispersants include ethylenediaminetetraacetic acid.

Examples of gases include nitrogen, helium, chlorofluorocarbons (CFCs), hydrofluorocarbons (HFCs), carbon dioxide, air, and mixtures thereof.

In certain embodiments, the siRNA and/or the polypeptide can be encapsulated in liposomes, or reside either internal or external to a liposome, or exist within liposome layers, or be administered by iontophoresis, or incorporated into other vehicles, such as hydrogels, cyclodextrins, biodegradable nanocapsules, bioadhesive microspheres, or proteinaceous vectors. *See,* for example, O'Hare and Normand, PCT International Publication No. WO 00/53722. Alternatively, a nucleic acid composition can be locally delivered by direct injection or by use of an infusion pump. Direct injection of the nucleic acid molecules of the invention, whether subcutaneous, intramuscular, or intradermal, can take place using standard needle and syringe methodologies, or by needle-free technologies such as those described in Conry et al., Clin. Cancer Res. 5:2330-2337, 1999, and Barry et al., International PCT Publication No. WO 99/31262.

The compositions of this invention can be effectively employed as pharmaceutical agents. Pharmaceutical agents prevent, modulate the occurrence or severity of, or treat (alleviate one or more symptom(s) to a detectable or measurable extent) of a disease state or other adverse condition in a patient.

In some embodiments, this invention provides pharmaceutical compositions and methods featuring the presence or administration of one or more polynucleic acid(s), typically one or more siRNAs, combined, complexed, or conjugated with a DILA2 amino acid compound or lipid, which may further be formulated with a pharmaceutically-acceptable carrier, such as a diluent, stabilizer, or buffer.

Typically, the siRNA will target a gene that is expressed at an elevated level as a causal or contributing factor associated with the subject disease state or adverse condition. In this context, the siRNA will effectively downregulate expression of the gene to levels that prevent, alleviate, or reduce the severity or recurrence of one or more associated disease symptoms. Alternatively, for various distinct disease models where expression of the target gene is not necessarily elevated as a consequence or sequel of disease or other adverse condition, down regulation of the target gene will nonetheless result in a therapeutic result by lowering gene expression *(i.e.,* to reduce levels of a selected mRNA and/or protein product of the target gene). Alternatively, siRNAs of the invention may be targeted to lower expression of one gene, which can result in upregulation of a "downstream" gene whose expression is negatively regulated by a product or activity of the target gene.

This siRNAs of this disclosure may be administered in any form, for example transdermally or by local injection (*e.g*., local injection at sites of psoriatic plaques to treat psoriasis, or into the joints of patients afflicted with psoriatic arthritis or RA). In more detailed embodiments, the invention provides formulations and methods to administer therapeutically effective amounts of siRNAs directed against of a mRNA of TNF-α, which effectively down-regulate the TNF-α RNA and thereby reduce or prevent one or more TNF-α-associated inflammatory condition(s). Comparable methods and compositions are provided that target expression of one or more different genes associated with a selected disease condition in animal subjects, including any of a large number of genes whose expression is known to be aberrantly increased as a causal or contributing factor associated with the selected disease condition.

The compositions of the present invention may also be formulated and used as tablets, capsules or elixirs for oral administration, suppositories for rectal administration, sterile solutions, suspensions for injectable administration, and the other forms known in the art.

A pharmacological composition or formulation refers to a composition or formulation in a form suitable for administration, for example, systemic administration, into a cell or patient, including for example a human. Suitable forms, in part, depend upon the use or the route of entry, for example oral, transdermal, transepithelial, or by injection. Such forms should not prevent the composition or formulation from reaching a target cell (*i.e.,* a cell to which the negatively charged nucleic acid is desirable for delivery). For example, pharmacological compositions injected into the blood stream should be soluble. Other factors are known in the art, and include considerations such as toxicity.

By "systemic administration" is meant *in vivo* systemic absorption or accumulation of drugs in the blood stream followed by distribution throughout the entire body. Administration routes which lead to systemic absorption include, without limitation: intravenous, subcutaneous, intraperitoneal, inhalation, oral, intrapulmonary and intramuscular.

Examples of agents suitable for formulation with the nucleic acid molecules of this invention include: P-glycoprotein inhibitors (such as Pluronic P85), which can enhance entry of drugs into the CNS (Jolliet-Riant and Tillement, Fundam. Clin. Pharmacol. 13:16-26, 1999); biodegradable polymers, such as poly (DL-lactide-coglycolide) microspheres for sustained release delivery after intracerebral implantation (Emerich, D.F., et al., Cell Transplant 8:47-58, 1999, Alkermes, Inc., Cambridge, Mass.); and loaded nanoparticles, such as those made of polybutylcyanoacrylate, which can deliver drugs across the blood brain barrier and can alter neuronal uptake mechanisms (Prog. Neuropsychopharmacol Biol. Psychiatry 23:941-949, 1999). Other examples of delivery strategies for the nucleic acid molecules of the instant invention include material described in Boado, et al., J. Pharm. Sci. 87:1308-1315, 1998; Tyler, et al., FEBS Lett. 421:280-284, 1999; Pardridge, et al., PNAS USA. 92:5592-5596, 1995; Boado, Adv. Drug Delivery Rev. 15:73-107, 1995; Aldrian-Herrada et al., Nucleic Acids Res. 26:4910-4916, 1998; and Tyler, et al., PNAS USA. 96:7053-7058, 1999.

The present invention also includes compositions prepared for storage or administration, which include a pharmaceutically effective amount of the desired compounds in a pharmaceutically acceptable carrier or diluent. Acceptable carriers or diluents for therapeutic use are well known in the pharmaceutical art, and are described, for example, in Remington's Pharmaceutical Sciences, Mack Publishing Co. (A.R. Gennaro ed. 1985). For example, preservatives, stabilizers, dyes and flavoring agents may be provided. These include sodium benzoate, sorbic acid and esters of p-hydroxybenzoic acid. In addition, antioxidants and suspending agents may be used.

A pharmaceutically effective dose is that dose required to prevent, inhibit the occurrence of, treat, or alleviate a symptom to some extent of a disease state. An amount of from 0.01 mg/kg to 50 mg/kg body weight/day of active nucleic acid should be administered.

Aqueous suspensions contain the active materials in admixture with excipients suitable for the manufacture of aqueous suspensions. Such excipients are suspending agents, for example sodium carboxymethylcellulose, methylcellulose, hydropropyl-methylcellulose, sodium alginate, polyvinylpyrrolidone, gum tragacanth and gum acacia; dispersing or wetting agents can be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions can also contain one or more preservatives, for example ethyl, or n-propyl p-hydroxybenzoate, one or more coloring agents, one or more flavoring agents, and one or more sweetening agents, such as sucrose or saccharin.

Oily suspensions can be formulated by suspending the active ingredients in a vegetable oil, for example arachis oil, olive oil, sesame oil or coconut oil, or in a mineral oil such as liquid paraffin. The oily suspensions can contain a thickening agent, for example beeswax, hard paraffin or cetyl alcohol. Sweetening agents and flavoring agents can be added to provide palatable oral preparations. These compositions can be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Other excipients, for example sweetening, flavoring and coloring agents, can also be present.

Pharmaceutical compositions of the invention can also be in the form of oil-in-water emulsions. The oily phase can be a vegetable oil or a mineral oil or mixtures of these. Suitable emulsifying agents can be naturally-occurring gums, for example gum acacia or gum tragacanth, naturally-occurring phosphatides, for example soy bean, lecithin, and esters or partial esters derived from fatty acids and hexitol, anhydrides, for example sorbitan monooleate, and condensation products of the said partial esters with ethylene oxide, for example polyoxyethylene sorbitan monooleate. The emulsions can also contain sweetening and flavoring agents.

The pharmaceutical compositions can be in the form of a sterile injectable aqueous or oleaginous suspension. This suspension can be formulated according to the known art using those suitable dispersing or wetting agents and suspending agents that have been mentioned above. The sterile injectable preparation can also be a sterile injectable solution or suspension in a non-toxic parentally acceptable diluent or solvent, for example as a solution in 1,3-butanediol. Among the acceptable vehicles and solvents that can be employed are water, Ringer's solution and isotonic sodium chloride solution. In addition, sterile, fixed oils may be employed as a solvent or suspending medium. For this purpose, any bland fixed oil can be employed including synthetic mono-or diglycerides. In addition, fatty acids such as oleic acid find use in the preparation of injectables,

The siRNAs can also be administered in the form of suppositories, for example, for rectal administration of the drug. These compositions can be prepared by mixing the drug with a suitable non-irritating excipient that is solid at ordinary temperatures but liquid at the rectal temperature and will therefore melt in the rectum to release the drug. Such materials include cocoa butter and polyethylene glycols.

The siRNAs can be modified extensively to enhance stability by modification with nuclease resistant groups, for example, 2'-amino, 2'-C-allyl, 2'-fluoro, 2'-O-methyl, 2'-H. For a review *see* Usman and Cedergren, TIBS 17:34, 1992; Usman, et al., Nucleic Acids Symp. Ser. 31:163, 1994. siRNA constructs can be purified by gel electrophoresis using general methods or can be purified by high pressure liquid chromatography and resuspended in water.

Chemically synthesizing nucleic acid molecules with modifications (base, sugar and/or phosphate) can prevent their degradation by serum ribonucleases, which can increase their potency. *See* for example, Eckstein, et al., International Publication No. WO 92/07065; Perrault et al., Nature 344:565, 1990; Pieken, et al., Science 253, 314, 1991; Usman and Cedergren, Trends in Biochem. Sci. 17:334, 1992; Usman, et al., International Publication No. WO 93/15187; and Rossi et al., International Publication No. WO 91/03162; Sproat, U.S. Patent No. 5,334,711; and Gold, et al., U.S. Patent No. 6,300,074. All of the above references describe various chemical modifications that can be made to the base, phosphate and/or sugar moieties of the nucleic acid molecules described herein.

There are several examples in the art describing sugar, base and phosphate modifications that can be introduced into nucleic acid molecules with significant enhancement in their nuclease stability and efficacy. For example, oligonucleotides are modified to enhance stability and/or enhance biological activity by modification with nuclease resistant groups, for example, 2'-amino, 2'-C-allyl, 2'-fluoro, 2'-O-methyl, 2'-O-allyl, 2'-H, nucleotide base modifications. For a review, *see* Usman and Cedergren, TIBS 17:34, 1992; Usman, et al., Nucleic Acids Symp. Ser. 31:163, 1994; Burgin, et al., Biochemistry 35:14090, 1996. Sugar modification of nucleic acid molecules have been extensively described in the art. *See* Eckstein et al., International Publication PCT No. WO 92/07065; Perrault, et al. Nature 344:565-568, 1990; Pieken, et al. Science 253:314-317, 1991; Usman and Cedergren, Trends in Biochem. Sci. 17:334-339, 1992; Usman et al. International Publication PCT No. WO 93/15187; Sproat, U.S. Patent No. 5,334,711 and Beigelman, et al., J. Biol. Chem. 270:25702, 1995; Beigelman, et al., International PCT Publication No. WO 97/26270; Beigelman, et al., U.S. Patent No. 5,716,824; Usman, et al., U.S. Patent No. 5,627,053; Woolf, et al., International PCT Publication No. WO 98/13526; Thompson, *et al.,* Karpeisky, et al., Tetrahedron Lett. 39:1131, 1998; Earnshaw and Gait, Biopolymers (Nucleic Acid Sciences) 48:39-55, 1998; Verma and Eckstein, Annu. Rev. Biochem. 67:99-134, 1998; and Burlina, et al., Bioorg. Med. Chem. 5:1999-2010, 1997. Such publications describe general methods and strategies to determine the location of incorporation of sugar, base and/or phosphate modifications and the like into nucleic acid molecules without modulating catalysis. In view of such teachings, similar modifications can be used as described herein to modify the siRNA nucleic acid molecules of the instant invention so long as the ability of siRNA to promote RNAi in cells is not significantly inhibited.

While chemical modification of oligonucleotide internucleotide linkages with phosphorothioate, phosphorodithioate, and/or 5'-methylphosphonate linkages improves stability, excessive modifications can cause some toxicity or decreased activity. Therefore, when designing nucleic acid molecules, the amount of these internucleotide linkages should be minimized. The reduction in the concentration of these linkages should lower toxicity, resulting in increased efficacy and higher specificity of these molecules.

In some embodiments, the invention features modified siRNA molecules, with phosphate backbone modifications comprising one or more phosphorothioate, phosphorodithioate, methylphosphonate, phosphotriester, morpholino, amidate carbamate, carboxymethyl, acetamidate, polyamide, sulfonate, sulfonamide, sulfamate, formacetal, thioformacetal, and/or alkylsilyl, substitutions. For a review of oligonucleotide backbone modifications, *see* Hunziker and Leumann, Nucleic Acid Analogues: Synthesis and Properties, in Modern Synthetic Methods, VCH, 1995, pp. 331-417, and Mesmaeker, et al., "Novel Backbone Replacements for Oligonucleotides, in Carbohydrate Modifications in Antisense Research," ACS, 1994, pp. 24-39.

Methods for the delivery of nucleic acid molecules are described in Akhtar, et al., Trends Cell Bio. 2:139, 1992; "Delivery Strategies for Antisense Oligonucleotide Therapeutics," ed. Akhtar, 1995; Maurer, et al., Mol. Membr. Biol. 16:129-140, 1999; Hofland and Huang, Handb. Exp. Pharmacol. 137:165-192, 1999; and Lee, et al., ACS Symp. Ser. 752:184-192, 2000. Beigelman, et al., U.S. Patent No. 6,395,713, and Sullivan et al., PCT WO 94/02595 further describe the general methods for delivery of nucleic acid molecules. These protocols can be utilized for the delivery of virtually any nucleic acid molecule. Nucleic acid molecules can be administered to cells by a variety of methods known to those of skill in the art, including, but not restricted to, encapsulation internally or externally by liposomes, by iontophoresis, or by incorporation into other vehicles, such as biodegradable polymers, hydrogels, cyclodextrins (*see e.g.* Gonzalez, et al., Bioconjugate Chem. 10:1068-1074, 1999; Wang, et al., International PCT Publication Nos. WO 03/47518 and WO 03/46185), poly(lactic-co-glycolic)ac- id (PLGA) and PLCA microspheres (*see e.g.* U.S. Patent No. 6,447,796 and U.S. Patent Application Publication No. US 2002130430), biodegradable nanocapsules, and bioadhesive microspheres, or by proteinaceous vectors (O'Hare and Normand, International PCT Publication No. WO 00/53722). Alternatively, the nucleic acid/vehicle combination is locally delivered by direct injection or by use of an infusion pump. Direct injection of the nucleic acid molecules of the invention, whether subcutaneous, intramuscular, or intradermal, can take place using standard needle and syringe methodologies, or by needle-free technologies such as those described in Conry, et al., Clin. Cancer Res. 5:2330-2337, 1999, and Barry, et al., International PCT Publication No. WO 99/31262. The molecules of the instant invention can be used as pharmaceutical agents. Pharmaceutical agents prevent, modulate the occurrence, or treat (alleviate a symptom to some extent, preferably all of the symptoms) of a disease state in a subject.

By "RNA" is meant a molecule comprising at least one ribonucleotide residue. By "ribonucleotide" is meant a nucleotide with a hydroxyl group at the 2' position of a .beta.-D-ribo-furanose moiety. The terms include double-stranded RNA, single-stranded RNA, isolated RNA such as partially purified RNA, essentially pure RNA, synthetic RNA, recombinantly produced RNA, as well as altered RNA that differs from naturally occurring RNA by the addition, deletion, substitution and/or alteration of one or more nucleotides. Such alterations can include addition of non-nucleotide material, such as to the end(s) of the siRNA or internally, for example, at one or more nucleotides of the RNA. Nucleotides in the RNA molecules of the instant invention can also comprise non-standard nucleotides, such as non-naturally occurring nucleotides or chemically synthesized nucleotides or deoxynucleotides. These altered RNAs can be referred to as analogs or analogs of naturally-occurring RNA.

By "cap structure" is meant chemical modifications, which have been incorporated at either terminus of the oligonucleotide (*see, e.g.* Adamic, et al., U.S. Patent No. 5,998,203, incorporated by reference herein). These terminal modifications protect the nucleic acid molecule from exonuclease degradation, and may help in delivery and/or localization within a cell. The cap may be present at the 5'-terminus (5'-cap) or at the 3'-terminal (3'-cap) or may be present on both termini. In non-limiting examples, the 5'-cap includes, but is not limited to, glyceryl, inverted deoxy abasic residue (moiety); 4',5'-methylene nucleotide; 1-(beta-D-erythrofuranosyl) nucleotide, 4'-thio nucleotide; carbocyclic nucleotide; 1,5-anhydrohexitol nucleotide; L-nucleotides; alpha-nucleotides; modified base nucleotide; phosphorodithioate linkage; threo-pentofuranosyl nucleotide; acyclic 3',4'-seco nucleotide; acyclic 3,4-dihydroxybutyl nucleotide; acyclic 3,5-dihydroxypentyl nucleotide, 3'-3'-inverted nucleotide moiety; 3'-3'-inverted abasic moiety; 3'-2'-inverted nucleotide moiety; 3'-2'-inverted abasic moiety; 1,4-butanediol phosphate; 3'-phosphoramidate; hexylphosphate; aminohexyl phosphate; 3'-phosphate; 3'-phosphorothioate; phosphorodithioate; or bridging or non-bridging methylphosphonate moiety.

Examples of the 3'-cap include, but are not limited to, glyceryl, inverted deoxy abasic residue (moiety), 4',5'-methylene nucleotide; 1-(beta-D-erythrofuranosyl) nucleotide; 4'-thio nucleotide, carbocyclic nucleotide; 5'-amino-alkyl phosphate; 1,3-diamino-2-propyl phosphate; 3-aminopropyl phosphate; 6-aminohexyl phosphate; 1,2-aminododecyl phosphate; hydroxypropyl phosphate; 1,5-anhydrohexitol nucleotide; L-nucleotide; alpha-nucleotide; modified base nucleotide; phosphorodithioate; threo-pentofuranosyl nucleotide; acyclic 3',4'-seco nucleotide; 3,4-dihydroxybutyl nucleotide; 3,5-dihydroxypentyl nucleotide, 5'-5'-inverted nucleotide moiety; 5'-5'-inverted abasic moiety; 5'-phosphoramidate; 5'-phosphorothioate; 1,4-butanediol phosphate; 5'-amino; bridging and/or non-bridging 5'-phosphoramidate, phosphorothioate and/or phosphorodithioate, bridging or non bridging methylphosphonate and 5'-mercapto moieties (for more details *see* Beaucage and Lyer, Tetrahedron 49:1925, 1993; incorporated by reference herein).

By the term "non-nucleotide" is meant any group or compound which can be incorporated into a nucleic acid chain in the place of one or more nucleotide units, including either sugar and/or phosphate substitutions, and allows the remaining bases to exhibit their enzymatic activity. The group or compound is abasic in that it does not contain a commonly recognized nucleotide base, such as adenosine, guanine, cytosine, uracil or thymine and therefore lacks a base at the 1'-position.

By "nucleotide" as used herein is as recognized in the art to include natural bases (standard), and modified bases well known in the art. Such bases are generally located at the 1' position of a nucleotide sugar moiety. Nucleotides generally comprise a base, sugar and a phosphate group. The nucleotides can be unmodified or modified at the sugar, phosphate and/or base moiety, (also referred to interchangeably as nucleotide analogs, modified nucleotides, non-natural nucleotides, non-standard nucleotides and other; *see, e.g.* Usman and McSwiggen, *supra*; Eckstein, et al., International PCT Publication No. WO 92/07065; Usman, et al, International PCT Publication No. WO 93/15187; Uhlman & Peyman, *supra,* all are hereby incorporated by reference herein). There are several examples of modified nucleic acid bases known in the art as summarized by Limbach, et al., Nucleic Acids Res. 22:2183, 1994. Some of the non-limiting examples of base modifications that can be introduced into nucleic acid molecules include, inosine, purine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidines (*e.g*., 5-methylcytidine), 5-alkyluridines (*e.g.,* ribothymidine), 5-halouridine (*e.g., 5-*bromouridine) or 6-azapyrimidines or 6-alkylpyrimidines (*e.g*. 6-methyluridine), propyne, and others (Burgin, et al., Biochemistry 35:14090, 1996; Uhlman & Peyman, *supra*). By "modified bases" in this aspect is meant nucleotide bases other than adenine, guanine, cytosine and uracil at 1' position or their equivalents.

By "target site" or "target sequence" or "targeted sequence" is meant a sequence within a target nucleic acid *(e.g.,* RNA) that is "targeted" for cleavage mediated by a siRNA construct which contains sequences within its antisense region that are complementary to the target sequence.

The siRNA molecules can be complexed with DILA2 amino acid compounds or cationic lipids, packaged within liposomes, or otherwise delivered to target cells or tissues. The nucleic acid or nucleic acid complexes can be locally administered to through injection, infusion pump or stent, with or without their incorporation in biopolymers. In another embodiment, polyethylene glycol (PEG) can be covalently attached to siRNA compounds of the present invention, to the polypeptide, or both. The attached PEG can be any molecular weight, preferably from about 2,000 to about 50,000 daltons (Da).

The sense region can be connected to the antisense region via a linker molecule, such as a polynucleotide linker or a non-nucleotide linker.

"Inverted repeat" refers to a nucleic acid sequence comprising a sense and an antisense element positioned so that they are able to form a double stranded siRNA when the repeat is transcribed. The inverted repeat may optionally include a linker or a heterologous sequence such as a self-cleaving ribozyme between the two elements of the repeat. The elements of the inverted repeat have a length sufficient to form a double stranded RNA. Typically, each element of the inverted repeat is about 15 to about 100 nucleotides in length, preferably about 20-30 base nucleotides, preferably about 20-25 nucleotides in length, *e.g.,* 20, 21, 22, 23, 24, 25, 26, 27, 28, 29, or 30 nucleotides in length.

"Nucleic acid" refers to deoxyribonucleotides or ribonucleotides and polymers thereof in single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2'-O-methyl ribonucleotides, peptide-nucleic acids (PNAs).

"Large double-stranded RNA" refers to any double-stranded RNA having a size greater than about 40 bp for example, larger than 100 bp or more particularly larger than 300 bp. The sequence of a large dsRNA may represent a segment of a mRNA or the entire mRNA. The maximum size of the large dsRNA is not limited herein. The double-stranded RNA may include modified bases where the modification may be to the phosphate sugar backbone or to the nucleoside. Such modifications may include a nitrogen or sulfur heteroatom or any other modification known in the art.

The double-stranded structure may be formed by self-complementary RNA strand such as occurs for a hairpin or a micro RNA or by annealing of two distinct complementary RNA strands.

"Overlapping" refers to when two RNA fragments have sequences which overlap by a plurality of nucleotides on one strand, for example, where the plurality of nucleotides (nt) numbers as few as 2-5 nucleotides or by 5-10 nucleotides or more.

"One or more dsRNAs" refers to dsRNAs that differ from each other on the basis of primary sequence.

"Target gene or mRNA" refers to any gene or mRNA of interest. Target genes or mRNA may include developmental genes and regulatory genes as well as metabolic or structural genes or genes encoding enzymes. The target gene may be endogenous or exogenous. The target gene may be expressed in those cells in which a phenotype is being investigated or in an organism in a manner that directly or indirectly impacts a phenotypic characteristic. Such cells include any cell in the body of an adult or embryonic animal or plant including gamete or any isolated cell such as occurs in an immortal cell line or primary cell culture.

### Uses for delivery of active agents

The compounds and compositions of this invention may be used for delivery of any physiologically or biologically active agent, as well as any combination of active agents, as described above or known in the art. The active agent may be present in the compositions and uses of this invention in an amount sufficient to provide the desired physiological or ameliorative effect.

The compounds and compositions of this invention are directed toward enhancing delivery of a range of drug agents and biologically active agents in mammalian subjects including small molecule compounds and drugs, peptides, proteins, antibodies, monoclonal antibodies, antibody-based drugs, and vaccine agents.

Examples of active agents include a peptide, a protein, a nucleic acid, a double-stranded RNA, a hematopoietic, an antiinfective; an antidementia; an antiviral, an antitumoral, an antipyretic, an analgesic, an anti-inflammatory, an antiulcerative, an antiallergenic, an antidepressant, a psychotropic, a cardiotonic, an antiarrythmic, a vasodilator, an antihypertensive, a hypotensive diuretic, an antidiabetic, an anticoagulant, a cholesterol-lowering agent, a therapeutic for osteoporosis, a hormone, an antibiotic, a vaccine, a cytokine, a hormone, a growth factor, a cardiovascular factor, a cell adhesion factor, a central or peripheral nervous system factor, a humoral electrolyte factor, a hemal organic substance, a bone growth factor, a gastrointestinal factor, a kidney factor, a connective tissue factor, a sense organ factor, an immune system factor, a respiratory system factor, a genital organ factor, an androgen, an estrogen, a prostaglandin, a somatotropin, a gonadotropin, an interleukin, a steroid, a bacterial toxoid, an antibody, a monoclonal antibody, a polyclonal antibody, a humanized antibody, an antibody fragment, and an immunoglobin.

Examples of active agents include erythropoietin, granulocyte-colony stimulating factor, insulin, Factor VII, Factor VIII, Factor IX, interferon, heparin, hirugen, hirulos, and hirudine.

Examples of active agents include morphine, hydromorphone, oxymorphone, lovorphanol, levallorphan, codeine, nalmefene, nalorphine, nalozone, naltrexone, buprenorphine, butorphanol, or nalbufine, cortisone, hydrocortisone, fludrocortisone, prednisone, prednisolone, methylprednisolone, triamcinolone, dexamethoasone, betamethoasone, paramethosone, fluocinolone, colchicine, acetaminophen, a non-steroidal anti-inflammatory agent NSAID, acyclovir, ribavarin, trifluorothyridine, Ara-A Arabinofuranosyladenine, acylguanosine, nordeoxyguanosine, azidothymidine, dideoxyadenosine, dideoxycytidine, spironolactone, testosterone, estradiol, progestin, gonadotrophin, estrogen, progesterone, papaverine, nitroglycerin, a vasoactive intestinal peptide, calcitonin gene-related peptide, cyproheptadine, doxepin, imipramine, cimetidine, dextromethorphan, clozaril, superoxide dismutase, neuroenkephalinase, amphotericin B, griseofulvin, miconazole, ketoconazole, tioconazol, itraconazole, fluconazole, cephalosporin, tetracycline, aminoglucoside, erythromicin, gentamicin, polymyxin B, 5-fluorouracil, bleomycin, methotrexate, hydroxyurea, dideoxyinosine, floxuridine, 6-mercaptopurine, doxorubicin, daunorubicin, I-darubicin, taxol, paclitaxel, tocopherol, quinidine, prazosin, verapamil, nifedipine, diltiazem, tissue plasminogen activator TPA, epidermal growth factor EGF, fibroblast growth factor FGF-acidic or basic, platelet derived growth factor PDGF, transforming growth factor TGF-alpha or beta, vasoactive intestinal peptide, tumor necrosis factor TNF, hypothalmic releasing factor, prolactin, thyroid stimulating hormone TSH, adrenocorticotropic hormone ACTH, parathyroid hormone PTH, follicle stimulating hormone FSF, luteinizing hormone releasing hormone LHRH, endorphin, glucagon, calcitonin, oxytocin, carbetocin, aldoetecone, enkaphalin, somatostin, somatotropin, somatomedin, alpha-melanocyte stimulating hormone, lidocaine, sufentainil, terbutaline, droperidol, scopolamine, gonadorelin, ciclopirox, buspirone, cromolyn sodium, midazolam, cyclosporin, lisinopril, captopril, delapril, ranitidine, famotidine, superoxide dismutase, asparaginase, arginase, arginine deaminease, adenosine deaminase ribonuclease, trypsin, chemotrypsin, papain, bombesin, substance P, vasopressin, alpha-globulins, transferrin, fibrinogen, beta-lipoprotein, beta-globulin, prothrombin, ceruloplasmin, alpha2-glycoprotein, alpha2-globulin, fetuin, alpha1-lipoprotein, alpha1-globulin, albumin, and prealbumin.

Examples of active agents include opioids or opioid antagonists, such as morphine, hydromorphone, oxymorphone, lovorphanol, levallorphan, codeine, nalmefene, nalorphine, nalozone, naltrexone, buprenorphine, butorphanol, and nalbufine; corticosterones, such as cortisone, hydrocortisone, fludrocortisone, prednisone, prednisolone, methylprednisolone, triamcinolone, dexamethoasone, betamethoasone, paramethosone, and fluocinolone; other anti-inflammatories, such as colchicine, ibuprofen, indomethacin, and piroxicam; anti-viral agents such as acyclovir, ribavarin, trifluorothyridine, Ara-A (Arabinofuranosyladenine), acylguanosine, nordeoxyguanosine, azidothymidine, dideoxyadenosine, and dideoxycytidine; antiandrogens such as spironolactone; androgens, such as testosterone; estrogens, such as estradiol; progestins; muscle relaxants, such as papaverine; vasodilators, such as nitroglycerin, vasoactive intestinal peptide and calcitonin related gene peptide; antihistamines, such as cyproheptadine; agents with histamine receptor site blocking activity, such as doxepin, imipramine, and cimetidine; antitussives, such as dextromethorphan; neuroleptics such as clozaril; antiarrhythmics; antiepileptics; enzymes, such as superoxide dismutase and neuroenkephalinase; anti-fungal agents, such as amphotericin B, griseofulvin, miconazole, ketoconazole, tioconazol, itraconazole, and fluconazole; antibacterials, such as penicillins, cephalosporins, tetracyclines, aminoglucosides, erythromicin, gentamicins, polymyxin B; anti-cancer agents, such as 5-fluorouracil, bleomycin, methotrexate, and hydroxyurea, dideoxyinosine, floxuridine, 6-mercaptopurine, doxorubicin, daunorubicin, I-darubicin, taxol, and paclitaxel; antioxidants, such as tocopherols, retinoids, carotenoids, ubiquinones, metal chelators, and phytic acid; antiarrhythmic agents, such as quinidine; antihypertensive agents such as prazosin, verapamil, nifedipine, and diltiazem; analgesics such as acetaminophen and aspirin; monoclonal and polyclonal antibodies, including humanized antibodies, and antibody fragments; anti-sense oligonucleotides; and RNA, regulatory RNA, interfering RNA, DNA, and viral vectors comprising genes encoding therapeutic peptides and proteins.

### Compositions and Formulations for Administration

As used herein, the terms "administering" and "administration" encompass all means for directly and indirectly delivering a compound or composition to a site of action. The compounds and compositions of this disclosure may be administered alone, or in combination with other compounds, compositions, or therapeutic agents which are not disclosed herein.

The compositions and methods of the invention may be administered to subjects by a variety of mucosal administration modes, including by oral, rectal, vaginal, intranasal, intrapulmonary, or transdermal delivery, or by topical delivery to the eyes, ears, skin or other mucosal surfaces. In some aspects of this invention, the mucosal tissue layer includes an epithelial cell layer. The epithelial cell can be pulmonary, tracheal, bronchial, alveolar, nasal, buccal, epidermal, or gastrointestinal. Compositions of this invention can be administered using actuators such as mechanical spray devices, as well as pressurized, electrically activated, or other types of actuators.

Compositions of this invention may be administered in an aqueous solution as a nasal or pulmonary spray and may be dispensed in spray form by a variety of methods known to those skilled in the art. Pulmonary delivery of a composition of this invention may be achieved by administering the composition in the form of drops, particles, or spray, which can be, for example, aerosolized, atomized, or nebulized. Pulmonary delivery may be performed by administering the composition in the form of drops, particles, or spray, via the nasal or bronchial passages. Particles of the composition, spray, or aerosol can be in a either liquid or solid form. Preferred systems for dispensing liquids as a nasal spray are disclosed in U.S. Patent No. 4,511,069. Such formulations may be conveniently prepared by dissolving compositions according to the present invention in water to produce an aqueous solution, and rendering said solution sterile. The formulations may be presented in multi-dose containers, for example in the sealed dispensing system disclosed in U.S. Patent No. 4,511,069. Other suitable nasal spray delivery systems have been described in Transdermal Systemic Medication, Y.W. Chien ed., Elsevier Publishers, New York, 1985; and in U.S. Patent No. 4,778,810. Additional aerosol delivery forms may include, for example, compressed air-, jet-, ultrasonic-, and piezoelectric nebulizers, which deliver the biologically active agent dissolved or suspended in a pharmaceutical solvent, for example, water, ethanol, or mixtures thereof.

Nasal and pulmonary spray solutions of the present invention typically comprise the drug or drug to be delivered, optionally formulated with a surface active agent, such as a nonionic surfactant (*e.g*., polysorbate-80), and one or more buffers. In some embodiments of the present invention, the nasal spray solution further comprises a propellant. The pH of the nasal spray solution may be from about pH 6.8 to 7.2. The pharmaceutical solvents employed can also be a slightly acidic aqueous buffer of pH 4-6. Other components may be added to enhance or maintain chemical stability, including preservatives, surfactants, dispersants, or gases.

In some embodiments, this invention is a pharmaceutical product which includes a solution containing a composition of this invention and an actuator for a pulmonary, mucosal, or intranasal spray or aerosol.

A dosage form of the composition of this invention can be liquid, in the form of droplets or an emulsion, or in the form of an aerosol.

A dosage form of the composition of this invention can be solid, which can be reconstituted in a liquid prior to administration. The solid can be administered as a powder. The solid can be in the form of a capsule, tablet or gel.

To formulate compositions for pulmonary delivery within the present invention, the biologically active agent can be combined with various pharmaceutically acceptable additives or delivery-enhancing components, as well as a base or carrier for dispersion of the active agent(s). Examples of additives or delivery-enhancing components include pH control agents such as arginine, sodium hydroxide, glycine, hydrochloric acid, citric acid, and mixtures thereof. Other additives or delivery-enhancing components include local anesthetics (*e.g*., benzyl alcohol), isotonizing agents *(e.g.,* sodium chloride, mannitol, sorbitol), adsorption inhibitors *(e.g.,* Tween 80), solubility enhancing agents (*e.g.,* cyclodextrins and derivatives thereof), stabilizers (*e.g*., serum albumin), and reducing agents (*e.g*., glutathione). When the composition for mucosal delivery is a liquid, the tonicity of the formulation, as measured with reference to the tonicity of 0.9% (w/v) physiological saline solution taken as unity, is typically adjusted to a value at which no substantial, irreversible tissue damage will be induced in the mucosa at the site of administration. Generally, the tonicity of the solution is adjusted to a value of about 1/3 to 3, more typically 1/2 to 2, and most often 3/4 to 1.7.

The biologically active agent may be dispersed in a base or vehicle, which may comprise a hydrophilic compound having a capacity to disperse the active agent and any desired additives. The base may be selected from a wide range of suitable carriers, including but not limited to, copolymers of polycarboxylic acids or salts thereof, carboxylic anhydrides (*e.g.,* maleic anhydride) with other monomers (*e.g.,* methyl (meth)acrylate, acrylic acid, *etc*.), hydrophilic vinyl polymers such as polyvinyl acetate, polyvinyl alcohol, polyvinylpyrrolidone, cellulose derivatives such as hydroxymethylcellulose, hydroxypropylcellulose, *etc.,* and natural polymers such as chitosan, collagen, sodium alginate, gelatin, hyaluronic acid, and nontoxic metal salts thereof. A biodegradable polymer may be selected as a base or carrier, for example, polylactic acid, poly(lactic acid-glycolic acid) copolymer, polyhydroxybutyric acid, poly(hydroxybutyric acid-glycolic acid) copolymer and mixtures thereof. Synthetic fatty acid esters such as polyglycerin fatty acid esters, sucrose fatty acid esters, *etc*., can be employed as carriers. Hydrophilic polymers and other carriers can be used alone or in combination, and enhanced structural integrity can be imparted to the carrier by partial crystallization, ionic bonding, crosslinking and the like. The carrier can be provided in a variety of forms, including, fluid or viscous solutions, gels, pastes, powders, microspheres and films for direct application to the nasal mucosa. The use of a selected carrier in this context may result in promotion of absorption of the biologically active agent.

The biologically active agent can be combined with the base or carrier according to a variety of methods, and release of the active agent may be by diffusion, disintegration of the carrier, or associated formulation of water channels. In some circumstances, the active agent is dispersed in microcapsules (microspheres) or nanocapsules (nanospheres) prepared from a suitable polymer, *e*.*g*., isobutyl 2-cyanoacrylate (*see, e.g.,* Michael, et al., J. Pharmacy Pharmacol. 43:1-5, 1991), and dispersed in a biocompatible dispersing medium applied to the nasal mucosa, which yields sustained delivery and biological activity over a protracted time.

Formulations for mucosal, nasal, or pulmonary delivery may contain a hydrophilic low molecular weight compound as a base or excipient. Such hydrophilic low molecular weight compounds provide a passage medium through which a water-soluble active agent, such as a physiologically active peptide or protein, may diffuse through the base to the body surface where the active agent is absorbed. The hydrophilic low molecular weight compound optionally absorbs moisture from the mucosa or the administration atmosphere and dissolves the water-soluble active peptide. The molecular weight of the hydrophilic low molecular weight compound is generally not more than 10,000 and preferably not more than 3000. Examples of hydrophilic low molecular weight compounds include polyol compounds, such as oligo-, di- and monosaccarides including sucrose, mannitol, lactose, L-arabinose, D-erythrose, D-ribose, D-xylose, D-mannose, D-galactose, lactulose, cellobiose, gentibiose, glycerin, polyethylene glycol, and mixtures thereof. Further examples of hydrophilic low molecular weight compounds include N-methylpyrrolidone, alcohols (*e.g*., oligovinyl alcohol, ethanol, ethylene glycol, propylene glycol, *etc.*)*,* and mixtures thereof.

The compositions of this invention may alternatively contain as pharmaceutically acceptable carriers substances as required to approximate physiological conditions, such as pH adjusting and buffering agents, tonicity adjusting agents, and wetting agents, for example, sodium acetate, sodium lactate, sodium chloride, potassium chloride, calcium chloride, sorbitan monolaurate, triethanolamine oleate, and mixtures thereof. For solid compositions, nontoxic pharmaceutically acceptable carriers can be used which include, for example, pharmaceutical grades of mannitol, lactose, starch, magnesium stearate, sodium saccharin, talcum, cellulose, glucose, sucrose, magnesium carbonate, and the like.

In certain embodiments of the invention, the biologically active agent may be administered in a time release formulation, for example in a composition which includes a slow release polymer. The active agent can be prepared with carriers that will protect against rapid release, for example a controlled release vehicle such as a polymer, microencapsulated delivery system or bioadhesive gel. Prolonged delivery of the active agent, in various compositions of the invention can be brought about by including in the composition agents that delay absorption, for example, aluminum monosterate hydrogels and gelatin.

Within certain embodiments of this invention, a composition may contain one or more natural or synthetic surfactants. Certain natural surfactants are found in human lung (pulmonary surfactant), and are a complex mixture of phospholipids and proteins that form a monolayer at the alveolar air-liquid interface and reduces surface tension to near zero at expiration and prevents alveolar collapse. Over 90% (by weight) of pulmonary surfactant is composed of phospholipids with approximately 40-80% being DPPC and the remainder being unsaturated phosphatidylcholines POPG, POPC and phosphatidylglycerols. The remaining 10% (by weight) of surfactant is composed of plasma proteins and apoproteins, such as surface proteins (SP)-A, SP-B, SP-C and SP-D.

Examples of natural surfactants that may be used in this invention include SURVANTA™ (beractant), CUROSURF™ (poractant alfa) and INFASURF™ (calfactant), and mixtures thereof.

Examples of synthetic surfactants include sinapultide; a combination of dipalmitoylphosphatidylcholine, palmitoyloleoyl phosphatidylglycerol and palmitic acid; SURFAXIN™ (lucinactant); and EXOSURF™ (colfosceril); components which may contain tyloxapol, DPPC, and hexadecanol; and mixtures thereof.

Methods of making delivery compositions include ethanol injection methods and extrusion methods using a Northern Lipids Lipex Extruder system with stacked polycarbonate membrane filters of defined pore size. Sonication using probe tip and bath sonicators can be employed to produce particles of uniform size. Homogenous and monodisperse particle sizes can be obtained without the addition of the nucleic acid component. For *in vitro* transfection compositions, the nucleic acid component can be added after the transfection agent is made and stabilized by buffer components. For *in vivo* delivery compositions, the nucleic acid component is part of the formulation.

The compositions and formulations of this invention may be administered by various routes, for example, to effect systemic delivery via intravenous, parenteral, or intraperitoneal routes. In some embodiments, an agent may be delivered intracellularly, for example, in cells of a target tissue such as lung or liver, or in inflamed tissues. Included within this disclosure are compositions and methods for delivery of an agent by removing cells of a subject, delivering an agent to the removed cells, and reintroducing the cells into a subject. In some embodiments, this invention provides a method for delivery of an agent *in vivo.* A composition may be administered intravenously, subcutaneously, or intraperitoneally to a subject. In some embodiments, the invention provides methods for *in vivo* delivery of an agent to the lung of a mammalian subject.

The active agent liposomal compositions of this disclosure may be used in pharmaceutical compositions *in vivo*. Administration of the active agent liposomal compositions of this disclosure to a subject may be parenteral, oral, by inhalation, topical, mucosal, rectal, or buccal routes. Parenteral use includes subcutaneous, intracutaneous, intravenous, intramuscular, intraarticular, intrasynovial, intrastemal, intrathecal, intralesional, and intracranial injection or infusion techniques.

An effective amount of an active agent liposomal composition of this disclosure for treating a particular disease is generally an amount sufficient to ameliorate or reduce a symptom of the disease. The composition may be administered as a single dosage, or may be administered by repeated dosing.

### Additional Embodiments

All publications, references, patents, patent publications and patent applications cited herein are each hereby specifically incorporated by reference in their entirety.

While this invention has been described in relation to certain embodiments, and many details have been set forth for purposes of illustration, it will be apparent to those skilled in the art that this invention includes additional embodiments, and that some of the details described herein may be varied considerably without departing from this invention. This invention includes such additional embodiments, modifications and equivalents. In particular, this invention includes any combination of the features, terms, or elements of the various illustrative components and examples.

The use herein of the terms "a," "an," "the" and similar terms in describing the invention, and in the claims, are to be construed to include both the singular and the plural.

The terms "comprising," "having," "including" and "containing" are to be construed as open-ended terms which mean, for example, "including, but not limited to." Thus, terms such as "comprising," "having," "including" and "containing" are to be construed as being inclusive, not exclusive.

Recitation of a range of values herein refers individually to each and any separate value falling within the range as if it were individually recited herein, whether or not some of the values within the range are expressly recited. For example, the range "4 to 12" includes without limitation the values 5, 5.1, 5.35 and any other whole, integer, fractional, or rational value greater than or equal to 4 and less than or equal to 12. Specific values employed herein will be understood as exemplary and not to limit the scope of the invention.

Recitation of a range of number of carbon atoms herein refers individually to each and any separate value falling within the range as if it were individually recited herein, whether or not some of the values within the range are expressly recited. For example, the term "C1-22" includes without limitation the species C1, C2, C3, C4, C5, C6, C7, C8, C9, C10, C11, C12, C13, C14, C15, C16, C17, C18, C19, C20, C21, and C22.

Definitions of technical terms provided herein should be construed to include without recitation those meanings associated with these terms known to those skilled in the art, and are not intended to limit the scope of the invention. Definitions of technical terms provided herein shall be construed to dominate over alternative definitions in the art or definitions which become incorporated herein by reference to the extent that the alternative definitions conflict with the definition provided herein.

The examples given herein, and the exemplary language used herein are solely for the purpose of illustration, and are not intended to limit the scope of the invention.

When a list of examples is given, such as a list of compounds or molecules suitable for this invention, it will be apparent to those skilled in the art that mixtures of the listed compounds or molecules are also suitable.

### EXAMPLES

### EXAMPLE 1

### Methods for preparing an RNA-containing liposomal formulation

This example describes embodiments of methods for making an RNA-containing liposomal formulation. Some materials used in the method are summarized below:
C18:1-norArg-C16 (Palmitoyl Oleyl nor-Arginine, PONA) (MDRNA, Inc.) (formula weight 683.3)
1,2-Dimyristoyl-sn-Glycero-3-Phosphoethanolamine-N-[Methoxy(Polyethylene glycol)-2000] (Ammonium Salt) (DMPE-PEG2k) (Genzyme Pharmaceuticals, Cambridge, Mass.)
Cholesterol (Solvay Pharmaceuticals)
Cholesteryl-hemisuccinate (CHEMS) GMP (Merck Eprova AG)
Ethanol (absolute, 200 proof); Sterile water for injection
Sodium phosphate: monobasic, anhydrous, dibasic, anhydrous
Sucrose, 99+%
5 N sodium hydroxide; 2 N hydrochloric acid; Glacial acetic acid
Tromethamine (Tris) USP Grade (Research Organics)
150 mL Capacity 0.2 µm filter bottles, PES
Calibrated Rainin 20 µL, 200 µL, and 1 mL pipettors
Iso-disc filter PTFE25-10
Cole-Parmer In-line static mixer
Watson Marlow 520 Di pump; Watson Marlow 523 pump; Filtertec pump
Vivaflow 50 100,00 MWCO PES (Sartorius)
Slide-a-Lyser dialysis cassette 10,000 MWCO (Pierce)

The buffer solution Sucrose Phosphate (SUP) Formulation Buffer (20 mM sodium phosphate, 215 mM sucrose, pH 7.4) was prepared as follows. 2.17 g anhydrous monobasic sodium phosphate and 8.79 g anhydrous dibasic sodium phosphate were added to 3600 mL of Milli-Q DI water in a graduated cylinder and mixed thoroughly with a stir bar. The pH was adjusted with 5N sodium hydroxide or 2N hydrogen chloride to pH 7.4. 294.38 g sucrose was added slowly and dissolved thoroughly. Final water volume was adjusted to 4 L. The solution was filtered with a 0.2 µm filter.

A 25 mM stock solution of liposome-forming molecules in 90% v/v ethanol USP was prepared as follows. 90 mL of ethanol USP (200 proof) was dispensed into a clean autoclaved 100 mL Pyrex bottle. To the ethanol were added successively 1291 umol of C18:1-norArg-C16 (PONA), 721.6 umol of cholesteryl-hemisuccinate (CHEMS) powder, 61.7 umol of DMPE-PEG2K powder, and 515 umol of cholesterol. The ingredients were each added to the solution and mixed thoroughly with a stir bar. The mixture was sonicated for 15 minutes. 10 mL of sterile water for injection USP was added with thorough mixing. The stock solution was filtered through an ISO-DISC filter PTFE-25 mm, 1 um pore size. The stock solution was stored at 80 °C and analyzed for DILA2 amino acid compounds and lipid components by Reverse Phase HPLC with Evaporative Light Scattering Detection.

An siRNA stock solution was prepared in sterile water for injection as follows. 5 mL of sterile water for injection was dispensed into a sterile 15 mL Falcon tube. 100 mg of siRNA powder was added to the tube and vortexed thoroughly. The solution was filtered through a 0.22 uM Millex GP filter unit using a 10 mL syringe. The siRNA solution was stored at -20°C and tested by OD (A260 and A280) for purity and concentration with 1:1000 dilution.

A Watson Marlow 520Di peristaltic pump was calibrated to a flow rate of 40 mL/min. The pump was set to 210 rpm and disconnected from the tubing. 40 mL of 90% ethanol was pumped through to rinse the line. Ethanol was pumped into a beaker for 15 sec and weighed to determine the flow rate in mL/min. The pump speed was adjusted to provide a flow rate of 40 ± 0.5 mL/min. Pumps for siRNA and sucrose phosphate solutions were calibrated in a similar manner.

Three solutions were used to prepare an siRNA formulation as follows. (a) The first solution for pumping was an siRNA solution. The first solution was made by diluting the siRNA with SUP buffer in a 50 mL conical tube and vortexing thoroughly. (b) The second solution for pumping was a solution of a DILA2 amino acid compound plus three lipids. A mixed lipid stock in 90% ethanol was prepared containing the following lipids: CHEMS, cholesterol, and DMPE-PEG. To the lipid stock was added a DILA2 amino acid compound. To the lipid stock was added an aliquot of Tris in sterile water for injection to make a 1:1 molar Tris:CHEMS concentration in the solution. The second solution for pumping was made with the mixed lipid stock by pipetting with a positive displacement pipette into a 50 mL conical tube, diluting with 90% ethanol, and vortexing thoroughly. (c) A third solution for pumping was an SUP buffer solution.

An siRNA formulation was prepared as follows. The first siRNA solution and the second solution of liposome-forming molecules were simultaneously pumped into an impinging stream. The first 1 mL of the effluent impinging stream was discarded, then the siRNA formulation was collected in a vessel. A Watson Marlow 323 pump was used to pump SUP buffer solution into the vessel to adjust the concentration of ethanol to be about 33%. The siRNA formulation in the vessel was incubated with gentle agitation on magnetic stir plate for 1 hr.

After incubation, the formulation was loaded into a Pierce slide-a-lyzer dialysis cassette with 10,000 MWCO, and dialyzed for 12-18 hrs at 4 °C against 100 volumes of SUP.

This example further describes embodiments of methods for making an RNA-containing liposomal formulation by tangential flow and diafiltration. A siRNA formulation was provided as described above, except that the last dialysis step was replaced by a tangential flow filtration (TFF) process.

The siRNA formulation was diluted to 10% (v/v) final ethanol concentration under gentle agitation on magnetic stir plate for 2 min.

A TFF system using a Sartorius Vivaflow 50 100,000 MWCO PES membrane was rinsed with 50 mL of 70% ethanol USP, and then re-circulated with 100 mL of 70% ethanol at a pump flow rate of 60 mL/min. The TFF system was rinsed with 50 mL of sterile water and then re-circulated with 100 mL of sterile water at a pump flow rate of 60 mL/min. The TFF system was rinsed with 50 mL of SUP and then re-circulated with 100 mL of SUP at a pump flow rate of 60 mL/min.

The diluted siRNA formulation was loaded into the TFF vessel and concentrated by 5 times to a final siRNA concentration of 0.5 mg/mL (feed pressure ∼20 psi, retentate pressure <0.2 psi and a permeate flow rate of ∼2 mL/min). A maximum of 1 mg of siRNA formulated in the liposomal composition was processed per cm² of membrane.

The concentrated siRNA formulation was filtered by diafiltration against 5 volumes of SUP, in which ethanol was removed, at flow rate 2mL/min.

The concentrated siRNA formulation was further concentrated to the desired volume, at 1 mg/ml siRNA.

This example further describes embodiments of methods for making an RNA-containing liposomal formulation by sterile filtration of the siRNA liposomal formulation. A siRNA formulation was provided as described above. 10 mL of the siRNA formulation was drawn up in a 10 mL polypropylene syringe, and air bubbles were removed. The siRNA formulation was filtered through a 0.22 uM Millex GP filter unit. 10 mg of siRNA formulation (1 mg siRNA/mL) was filtered though the Millex GP filter unit with moderate pressure on the syringe, 1 mL aliquots of this drug product were stored in 3 mL type I sterile glass vials at 80 °C prior to use.

### EXAMPLE 2

### siRNA Liposomal Formulation

An example of a liposomal siRNA formulation embodiment of this disclosure is shown in Table 6.

**Table 6: Liposomal siRNA Formulation**

| Component | µM | MW | mg/ml | mg/kg dosed |
|---|---|---|---|---|
| dsRNA | 7.5 | 13255.4 | 0.100 | 1.0 |
| DMPE-PEG2K | 38.5 | 2815 | 0.108 | 1.1 |
| chol | 366.2 | 386 | 0.141 | 1.4 |
| CHEMS | 506.8 | 486 | 0.246 | 2.5 |
| PONA | 929.3 | 683 | 0.635 | 6.3 |

### EXAMPLE 3

### Effects of physical process parameters on RNA-containing liposomal compositions

In this example, the effects of certain process parameters for collection, incubation and quenching on the properties of siRNA liposomal compositions were observed. Compositions were prepared by using the basic protocol described in Example 1.

In each example, the active agent of the composition was a dsRNA for silencing ApoB. The liposome-forming component was an ethanol-water solution containing the DILA2 amino acid compound C18:1-norArg(NH₃Cl)-C16, along with the lipids cholesteryl hemisuccinate (CHEMS, Anatrace, CH210), cholesterol (Anatrace CH200), and DMPE-PEG2k (Genzyme).

In a first example, the effects of the concentration of organic solvent at the collection step on liposome particle size and dispersity were observed as shown in Table 7. The concentration of organic solvent ethanol was calculated from flow rates and transfer tube diameters. The incubating period for each of the formulations in Table 7 was 4 hours.

**Table 7: Collection and incubation of liposomal siRNA formulations**

| Formulation | pH | Z-avg | PdI Avg | Encapsulation |
|---|---|---|---|---|
| Collection at 33% EtOH | 7.4 | 152 | 0.11 | 89% |
| Collection at 37% EtOH | 7.4 | 161 | 0.12 | 89% |
| Collection at 40% EtOH | 7.4 | 242 | 0.30 | 89% |

The results in Table 7 showed in general that the size of liposomal particles increased as the concentration of organic solvent ethanol in the collection reservoir increased. The dispersity of the particle size distribution also increased as the concentration of organic solvent increased. The results in Table 7 showed that high levels of encapsulation of the active siRNA agent were achieved with liposomal compositions prepared from an impinging stream and collection reservoir mixture at pH 7.4.

In a second example, effects of the incubating period on the gene-silencing activity of the liposomal siRNA formulation *in vivo* mouse were observed. ApoB gene silencing activity was determined *in vivo* mouse liver for liposomal formulations. Some RNAi-agents for silencing ApoB are described in WO08/109357.

ApoB gene silencing activity was determined *in vivo* mouse liver for certain liposomal formulations and compared to mouse serum cholesterol levels. The ApoB mRNA reduction activity *in vivo* and the corresponding serum cholesterol reduction *in vivo* are shown in Table 8. Each liposomal formulation in Table 8 was [C18-norArg-C16/CHEMS/chol/DMPE-PEG2k (50/28/20/2)]. The dose in each case was 1.0 mg/kg/day. Each of the formulations was prepared with a concentration of ethanol in the collection reservoir of 33% based on flow rates and transfer tube diameters.

**Table 8: Effect of incubation period on in vivo gene-silencing activity in mouse**

| Incubating period (hr) | *In vivo* ApoB knockdown (%) | Reduction in serum cholesterol (%) |
|---|---|---|
| 0 | 19 | 8 |
| 1 | 31 | 24 |
| 2 | 38 | 6 |
| 4 | 51 | 27 |

The results in Table 8 showed that the *in vivo* gene-silencing knockdown activity for liposomal formulations containing an ApoB gene silencing RNAi-agent increased to advantageous levels as the incubating period increased.

In a third example, the effects of an incubating period on the *in vivo* gene-silencing activity of liposomal siRNA formulations were observed as shown in Table 9. In these experiments, the liposomal siRNA formulations were prepared with dialysis rather than TFF filtration. The compositions were prepared with an impinging stream and collection reservoir mixture at pH 7.4. The concentration of organic solvent ethanol in the collection reservoir was varied from 30-36% as shown in Table 9. Each liposomal formulation in Table 9 was [C18-norArg-C16/CHEMS/chol/DMPE-PEG2k (50/28/20/2)].

**Table 9: Incubation effects on in vivo gene-silencing activity of liposomal siRNA formulations at various ethanol concentrations in the collection reservoir**

| Protocol | Incubating period (hr) | *In vivo* ApoB knockdown (%) | Reduction in serum cholesterol (%) |
|---|---|---|---|
| EtOH 30% | 0 | 11 | 6 |
| EtOH 30% | 4 | 48 | 31 |
| EtOH 33% | 0 | 50 | 31 |
| EtOH 33% | 4 | 72 | 52 |
| EtOH 33%, turbulent mixing | 0 | 8 | 19 |
| EtOH 33%, turbulent mixing | 4 | 62 | 54 |
| EtOH 36% | 0 | 15 | 18 |
| EtOH 36% | 4 | 59 | 40 |

The results in Table 9 showed that the *in vivo* mouse gene-silencing activity for liposomal formulations containing an ApoB gene silencing RNAi-agent significantly increased to advantageous levels when an incubating period was used.

In a fourth example, the effects of quenching the liposomal siRNA formulations on their *in vivo* mouse gene-silencing activity were observed as shown in Table 10. In these experiments, the liposomal siRNA formulations were prepared with an impinging stream and collection reservoir mixture at pH 7.4 and an incubating period of 1 hour. The concentration of organic solvent ethanol in the collection reservoir was quenched from 33% to a lower concentration as shown in Table 10. Each liposomal formulation in Table 10 was [C18-norArg-C16/CHEMS/chol/DMPE-PEG2k]. The stability of the formulation was determined by measuring average particle size and encapsulation of the siRNA active agent at 1 hour and 48 hours after quenching.

**Table 10: Quenching of liposomal siRNA formulations**

| Reduced EtOH | Z-avg particle size (nm) | | % Encapsulation | |
|---|---|---|---|---|
| | 1 hr | 48 hr | 1 hr | 48 hr |
| 30 | 171 | 186 | 85 | 72 |
| 25 | 163 | 169 | 83 | 87 |
| 20 | 158 | 159 | 84 | 88 |
| 15 | 164 | 166 | 83 | 87 |
| 10 | 158 | 162 | 83 | 85 |
| 5 | 160 | 161 | 83 | 85 |

The results in Table 10 showed that liposomal formulations containing an RNAi-agent maintained a stable average particle size and high level of encapsulation of the RNAi-agent over 48 hours after quenching to a concentration of ethanol below about 25%.

### EXAMPLE 4

### Effect of pH on liposomal compositions prepared by incubation

In this example, the effect of pH on the preparation of liposomal compositions was observed. Compositions were prepared by impinging and incubating using the basic protocol described in Example 1.

The active agent was a dsRNA for silencing ApoB that was prepared in an aqueous solution to 1 mg/mL.

The liposome-forming component was an ethanol solution containing the DILA2 amino acid compound C18:1-norArg(NH₃Cl)-C16, along with the lipids cholesteryl hemisuccinate (CHEMS, Anatrace, CH210), cholesterol (Anatrace CH200), and DMPE-PEG2k (Genzyme). The relative amounts of the DILA2 amino acid compound and the lipids was (50/28/20/2), which represents the percent (w/w) of each component with respect to the total amount of the DILA2 amino acid compound plus lipids, for the composition (C18:1-norArg(NH₃Cl)-C16 / CHEMS / cholesterol / DMPE-PEG2k).

As shown in Table 11, dsRNA formulations having a Z-avg particle size from 128-137 nm were made at both pH 7.4 and pH 4. The protocol for making the formulations of Table 11 was to add buffer to the impinging stream to adjust the concentration of ethanol to about 33%, followed by turbulent mixing. The stream was collected, and the collected mixture was incubated for 1 hour.

**Table 11: Liposomal compositions at pH 7.4 and 4**

| pH | PdI | Z-Avg | D(v) 0.25 | D(v) 0.5 | % Encapsulation |
|---|---|---|---|---|---|
| 7.4 | 0.11 | 134 | 94 | 120 | 90 |
| | 0.13 | 128 | 80 | 107 | 89 |
| 4 | 0.15 | 137 | 59 | 99 | --- |
| | 0.12 | 130 | 78 | 105 | --- |

In sum, the results shown in Table 11, as well as the results shown in Tables 7, 9 and 10 of Example 3 showed that a formulation of a liposome-encapsulated RNAi-inducing agent was prepared at a pH of 7.4.

### EXAMPLE 5

### Preparation of RNA-containing liposomal compositions by flow rate control

In this example, liposomal compositions were prepared by controlling the composition of an impinging stream using the flow rates of the RNAi-agent solution and the solution of liposome-forming components. Compositions were prepared by impinging and incubating using the basic protocol described in Example 1, except that the flow rates of the RNAi-agent solution and the solution of liposome-forming components were adjusted to achieve a certain concentration of organic solvent and RNAi-agent in the collection reservoir without additional SUP buffer. The formulations were prepared with an impinging stream that was collected and incubated for 1 hour. The pH was 7.4 and the active agent was a dsRNA for silencing ApoB.

The liposome-forming component was an ethanol solution containing the DILA2 amino acid compound C18:1-norArg(NH₃Cl)-C16, along with the lipids cholesteryl hemisuccinate (CHEMS, Anatrace, CH210), cholesterol (Anatrace CH200), and DMPE-PEG2k (Genzyme). The relative amounts of the DILA2 amino acid compound and lipids was (50/28/20/2).

As shown in Table 12, dsRNA formulations were prepared using a ratio of the flow rate of the RNAi-agent solution to the flow rate of the solution of liposome-forming components of 1.7:1, 3:1 and 5:1.

**Table 12: Preparation of liposomal compositions with controlled flow rate ratio**

| dsRNA µM | Flow ratio RNAi:DILA2 | EtOH (%) | Z-avg (nm) | PdI | % Encapsulation | *In vivo* ApoB KD (%) |
|---|---|---|---|---|---|---|
| 12 | 5:1 | 15 | 80 | 0.21 | 58 | 48 |
| 12 | 5:1 | 15 | 170 | 0.16 | 83 | 71 |
| 18 | 3:1 | 22 | 80 | 0.10 | 75 | 59 |
| 18 | 3:1 | 22 | 170 | 0.22 | 91 | 69 |
| 26 | 1.7:1 | 33 | 189 | 0.17 | 73 | 75 |

The results in Table 12 showed that liposomal compositions having good encapsulation of active agent and gene-silencing activity for ApoB *in vivo* mouse were prepared with a ratio of the flow rate of the RNAi-agent solution to the flow rate of the solution of liposome-forming components of from about 2 to about 5.

The results in Table 12 show that an average particle size as low as 80 nm with low particle size dispersity was achieved with a ratio of the flow rate of the RNAi-agent solution to the flow rate of the solution of liposome-forning components of from about 3 to about 5.

### EXAMPLE 6

### Filtration of RNA-containing liposomal compositions

In this example, the effect of concentrating the active RNAi-agent by tangential flow filtration in the preparation of liposomal compositions was observed. Compositions were prepared at pH 7.4 using the basic protocol described in Example 1 by collecting the impinging stream at 22% EtOH in the collection reservoir and incubating for 30 minutes. The composition was quenched to a concentration of EtOH of 10% for tangential flow filtration.

The liposome-forming component was an ethanol solution containing the DILA2 amino acid compound C18:1-norArg-C16, along with the lipids cholesteryl hemisuccinate (CHEMS, Anatrace, CH210), cholesterol (Anatrace CH200), and DMPE-PEG2k (Genzyme). The relative amounts of the DILA2 amino acid compound and lipids was (50/28/20/2).

The formulations were filtered by tangential flow filtration using an Amersham PES column. As shown in Table 13, the compositions remained stable regarding particle size and encapsulation of the active RNAi-agent under tangential flow filtration performed to concentrate the active RNAi-agent by a factor of up to sixteen. The final concentration of the active RNAi-agent was up to 5 mg/ml.

**Table 13: In vivo gene-silencing activity of liposomal RNAi formulations made with incubation and filtration**

| Protocol | Conc. factor | Z-avg (nm) | PdI | % Encapsulation |
|---|---|---|---|---|
| TFF | 1X | 130 | 0.17 | 85 |
| TFF | 2X | 132 | 0.13 | 84 |
| TFF | 4X | 130 | 0.16 | 84 |
| TFF | 8X | 133 | 0.15 | 80 |
| TFF | 12X | 132 | 0.16 | 82 |
| TFF | 16X | 134 | 0.17 | 84 |

### EXAMPLE 7

### Stability of RNA-containing liposomal compositions

In this example, the stability of a liposomal compositions was observed after being held for 7 days at elevated temperature. The composition was prepared by impinging and incubating for 1 hour using the basic protocol described in Example 1. A turbulent mixing tube was used and the concentration of EtOH in the collection reservoir was 33%. After preparation, the formulation was held for 7 days at a temperature of 45 °C. After 7 days, the average particle size was 116 nm and the encapsulation was 71%. For the heat-treated formulation, no loss of gene-silencing activity for ApoB *in vivo* mouse was observed after 7 days.

### EXAMPLE 8

### Peptide Binding Regions

The relative strength of the binding of a cationic peptide to an RNAi-inducing agent was measured with a dye binding assay.

The RNAi-inducing agent was prepared at 7.8 µl in 10ml to make a 20 µg/ml stock, then 75 µl/well. A SYBR gold dilution was prepared at 3.75 µl in 15 ml for 1:4000 dilution for a 2.5X stock.

Peptides were dissolved in Hepes buffer with 5% dextrose and diluted. Peptides were further diluted so that 75 µl could be added to each well resulting in the desired N:P (ranging from 0-4). Peptides were assumed to have a purity of 50%, but actual peptide amount was unknown.

A SYBR-GOLD Dye Binding Assay was performed. A 96-well plate assay with sample volume of 150 µl per well. Final dsRNA concentration was 10µg/ml in 10mM hepes/5%dextrose at pH 7.4. Peptides were diluted into different working solutions such that equal volumes were added to reach different N/P ratios. For the addition procedure, dsRNA was first added (75 µl of 20 µg/ml), followed by 150µl of 2.5X SYBR Gold. Peptide (75µl) was then added to compete off the SYBR dye. Total volume was 300µl. Fluorescence was corrected from background of dye alone in buffer. SYBR-Gold ex/em was 495nm/537nm, read on Molecular Devices plate reader.

Formulation particle sizes were determined by transferring to 384-well plate to be tested using the Wyatt particle sizer. Each well of the 96 well plate was transferred in duplicate. Volume remaining in plate was 200 µl.

Peptide release was triggered by disulfide reduction or enzymatic cleavage where appropriate. Cysteine-terminated peptides were cleavable by glutathione reduction. V-Cit containing peptides were cleavable by enzymatic cleavage by Cathepsin B. Glutathione was present at 0.1-10 mM intracellularly; Cathepsin B was 1 mM in lysosomes. (For Cathepsin B at 0.14ng/µl, see Teich et al BMC Gastroenterology 2002, 2:16).

For release, the appropriate molecule was added to a final concentration of 1mM in one of the duplicated wells followed by measurement of SYBR GOLD fluorescence with time.

As shown in Figure 6, the binding of a polyarginine binding region to dsRNA increased with the length of the polyarginine binding region. In Figure 6, the strongest binding (best ability to displace SYBR-Gold dye) was observed with PN3499, peptide (SEQ ID NO:353) RRRRRCCRRRRR, which was a dimer peptide containing a total of 10 arginines.

### EXAMPLE 9

### In Vitro Assay for PPIB Gene Expression Knockdown in A549 cells

Cyclophilin B (PPIB) gene knockdown measurements can be used as a primary activity-based *in vitro* assay for interfering RNA delivery formulations. Typically, the measurements were made as described below, with minor variations.

Cyclophilin B (PPIB) gene expression knockdown was measured in A549 human alveolar basal epithelial cells. For PPIB gene knockdown measurements, A549 cells were transfected with an interfering RNA formulation, total RNA prepared 24 hours after transfection, and PPIB mRNA assayed by RT-PCR. QRT-PCR of 36B4 (acidic ribosomal phosphoprotein PO) mRNA expression was performed for normalization.

A549 cells were seeded at 7,500 cells/well (96-well) and incubated overnight in medium. Confluency was about 50% at the time of transfection. Transfection complex was prepared by adding an interfering RNA to medium (OptiMEM™) and vortexing, separately adding a delivery formulation to medium (OptiMEM™) and vortexing, and finally mixing the interfering RNA in medium with the delivery formulation in medium and incubating 20 minutes at room temperature to make the transfection complex. The medium for incubated cells was replaced with fresh OptiMEM™ and transfection complex was added to each well. Cells were incubated for 5 hrs at 37°C and 5% CO_{2,} then complete medium was added (to a final fetal bovine serum concentration 10%) and incubation continued until 24 hours post-transfection.

For PPIB gene knockdown cells were lysed and RNA prepared (Invisorb RNA Cell HTS 96-Kit/C, Invitek, Berlin, or RNeasy 96 Kit, Qiagen). Quantitative RT-PCR was performed using One-Step qRT-PCR kit (Invitrogen) on a DNA Engine Opticon2 thermal cycler (BioRad).

Primers used for PPIB were:
(SEQ ID NO:354)
   5'-GGCTCCCAGTTCTTCATCAC-3' (forward) and
(SEQ ID NO:355)
   5'-CCTTCCGCACCACCTC-3' (reverse) with
(SEQ ID NO:356)
   5'-FAM-CTAGATGGCAAGCATGTGGTGTTTGG-TAMRA-3' for the probe.

For 36B4, primers were:
(SEQ ID NO:357)
   5'-TCTATCATCAACGGGTACAAACGA-3' (forward) and
(SEQ ID NO:358)
   5'-CTTTTCAGCAAGTGGGAAGGTG-3' (reverse) with
(SEQ ID NO:359)
   5'-FAM-CCTGGCCTTGTCTGTGGAGACGGATTA-TAMRA-3' for the probe.

The structures of some double-stranded RNAs (dsRNA) of this disclosure are shown in Table 14.

**Table 14: Double-stranded RNAs**

| RNA | SEQUENCES |
|---|---|
| DX4227 ApoB | (SEQ ID NO:360) |
| | Sense 5'-GGAAUCₘUₘUAₘUAₘUₘUₘUGAUCₘCAsA-3' |
| | (SEQ ID NO:361) |
| | Antisense 5'-ₘUₘUGGAUₘCAAAₘUAₘUAAGAₘUUCₘCsₘCsU-3' |
| DX4221 PPIB | (SEQ ID NO: 362) |
| | Sense 5'-GGAAAGACUGUUCCAAAAACAGUdGdG-3' |
| | (SEQ ID NO:363) |
| | Antisense 5'-CCACUGUUUUUGGAACAGUCUUUCCUU-3' |
| DC4377 PPIB conjugate | (SEQ ID NO:364) |
| | Sense 5'-GGAAAGACUGUUCCAAAAAUU-3' |
| | (SEQ ID NO:365) |
| | Antisense 5'-UUUUUGGAACAGUCUUUCCUU-3' |
| | Conjugated with Transportan on 3' end of sense strand: |
| | (SEQ ID NO:366) |
| | Mal-GWTLNSAGYLLGKINLKALAALAKKIL-amide |
| DX2816 Non-target Qneg | (SEQ ID NO:367) |
| | Sense 5'-UUCUCCGAACGUGUCACGUdTdT-3' |
| | (SEQ ID NO:368) |
| | Antisense 5'-ACGUGACACGUUCGGAGAAdTdT-3' |
| DX2940 LacZ | (SEQ ID NO:369) |
| | Sense 5'-CUACACAAAUCAGCGAUUUdTdT-3' |
| | (SEQ ID NO:370) |
| | Antisense 5'-AAAUCGCUGAUUUGUGUAGdTdC-3' |
| DX2742 PPIB MoCypB | (SEQ ID NO:371) |
| | Sense 5'-GGAAAGACUGUUCCAAAAAUU-3' |
| | (SEQ ID NO:372) |
| | Antisense 5'-UUUUUGGAACAGUCUUUCCUU-3' |

In Table 14, "mU" represents 2'-0-methyl uridine, "mC" represents 2'-O-methyl cytidine, and "s" represents a phosphorothioate linkage.

### EXAMPLE 10

### PPIB gene expression knockdown using a layered carrier and triggered release peptide

Nanoparticle carriers for an RNAi-inducing agent were tested for PPIB gene knockdown activity in A549 cells. A binary complex of a dsRNA RNAi-inducing agent with a triggered release peptide was initially formed at a particular N/P ratio. An endosomolytic agent was added, which adjusted the N/P ratio to a final value.

Formulations of layered carriers were in general prepared by first vortexing a dsRNA into HEPES/Dextrose buffer. Triggered release peptide was added with vortexing to complex the dsRNA. The complex was incubated for 15 minutes. Glutaraldehyde was added and the core allowed to crosslink for 1.5 h. The reaction was quenched by addition of 1 M Tris buffer pH 7.4 Endosomolytic agent was added, and the carrier mixture incubated for 15 minutes before adding to cells.

PPIB gene expression knockdown measurements using a layered carrier comprising a triggered release peptide are shown in Table 15. The results in Table 15 indicate that the carrier comprising a triggered release peptide was effective in the presence of an endosomolytic agent to deliver an active dsRNA agent to cells to produce a significant gene silencing effect.

**Table 15: PPIB gene expression knockdown using a triggered release peptide**

| Triggered release peptide | Endosomolytic agent | Binary N/P | Final N/P | dsRNA | Knockdown (%) (vs dsRNA control) |
|---|---|---|---|---|---|
| PN4110 | none | 5 | --- | DC4377 | 0 |
| | | | | 100 nM | |
| PN4110 | PN3033 | 5 | 2.5 | DC4377 | 65 |
| | | | | 100 nM | |
| PN4110 | PN3033 | 10 | 5 | DC4377 | 63 |
| | | | | 100 nM | |
| RNAIMAX | none | | | DC4377 | 94 |
| | | | | 25 nM | |

Materials used in this example were the following:
PN4110
   SEQ ID NO:373
   WWHHKKRRCCRRKKHHWW
PN3033 (diINF7)
   SEQ ID NO:374
   NH₂-GLFEAIEGFIENGWEGMIDGWYGC-CO₂H

The effect of the final N/P ratio on PPIB gene expression knockdown measurements using a layered carrier comprising a triggered release peptide was determined, and the results are shown in Table 16. The results in Table 16 indicate that the carrier comprising a triggered release peptide was effective in the presence of an endosomolytic agent to deliver an active dsRNA agent to cells to produce a significant gene silencing effects. Further, the results in Table 16 indicate that *in vitro* knockdown for a layered carrier comprising a triggered release peptide is enhanced at a lower final N/P ratio of 2.5-3.5.

**Table 16: Effect of final N/P ratio on gen knockdown in vitro**

| Triggered release peptide | Endosomolytic agent | Binary N/P | Final N/P | dsRNA | Knockdown (%) | |
|---|---|---|---|---|---|---|
| | | | | | vs untransf | vs control |
| PN4110 | none | 5 | --- | DX4221 | 11 | 14 |
| | | | | 100 nM | | |
| PN4110 | PN3033 | 5 | 2.5 | DX4221 | 70 | 72 |
| | | | | 100 nM | | |
| PN4110 | PN3033 | 5 | 3.5 | DX4221 | 72 | 74 |
| | | | | 100 nM | | |
| PN4110 | PN3033 | 5 | 4.5 | DX4221 | 40 | 36 |
| | | | | 100 nM | | |
| PN4110 | PN3033 | 4 | 2.5 | DX4221 | 55 | 50 |
| | | | | 100 nM | | |
| PN4110 | PN3033 | 4 | 3.5 | DX4221 | 36 | 41 |
| | | | | 100 nM | | |

### EXAMPLE 11

### Carrier particles having advantageously low delivery efficiency ratio

A batch of carrier nanoparticles was prepared using DX4227 condensed with PN4110. The delivery efficiency ratio of the batch was 0.63. The particle diameter was 223 nm (Z-avg, PDI 0.2).

A batch of carrier nanoparticles was prepared using DX4227 condensed with PN183. The delivery efficiency ratio of the batch was 1.28. The particle diameter was 208 nm (Z-avg, PDI 0.2).

Materials used in this example were the following:
PN183
   SEQ ID NO:375
   NH₂-KETWWETWWTEWSQPGRKKRRQRRRPPQ

### EXAMPLE 12

### Liposomal formulations prepared from amino acid lipids loaded with carrier particles

Liposomal formulations of an RNAi-agent with an amino acid lipid were prepared with the compositions shown in Table 17. RNAi-agents directed to ApoB are described in WO08/109357.

**Table 17: Liposomal formulations of an ApoB RNAi-agent with an amino acid lipid**

| No. | Liposomal formulation | Carrier particles | Initial N/P | Particle size Z-avg diameter (nm) | Delivery efficiency ratio |
|---|---|---|---|---|---|
| 1 | C18:1-norArg-C16/ | DX4227/PN4110 | 1.6 | | 9.21 |
| | CHEMS/ | | | | |
| | Chol/ | | | 185 (pH 7.4) | |
| | DMPE-PEG2k | | | 202 (pH 4.0) | |
| | (50/32/16/2) | | | | |
| 2 | C18:1-norArg-C16/ | DX4227/PN183 | 1.6 | | 9.86 |
| | CHEMS/ | | | | |
| | Chol/ | | | 298 (pH 7.4) | |
| | DMPE-PEG2k | | | 312 (pH 4.0) | |
| | (50/32/16/2) | | | | |
| 3 | C18:1-norArg-C16/ | DX4227/PN183 | 1.6 | 180 (pH 7.4) | 9.86 |
| | CHEMS/ | | | | |
| | Chol/ | | | | |
| | DMPE-PEG2k | | | | |
| | (50/32/16/2) | | | | |
| 4 | C18:1-norArg-C16/ | DX4227/PN183 | 0.8 | 192 | --- |
| | CHEMS/ | | | | |
| | Chol/ | | | | |
| | DMPE-PEG2k | | | | |
| | (50/32/16/2) | | | | |

### EXAMPLE 13

### ApoB gene silencing knockdown in vitro HepG2 cells using liposomal formulations prepared from amino acid lipids loaded with peptide condensate carrier particles

ApoB gene silencing activity was determined *in vitro* for a liposomal formulation prepared from an amino acid lipid loaded with peptide condensate carrier particles. ApoB gene knockdown activity was obtained from an *in vitro* assay in HepG2 cells. The normalized ApoB mRNA expression values for the formulation were measured.

Methods and protocol for the HepG2 assay were as follows:
Day 1: 25 µL complexes were added to wells, then 75 µL cells were added to wells in DMEM with 10% FBS or in OPTIMEM, no-serum. If in no-serum OPTIMEM, 100 µL full medium with 20% serum was added 4-5 hrs later, with final 10% FBS concentration.
Day 2: Cells were lysed at 24 hrs, RNA was prepared, and qRT-PCR was performed for ApoB and 36B4, or GAPDH mRNA was performed on Day 3.

The liposomal formulation [C18:1-norArg-C16/CHEMS/chol/DMPE-PEG2k (50/32/16/2)] was prepared, where C18:1-norArg-C16 is an amino acid lipid as described in U.S. Pat. Application No. 12/114,284. The liposomal formulation was loaded with peptide condensate carrier particles DX4227/PN4110. The initial N/P ratio was 0.8. This formulation exhibited 91% knockdown compared to Qneg for a concentration of 100 nM of the DX4227 RNAi-agent.

Additional liposomal formulations [C18:1-norArg-C16/CHEMS/chol/DMPE-PEG2k (50/32/16/2)] were prepared and loaded with peptide condensate carrier particles DX4227/PN183 as shown in Table 18.

**Table 18: ApoB gene silencing knockdown in vitro HepG2 for liposomal formulations**

| No. | Carrier particles in liposomal formulation | N:P with peptide | Delivery efficiency ratio | %KD vs Qneg (25 nM) | %KD vs Qneg (2.5 nM) |
|---|---|---|---|---|---|
| 1 | DX4227/PN183 | 0.6 | 9.54 | 95 | 95 |
| 2 | DX4227/PN183 | 0.7 | 9.7 | 95 | 89 |
| 3 | DX4227/PN183 | 0.8 | 9.86 | 87 | 76 |
| 4 | DX4227/PN183 | 0.6 | 11.69 | 89 | 88 |
| 5 | DX4227/PN183 | 0.7 | 11.85 | 91 | 88 |
| 6 | DX4227/PN183 | 0.8 | 12.01 | 90 | 80 |
| 7 | DX4227 control in RNAIMAX | --- | --- | 69 | 78 |

As shown in Table 18, these formulations exhibited advantageously high knockdown activity compared to Qneg for concentrations of the DX4227 RNAi-agent of 25 nM and 2.5 nM.

### EXAMPLE 14

### ApoB gene silencing knockdown in vivo using liposomal formulations prepared from amino acid lipids loaded with peptide condensate carrier particles

Liposomal formulations were prepared with an amino acid lipid loaded with peptide condensate carrier particles containing an ApoB gene silencing RNAi-agent. ApoB gene silencing activity was determined *in vivo* mouse for these liposomal formulations and compared to mouse serum cholesterol levels. The ApoB mRNA reduction activity *in vivo* and the corresponding serum cholesterol reduction *in vivo* are shown in Table 19. The liposomal formulation in Table 19 was [C18:1-norArg-C16/CHEMS/chol/DMPE-PEG2k (50/32/16/2)] and the dose in each case was 2 mg/kg.

**Table 19: ApoB gene silencing in vivo mouse using liposomal formulations loaded with peptide condensate carrier particles**

| No. | Carrier particles | % Reduction ApoB mRNA (p-value) | % Body weight change (48 hrs) | % Reduction serum cholesterol (p-value) |
|---|---|---|---|---|
| | (initial N/P)/ (final N/P) | | | |
| 1 | DX4227 alone | 55 | -0.6 | 45 |
| | (0.8) / (---) | (0.003) | | (0.000) |
| 2 | DX4227 alone | 64 | +1.3 | 56 |
| | (1.4) / (---) | (0.001) | | (0.000) |
| 3 | DX4227/PN183 | 50 | +0.8 | 43 |
| | (0.8) / (0.6) | (0.009) | | (0.004) |
| 4 | DX4227/PN183 | 48 | +0.9 | 38 |
| | (0.8) / (0.7) | (0.007) | | (0.000) |
| 5 | DX4227/PN183 | 34 | +1.1 | 27 |
| | (0.8) / (0.8) | (0.036) | | (0.002) |
| 6 | DX4227/PN183 | 70 | +1.4 | 52 |
| | (1.0) / (0.6) | (0.001) | | (0.000) |
| 7 | DX4227/PN183 | 42 | +0.6 | 31 |
| | (1.0) / (0.7) | (0.014) | | (0.001) |
| 8 | DX4227/PN183 | 26 | +3.2 | 18 |
| | (1.0) / (0.8) | (0.073) | | (0.004) |
| 9 | Control | 0 | +3.5 | 0 |

The results in Table 19 show that the liposomal formulations loaded with peptide condensate carrier particles containing an ApoB gene silencing RNAi-agent were advantageously well-tolerated in mouse because of the generally higher body weight increase 48 hours after administration as compared to the same formulations without the peptide condensate carrier particles.

Further, the results in Table 19 show that the liposomal formulations loaded with peptide condensate carrier particles were advantageously highly active for ApoB gene silencing *in vivo,* both in terms of reducing ApoB mRNA and reducing serum cholesterol. The results in Table 19 show that a higher initial N/P of 1.0 and a lower final N/P of 0.6-0.7 was preferred.

Additional liposomal formulations were prepared with an amino acid lipid loaded with peptide condensate carrier particles containing an ApoB gene silencing RNAi-agent. ApoB gene silencing activity was determined *in vivo* mouse for these liposomal formulations and compared to mouse serum cholesterol levels. The ApoB mRNA reduction activity *in vivo* and the corresponding serum cholesterol reduction *in vivo* are shown in Table 20.

**Table 20: ApoB gene silencing in vivo mouse using-liposomal formulations loaded with peptide condensate carrier particles**

| No. | Liposomal formulation | Carrier particles (initial N/P) Dose (mg/kg) | % Reduction ApoB mRNA (p-value) | % Body weight change (48 hrs) | % Reduction serum cholesterol (p-value) |
|---|---|---|---|---|---|
| 1 | C18:1-norArg-C16/ | | | -0.5 | |
| | CHEMS/ | DX4227 alone | | | |
| | Chol/ | (0.8) | 71 | | 64 |
| | DMPE-PEG2k | 2 (mg/kg) | (0.001) | | (0.000) |
| | (50/32/16/2) | | | | |
| 2 | C18:1-norArg-C16/ | | | -1.2 | |
| | CHEMS/ | DX4227 alone | | | |
| | Chol/ | (1.6) | 82 | | 71 |
| | DMPE-PEG2k | 2 (mg/kg) | (0.0001) | | (0.000) |
| | (50/32/16/2) | | | | |
| 3 | C18:1-norArg-C16/ | | | +0.5 | |
| | CHEMS/ | DX4227/PN4110 | | | |
| | Chol/ | (1.6) | 71 | | 52 |
| | DMPE-PEG2k | 1.7 (mg/kg) | (0.0001) | | (0.0001) |
| | (50/32/16/2) | | | | |
| 4 | C18:1-norArg-C16/ | | | +3.7 | |
| | CHEMS/ | DX4227/PN183 | | | |
| | Chol/ | (1.6) | 88 | | 64 |
| | DMPE-PEG2k | 1.4 (mg/kg) | (0.0002) | | (0.0001) |
| | (50/32/16/2) | | | | |
| 5 | Control | PBS | -2 | 0.2 | 0 |

For the liposomal formulations loaded with peptide condensate carrier particles in Table 20, the delivery efficiency ratio for Formulation 3 was 9.21 and the delivery efficiency ratio for Formulation 4 was 9.86.

The results in Table 20 show that the liposomal formulations loaded with peptide condensate carrier particles containing an ApoB gene silencing RNAi-agent were advantageously well-tolerated in mouse because of the body weight increase 48 hours after administration as compared to the body weight loss for the same formulations without the peptide condensate carrier particles.

Further, the results in Table 20 show that the liposomal formulations loaded with peptide condensate carrier particles were advantageously highly active for ApoB gene silencing *in vivo*, both in terms of reducing ApoB mRNA and reducing serum cholesterol.

The following numbered embodiments correspond to the claims of the parent application as originally filed and constitute part of the disclosure of the present application.
1. A process for making a composition comprising an active agent, the process comprising:
   a) providing a first stream comprising an aqueous buffer solution of an active agent;
   b) providing a second stream comprising a non-aqueous solution of one or more liposome-forming compounds in organic solvent;
   c) impinging the first stream on the second stream, thereby forming an impinging stream having a concentration of the organic solvent of from about 20% to about 50% v/v, and having a pH of from about 6 to about 7.4;
   d) incubating the impinging stream in a collection reservoir for a period of from about 0.5 hours to about 8 hours at a temperature of from about 20 °C to about 35 °C, thereby forming an incubate comprising liposomes.
2. The process of embodiment 1, further comprising quenching the incubate by adding buffer to the incubate sufficient to make the concentration of the organic solvent less than about 20% v/v.
3. The process of embodiment 1, wherein the liposome-forming compounds are one or more DILA2 amino acid compounds.
4. The process of embodiment 1, wherein one of the liposome-forming compounds is PONA, C18:1-norArg-C16.
5. The process of embodiment 1, further comprising that the volume flow rate of the first stream is two times or more the volume flow rate of the second stream.
6. The process of embodiment 1, further comprising that the volume flow rate of the first stream is three times or more the volume flow rate of the second stream.
7. The process of embodiment 1, further comprising the volume flow rate of the first stream being five times or more the volume flow rate of the second stream.
8. The process of embodiment 1, further comprising adjusting the pH of the impinging stream to be from about 3 to about 6.
9. The process of embodiment 1, further comprising incubating at a pH from about 3 to about 6.
10. The process of embodiment 1, further comprising adding buffer to the collection reservoir to adjust the concentration of the organic solvent.
11. The process of embodiment 1, further comprising that the active agent is encapsulated in liposomes at a level greater than about 50%.
12. The process of embodiment 1, further comprising that the active agent is encapsulated in liposomes at a level greater than about 70%.
13. The process of embodiment 1, wherein the active agent is a gene-silencing agent, a gene-regulating agent, an antisense agent, a peptide nucleic acid agent, a ribozyme agent, an RNA agent, or a DNA agent.
14. The process of embodiment 1, wherein the active agent is a UsiRNA.
15. The process of embodiment 1, wherein the active agent is a pharmaceutical compound.
16. The process of embodiment 1, wherein the liposomal composition retains gene-silencing activity for 7 days at a temperature of 45 °C.
17. The process of embodiment 1, wherein the liposomal composition retains encapsulation of the active agent for 7 days at a temperature of 45 °C.
18. The process of embodiment 1, wherein after tangential flow filtration the liposomes are of uniform size less than about 160 nm in diameter.
19. The process of embodiment 1, wherein after tangential flow filtration the liposomes are of uniform size with an average diameter from about 40 nm to about 160 nm.
20. The process of embodiment 1, wherein after tangential flow filtration the liposomes are of uniform size with an average diameter from about 80 nm to about 150 nm.
21. The process of embodiment 1, further comprising filtering the incubate by tangential flow filtration and diafiltration.
22. The process of embodiment 1, further comprising sterilizing the incubate.
23. The process of embodiment 1, further comprising exchanging the organic solvent with a different pharmaceutically-acceptable buffer.
24. The process of embodiment 1, further comprising adding organic solvent to the first stream at a concentration of from about 1% to about 40% v/v.
25. The process of embodiment 1, wherein the organic solvent is a (C1 6)alkanol at a concentration of about 40 to about 99% v/v in sterile water for injection.
26. The process of embodiment 1, wherein the organic solvent is a (C1 6)alkanol at a concentration of about 70 to about 95% v/v in sterile water for injection.
27. The process of embodiment 1, wherein the incubating period is from about 1 hours to about 4 hours.
28. A pharmaceutical composition made by a process of any one of embodiments 1-27.
29. A method for delivering a therapeutic nucleic acid to a biological cell comprising preparing a composition according to a process of any one of embodiments 1-27 and treating the cell with the composition.
30. A method for inhibiting expression of a gene in a biological cell comprising preparing a composition according to a process of any one of embodiments 1-27 and treating the cell with the composition.
31. A method for inhibiting expression of a gene in a mammal comprising preparing a composition according to a process of any one of embodiments 1-27 and administering the composition to the mammal.
32. A method for treating a disease in a human comprising preparing a composition according to a process of any one of embodiments 1-27 and administering the composition to the human, wherein the disease is cancer, bladder cancer, liver cancer, liver disease, hypercholesterolemia, an inflammatory disease, a metabolic disease, inflammation, arthritis, rheumatoid arthritis, encephalitis, bone fracture, heart disease, and viral disease.
33. A use of a composition made according to a process of any one of embodiments 1-27 for treating a disease including cancer, bladder cancer, liver cancer, liver disease, hypercholesterolemia, an inflammatory disease, a metabolic disease, inflammation, arthritis, rheumatoid arthritis, encephalitis, bone fracture, heart disease, and viral disease.
34. A use of a composition made according to a process of any one of embodiments 1-27 in the preparation of a medicament for treating a disease including cancer, bladder cancer, liver cancer, liver disease, hypercholesterolemia, an inflammatory disease, a metabolic disease, inflammation, arthritis, rheumatoid arthritis, encephalitis, bone fracture, heart disease, and viral disease.
35. A composition comprising a liposome containing one or more carrier particles, each carrier particle comprising an active nucleic acid agent and a peptide, wherein the ratio of the mass of the peptide plus the mass of the liposome to the mass of the nucleic acid agent is less than about 15.
36. The composition of embodiment 35, wherein the ratio of the mass of the peptide plus the mass of the liposome to the mass of the nucleic acid agent is less than about 12.
37. The composition of embodiment 35, wherein the ratio of the mass of the peptide plus the mass of the liposome to the mass of the nucleic acid agent is less than about 10.
38. The composition of embodiment 35, wherein the ratio of the mass of the peptide plus the mass of the liposome to the mass of the nucleic acid agent is less than about 9.
39. The composition of embodiment 35, wherein the ratio of the mass of the peptide plus the mass of the liposome to the mass of the nucleic acid agent is less than about 8.
40. The composition of embodiment 35, wherein the ratio of the mass of the peptide plus the mass of the liposome to the mass of the nucleic acid agent is less than about 5.
41. The composition of embodiment 35, wherein the composition has a knockdown activity of 50% or greater for gene silencing of ApoB in vivo.
42. The composition of embodiment 35, wherein the composition has a knockdown activity of 70% or greater for gene silencing of ApoB in vivo.
43. The composition of embodiment 35, wherein the composition has a knockdown activity of 90% or greater for gene silencing of ApoB in vivo.
44. The composition of embodiment 35, wherein the composition contains a liposome comprising an amino acid lipid.
45. The composition of embodiment 35, wherein the composition contains a charged carrier particle.
46. The composition of embodiment 35, wherein the composition contains a negatively charged carrier particle.
47. The composition of embodiment 35, wherein the composition contains a positively charged carrier particle.
48. The composition of embodiment 35, wherein the active nucleic acid agent is an RNAi-inducing agent or an antisense agent.
49. The composition of embodiment 35, wherein the active nucleic acid agent is an RNAi-inducing agent or an antisense agent and each liposome contains 500 or more copies of the active agent molecule.
50. The composition of embodiment 35, wherein the active nucleic acid agent is an RNAi-inducing agent or an antisense agent and each liposome contains 1000 or more copies of the active agent molecule.
51. The composition of embodiment 35, wherein the active nucleic acid agent is an RNAi-inducing agent or an antisense agent and each liposome contains 5000 or more copies of the active agent molecule.
52. The composition of embodiment 35, wherein the peptide is a cleavable peptide.
53. The composition of embodiment 35, wherein the peptide is a crosslinkable peptide.
54. The composition of embodiment 35, wherein the peptide is PN4110 having SEQ ID NO:373.
55. The composition of embodiment 35, wherein the peptide is PN183 having SEQ ID NO:375.
56. A method for delivering an active nucleic acid agent to a cell comprising preparing a composition according to any one of embodiments 35-55 and treating the cell with the composition.
57. A method for inhibiting expression of a gene in a cell comprising preparing a composition according to any one of embodiments 35-55 and treating the cell with the composition.
58. A method for inhibiting expression of a gene in a mammal comprising preparing a composition according to any one of embodiments 35-55 and administering the composition to the mammal.
59. A method for treating a disease in a human, the disease being selected from inflammatory diseases including rheumatoid arthritis, metabolic diseases including hypercholesterolemia, liver disease, encephalitis, bone fracture, heart disease, viral disease including hepatitis and influenza, and cancer, comprising preparing a composition according to any one of embodiments 35-55 and administering the composition to the human.
60. A use of a composition according to any one of embodiments 35-55 in the preparation of a medicament for treating a disease including inflammatory diseases including rheumatoid arthritis, metabolic diseases including hypercholesterolemia, liver disease, encephalitis, bone fracture, heart disease, viral disease including hepatitis and influenza, and cancer.
61. A use of a composition according to any of embodiments 35-55 for treating a disease selected from inflammatory diseases including rheumatoid arthritis, metabolic diseases including hypercholesterolemia, liver disease, encephalitis, bone fracture, heart disease, viral disease including hepatitis and influenza, and cancer.

## Claims

1. A process for making a composition comprising an active agent, the process comprising:
a) providing a first stream comprising an aqueous buffer solution of an active agent;
b) providing a second stream comprising a non-aqueous solution of one or more liposome-forming compounds in organic solvent;
c) impinging the first stream and the second stream, thereby forming an impinging stream having a concentration of the organic solvent of from about 20% to about 50% v/v, and having a pH of from about 6 to about 7.4; and
d) incubating the impinging stream in a collection reservoir for a period of from about 0.5 hours to about 8 hours at a temperature of from about 20 °C to about 35 °C, thereby forming an incubate comprising liposomes.

2. The process of claim 1, further comprising quenching the incubate by adding buffer to the incubate sufficient to make the concentration of the organic solvent less than about 20% v/v.

3. The process of claim 1, wherein the liposome-forming compounds comprise CHEMS and at least one compound selected from dioleoylphosphatidylethanolamine (DOPE), diphytanoylphosphatidylethanolamine (DPhPE), 1,2-Dioleoyl-*sn*-Glycero-3-Phosphocholine (DOPC), and 1,2-Diphytanoyl-*sn*-Glycero-3-Phosphocholine (DPhPC), 1,2-Dilauroyl-sn-glycerol (DLG); 1,2-Dimyristoyl-sn-glycerol (DMG); 1,2-Dipalmitoyl-sn-glycerol (DPG); 1,2-Distearoyl-sn-glycerol (DSG); 1,2-Dilauroyl-sn-glycero-3-phosphatidic acid (sodium salt; DLPA); 1,2-Dimyristoyl-sn-glycero-3-phosphatidic acid (sodium salt; DMPA); 1,2-Dipalmitoyl-sn-glycero-3-phosphatidic acid (sodium salt; DPPA); 1,2-Distearoyl-sn-glycero-3-phosphatidic acid (sodium salt; DSPA); 1,2-Diarachidoyl-sn-glycero-3-phosphocholine (DAPC); 1,2-Dilauroyl-sn-glycero-3-phosphocholine (DLPC); 1,2-Dimyristoyl-sn-glycero-3-phosphocholine (DMPC); 1,2-Dipalmitoyl-sn-glycero-O-ethyl-3-phosphocholine (chloride or triflate; DPePC); 1,2-Dipalmitoyl-sn-glycero-3-phosphocholine (DPPC); 1,2-Distearoyl-sn-glycero-3-phosphocholine (DSPC); 1,2-Dilauroyl-sn-glycero-3-phosphoethanolamine (DLPE); 1,2-Dimyristoyl-sn-glycero-3-phosphoethanolamine (DMPE); 1,2-Dipalmitoyl-sn-glycero-3-phosphoethanolamine (DPPE); 1,2-Distearoyl-sn-glycero-3-phosphoethanolamine (DSPE); 1,2-Dilauroyl-sn-glycero-3-phosphoglycerol (sodium salt; DLPG); 1,2-Dimyristoyl-sn-glycero-3-phosphoglycerol (sodium salt; DMPG); 1,2-Dimyristoyl-sn-glycero-3-phospho-sn-1-glycerol (ammonium salt; DMP-sn-1-G); 1,2-Dipalmitoyl-sn-glycero-3-phosphoglycerol (sodium salt; DPPG); 1,2-Distearoyl-sn-glycero-3-phosphoglycero (sodium salt; DSPG); 1,2-Distearoyl-sn-glycero-3-phospho-sn-1-glycerol (sodium salt; DSP-sn-1-G); 1,2-Dipalmitoyl-sn-glycero-3-phospho-L-serine (sodium salt; DPPS); 1-Palmitoyl-2-linoleoyl-sn-glycero-3-phosphocholine (PLinoPC); 1-Palmitoyl-2-oleoyl-sn-glycero-3-phosphocholine (POPC); 1-Palmitoyl-2-oleoyl-sn-glycero-3-phosphoglycerol (sodium salt; POPG); 1-Palmitoyl-2-oleoyl-sn-glycero-3-phosphoglycerol (sodium salt; POPG); 1-Palmitoyl-2-oleoyl-sn-glycero-3-phosphoglycerol (ammonium salt; POPG); 1-Palmitoyl-2-4o-sn-glycero-3-phosphocholine (P-lyso-PC); 1-Stearoyl-2-lyso-sn-glycero-3-phosphocholine (S-lyso-PC); and mixtures thereof.

4. The process of claim 1, further comprising that the volume flow rate of the first stream is two times or more the volume flow rate of the second stream.

5. The process of claim 1, further comprising:
- adjusting the pH of the impinging stream to be from about 3 to about 6, or
- incubating at a pH from about 3 to about 6.

6. The process of claim 1, further comprising adding buffer to the collection reservoir to adjust the concentration of the organic solvent.

7. The process of claim 1, further comprising that the active agent is encapsulated in liposomes at a level greater than about 50%, preferably greater than about 70%.

8. The process of claim 1, wherein the active agent is a gene-silencing agent, a gene-regulating agent, an antisense agent, a peptide nucleic acid agent, a ribozyme agent, an RNA agent, or a DNA agent, preferably an unlocked nucleobase analog-containing siRNA.

9. The process of claim 1, wherein after tangential flow filtration the liposomes are of uniform size less than about 160 nm in diameter, or have an average diameter from about 40 nm to about 160 nm, or from about 80 nm to about 150 nm.

10. The process of claim 1, wherein the organic solvent is a (C1-6)alkanol at a concentration of about 40 to about 99% v/v in sterile water for injection.

11. The process of claim 1, wherein the incubating period is from about 1 hours to about 4 hours.

12. A pharmaceutical composition obtainable by a process of any one of claims 1 to 11.

13. A non-therapeutic method for delivering a therapeutic nucleic acid to a biological cell comprising preparing a composition according to a process of any one of claims 1-11 and treating the cell with the composition.

14. A use of a composition obtainable by a process of any one of claims 1-11 for treating a disease including cancer, bladder cancer, liver cancer, liver disease, hypercholesterolemia, an inflammatory disease, a metabolic disease, inflammation, arthritis, rheumatoid arthritis, encephalitis, bone fracture, heart disease, and viral disease.
